(19) Europäisches Patentamt
European Patent Office
Office européen des brevets

(11) **EP 4 678 193 A1**

(12) # EUROPEAN PATENT APPLICATION
published in accordance with Art. 153(4) EPC

(43) Date of publication:
**14.01.2026 Bulletin 2026/03**

(21) Application number: **24767458.3**

(22) Date of filing: **08.03.2024**

(51) International Patent Classification (IPC):
***A61K 48/00*** (2006.01) ***A61K 31/713*** (2006.01)
***A61K 38/26*** (2006.01) ***A61P 3/04*** (2006.01)
***C12N 15/113*** (2010.01) ***A61K 31/7105*** (2006.01)

(52) Cooperative Patent Classification (CPC):
**A61K 31/7105; A61K 31/713; A61K 38/26; A61K 48/00; A61P 3/04; C12N 15/113**

(86) International application number:
**PCT/KR2024/003039**

(87) International publication number:
**WO 2024/186171 (12.09.2024 Gazette 2024/37)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **08.03.2023 KR 20230030823**
**13.09.2023 KR 20230121500**

(71) Applicant: **OliX Pharmaceuticals, Inc.**
**Seongnam-si, Gyeonggi-do 13106 (KR)**

(72) Inventors:
- **PARK, June Hyun**
  **Suwon-si, Gyeonggi-do 16281 (KR)**
- **CHOE, Jeong Yong**
  **Suwon-si, Gyeonggi-do 16295 (KR)**

(74) Representative: **Weickmann & Weickmann PartmbB**
**Postfach 860 820**
**81635 München (DE)**

Remarks:
The complete document including Reference Table(s) and the Sequence Listing(s) can be downloaded from the EPO website

(54) **RNAI FORMULATION FOR PREVENTION OR TREATMENT OF OBESITY AND COMBINATION THERAPY USING SAME**

(57) The present disclosure relates to an RNAi agent for preventing or treating obesity and combination therapy using the same. Specifically, the present disclosure relates an RNAi agent including an antisense strand having sequence complementarity to a MARC1 mRNA sequence and a sense strand having sequence complementarity to the antisense strand, and a pharmaceutical composition including the RNAi agent for preventing or treating obesity, and combination therapy of (i) the RNAi agent and (ii) GLP-1 receptor agonist or GLP-1/GIP receptor dual agonist.

FIG. 19

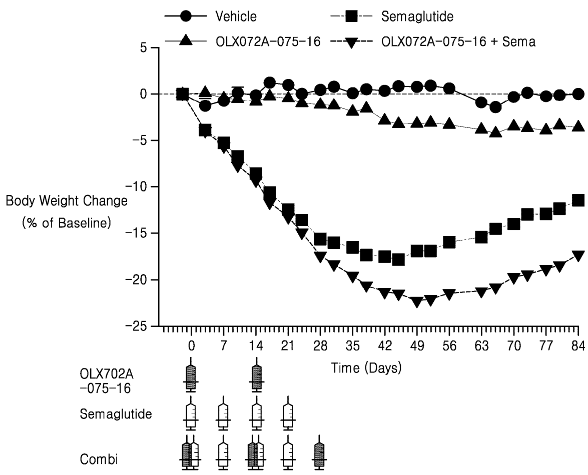


EP 4 678 193 A1

## Description

### Technical Field

The present disclosure relates to an RNAi agent for preventing or treating obesity and a combined therapy using the same, and more specifically, to a pharmaceutical composition for preventing or treating obesity, including an RNAi agent targeting MARC1, as an active ingredient.

### Background Art

A population of the obese is growing every year due to living environments, excessive nutritional intake from the rise of processed foods and eating out, and a decrease in physical activity. According to the World Health Organization (WHO), obesity is classified as a disease rather than a physiological phenomenon or a symptom, and obesity is diagnosed when the standard body mass index (BMI) is 30 or higher. In most cases, obesity refers to the body weight that is above the normal standard, but even if the body weight is below the standard, a high percentage of fat among body components is also referred to as obesity.

Obesity refers to excessive accumulation of body fat due to an imbalance between intake and consumption of energy, resulting in an increase in the number and size of adipocytes. The body stores energy in the form of triglycerides in adipocytes, but when the energy source is depleted, the stored fat is broken down into free fatty acids and glycerol and used as an energy source. However, excessive intake of energy promotes differentiation of adipocytes and increases the amount of stored fat in the body, thereby becoming a direct cause of obesity. Obesity not only changes a body shape due to accumulation of fat in the intestines and abdomen, but also acts as a risk factor that increases incidence of various diseases. When visceral fat accumulates excessively, problems arise with sugar metabolism in the body and symptoms such as abnormal hormone secretion and abnormal cytokine secretion occur. Obesity causes an increase in triglycerides and LDL-cholesterol and a decrease in HDL-cholesterol, leading to abnormalities in fat metabolism in the body, and a decrease in insulin receptors in tissues as well as a decrease in insulin sensitivity, and in this regard, the transport of glucose into cells is inhibited, causing hyperglycemia and diabetes. Also, obesity is known to be closely associated with the occurrence of metabolic diseases such as hyperlipidemia, cardiovascular disease, cancer, respiratory disorders, stroke, osteoarthritis, etc.

Medications for obesity can be broadly divided into appetite suppressants, fat absorption inhibitors, and glucagon-like peptide (GLP-1) analogues. Among these, semaglutide, one of the GLP-1 analogues, has been approved as an anti-obesity drug for long-term body weight management in adults by the U.S. Food and Drug Administration. However, regarding a product containing semaglutide as a main ingredient (product name: Wegovy®), the U.S. National Institutes of Health (NIH) has reported that, as a result of analyzing changes in body weight and cardiometabolic risk factors in patients one year after discontinuation of the therapeutic agents, the patients regained the body weight on average by two-thirds of the body weight loss after one year, and that cardiometabolic risk factors also increased at a similar level to the body weight.

On the other hand, treatment of a disease using the phenomenon of RNA interference has been attracting attention as a safer therapeutic agent for the treatment for a disease, since it targets mRNA and uses small interfering RNA (siRNA) to regulate gene expression at a translational level.

In this regard, as a result of putting intensive research efforts to develop a drug to treat obesity or to improve efficacy of existing therapeutic agents for obesity, the present inventors have developed RNA agents using RNA interference technology, thereby completing the present disclosure.

### Disclosure

### Technical Problem

An object of the present disclosure is to provide a pharmaceutical composition for preventing or treating obesity, including an RNAi agent that specifically inhibits expression of MARC1, as an active ingredient.

Another object of the present disclosure is to provide a combined preparation with a GLP-1 receptor agonist or GLP-1/GIP receptor dual agonist for preventing or treating obesity, including the RNAi agent, as an active ingredient.

Still another object of the present disclosure is to provide a method of preventing or treating obesity, including administering the RNAi agent to a subject.

### Technical Solution

To achieve the objectives, one aspect provides a pharmaceutical composition for preventing or treating obesity,

including an RNAi agent that specifically inhibits expression of MARC1, wherein the RNAi agent includes: an antisense strand consisting of EVP-mU*fG*mAmGmUmAfAmGfCmAmAmUmCfUmG*fG*mU*mC*mC*mU*mU, P-mA*fG*mAmUmCfCmAfGfAmGmCmUmGfCmG*fC*mC*mA*mC, or EVP-mU*fG*mAmUmCfCmAfGfAmGmCmUmGfCmG*fC*mC*mA*mC; and an antisense strand consisting of mC*mC*mAfGmAfUmUmGmCmUmUmAmCmUmCmA-GalNAc, mG*fC*mGfCfAfGmCfUmCfUmGfGmAfUmCfU-GalNAc, or mG*fC*mGfCfAfGmCfUmCfUmGfGmAfUmCfA-GalNAc.

Another aspect provides a combined preparation for preventing or treating obesity, including a GLP-1 receptor agonist or a GLP-1/GIP receptor dual agonist, and the RNAi agent as an active ingredient.

Another aspect provides a method of preventing or treating obesity, including administering the RNAi agent to a subject.

Another aspect provides use of the RNAi agent for preparation of a medicine for preventing or treating obesity.

Other purposes and advantages of the present disclosure will become more obvious with the following detailed descriptions, claims, and drawings. Contents not described herein will be sufficiently recognized and inferred by those skilled in the technical field of the present application or in a similar technical field therewith, and thus descriptions of such contents will be omitted.

## Advantageous Effects

The RNAi agent according to one aspect may reduce a body weight of a subject by inducing an increase in basal metabolism and promotion of fat oxidation in a subject, and thus may be used as an active ingredient in a therapeutic agent for obesity.

The RNAi agent according to one aspect not only exhibits an improved body weight loss effect when administered in combination with a GLP-1 receptor agonist- or a GLP-1/GIP dual agonist-based drug, but also maintains the body weight loss effect for a long period of time even after discontinuation of drug administration. Therefore, the RNAi agent may be used as an active ingredient in a combined preparation to improve the efficacy or reduce the side effects of existing obesity therapeutic agents.

## Description of Drawings

FIG. 1 shows results of confirming the relative body weight change (%) over time compared to the day a test substance is first administered, after administering OLX702A-031-1 according to an aspect to an obese animal model.

FIG. 2 shows changes in the body weight confirmed with the naked eye about 4 weeks after the day a test substance is administered, after administering OLX702A-031-1 according to an aspect to the obese animal model, wherein FIG. 2A shows results in a group provided with a normal diet, FIG. 2B shows results in a group of obese animal models administered with 1X PBS, FIG. 2C shows results in a group of obese animal models administered with OLX700A-001-8, and FIG. 2D shows results in a group of obese animal models administered with OLX702A-031-1.

FIG. 3 shows results of confirming the relative body weight change (%) over time compared to a control group, after administering OLX702A-031-2 according to an aspect to an obese animal model.

FIG. 4 shows changes in the dietary intake, after administering OLX702A-031-2 according to an aspect to an obese animal model, wherein FIG. 4A shows results of confirming the cumulative dietary intake over time, and FIG. 4B shows results of calculating and comparing the average daily dietary intake.

FIG. 5 shows changes in the activity levels for 48 hours, after administering OLX702A-031-2 according to an aspect to an obese animal model, wherein FIG. 5A shows results of confirming the activity levels over time, and FIG. 5B shows results of calculating and comparing the average activity level per hour.

FIG. 6 shows changes in the energy consumption levels for 48 hours, after administering OLX702A-031-2 according to an aspect to an obese animal model, wherein FIG. 6A shows results of confirming the energy consumption levels over time, and FIG. 6B shows results of calculating and comparing the average energy consumption level.

FIG. 7 shows changes in the respiration exchange rate (RER) for 48 hours, after administering OLX702A-031-2 according to an aspect to an obese animal model, wherein FIG. 7A shows results of confirming the RER over time, and FIG. 7B shows results of calculating and comparing the RER during an experiment period.

FIG. 8 shows results of confirming RER under conditions with light or dark for 48 hours, after administering OLX702A-031-2 according to an aspect to an obese animal model, wherein FIG. 8A shows results of calculating and comparing the RER under conditions with light, and FIG. 8B shows results of calculating and comparing the RER under conditions with dark.

FIG. 9 shows FGF 21 levels after administering OLX702A-031-2 according to an aspect to an obese animal, wherein FIG. 9A shows results of comparing expression levels of FGF 21 mRNA in liver tissue, and FIG. 9B shows results of

comparing FGF 21 levels in serum.

FIG. 10 shows results of comparing the FGF 21 levels compared with those in a group administered with semaglutide, after administering OLX702A-031-2 according to an aspect to an obese animal, wherein FIG. 10A shows results of comparing the expression levels of FGF 21 mRNA in liver tissue, and FIG. 10B shows results of comparing the FGF 21 levels in serum.

FIG. 11 shows results of confirming the relative body weight change (%) over time compared to a control group, after administering OLX702A-031-2 according to an aspect in combination with semaglutide to an obese animal model.

FIG. 12 shows changes in the dietary intake after administering OLX702A-031-2 according to an aspect in combination with semaglutide to an obese animal model, wherein FIG. 12A shows results of confirming the cumulative dietary intake over time, FIG. 12B shows results of calculating and comparing the average daily dietary intake for the period prior to discontinuation of semaglutide administration, and FIG. 12C shows results of calculating and comparing the average daily dietary intake for the entire experiment period.

FIG. 13 shows results of confirming changes in the relative body weight change (%) over time compared to a control group (Vehicle), after administering OLX702A-031-2 according to an aspect in combination with semaglutide to an obese animal model under conditions in which semaglutide is administered at 1-week intervals.

FIG. 14 shows results of confirming the cumulative dietary intake over time, after administering OLX702A-031-2 according to an aspect in combination with semaglutide to an obese animal model under conditions in which semaglutide is administered at 1-week intervals.

FIG. 15 shows results of confirming the relative body weight change (%) over time compared to a control group, after administering OLX702A-031-2 according to an aspect in combination with semaglutide to an obese animal model under conditions in which semaglutide is administered at 1-day intervals.

FIG. 16 shows results of confirming changes in the cumulative dietary intake over time, after administering OLX702A-031-2 according to an aspect in combination with semaglutide to an obese animal model under conditions in which semaglutide is administered at 1-day intervals.

FIG. 17 shows results of confirming the relative body weight change (%) over time compared to a control group (Normal Chow VC), after administering OLX702A-031-2 according to an aspect in combination with tirzepatide to an obese animal model.

FIGS. 18A to 18C show results of confirming changes in the dietary intake, after administering OLX702A-031-2 according to an aspect in combination with tirzepatide to an obese animal model, wherein FIG. 18A shows results of confirming the cumulative dietary intake over time, FIG. 18B shows results of calculating and comparing the average daily dietary intake for the period prior to discontinuation of tirzepatide administration, and FIG. 18C shows results of calculating and comparing the average daily dietary intake for the entire experiment period.

FIG. 19 shows results of confirming the relative body weight change (%) over time compared to the day a test substance is first administered, after administering OLX702A-075-16 according to an aspect in combination with semaglutide to an obese animal model.

FIG. 20 shows results of confirming the relative average daily dietary intake change (%) compared to the day a test substance is first administered, after administering OLX702A-075-16 according to an aspect in combination with semaglutide to an obese primate animal model.

FIG. 21 shows results of calculating and comparing the body fat percentage over time, after administering OLX702A-075-16 according to an aspect in combination with semaglutide to an obese primate animal model.

FIG. 22 shows results of measuring and comparing abdominal circumference over time, after administering OLX702A-075-16 according to an aspect in combination with semaglutide to an obese animal model.

BEST MODE

Mode for Invention

Each description and embodiment disclosed in the present application may also be applied to other descriptions and embodiments. That is, all combinations of the various elements disclosed in the application fall within the scope of the application. In addition, it should not be construed that the scope of the present application is limited by the detailed description described below.

One aspect provides a pharmaceutical composition for preventing or treating obesity, including a double-stranded RNAi agent including a sense strand and an antisense strand, as an active ingredient, wherein the antisense strand consists of EVP-mU*fG*mAmGmUmAfAmGfCmAmAmUmCfUmG*fG*mU*mC*mC*mU*mU, P-mA*fG*mAmUmCfCmAfGfAmGmCmUmGfCmG*fC*mC*mA*mC, or EVP-mU*fG*mAmUmCfCmAfGfAmGmCmUmGfCmG*fC*mC*mA*mC, each having complementary to a mitochondrial amidoxime reducing component 1 (MARC1) mRNA sequence, and the sense strand consists of mC*mC*mAfGmAfUmUmGmCmUmUmAmCmUmC-mA-GalNAc, mG*fC*mGfCfAfGmCfUmCfUmGfGmAfUmCfU-GalNAc, or mG*fC*mGfCfAfGmCfUmCfUmGfGmAfUmCfA-GalNAc.

Another aspect includes use of the RNAi agent, including: medicinal use of the RNAi agent for preventing or treating obesity; and use of the RNAi agent for preparing a medicament for preventing or treating obesity.

*RNAi agent*

The term "RNA interference (RNAi)" as used in the present specification refers to a mechanism for suppressing expression of a target gene by inducing degradation of mRNA of the target gene by introduction of double-stranded RNA (dsRNA) consisting of a strand having a sequence that is homologous to mRNA of the target gene and a strand having a sequence that is complementary to the aforementioned strand.

The term "RNAi agent" or "nucleic acid molecules inducing RNAi" as used in the present specification refers to any agent or nucleic acid molecule that is capable of suppressing or downregulating gene expression or viral replication by mediating the RNA interference in a sequence-specific manner. The term may refer to both an individual nucleic acid molecule, a plurality of the nucleic acid molecules, or a pool of the nucleic acid molecules. In an embodiment, the RNAi agent may be siRNA.

The term "small interfering RNA (siRNA, also referred to as short interfering RNA)" as used in the present specification refers to short double-stranded RNA (dsRNA) that sequence-specifically mediates efficient gene silencing.

The term "gene" as used in the present specification should be considered in its broadest sense, and may encode a structural protein or a regulatory protein. Here, the regulatory protein includes a transcription factor, a heat shock protein, or a protein involved in DNA/RNA replication, transcription, and/or translation. In the present disclosure, a target gene subject to expression suppression is inherent in the viral genome, and may be integrated into an animal genome or exist as an extrachromosomal component.

The term "antisense strand" as used in the present specification refers to a polynucleotide that is substantially or 100 % complementary to a target nucleic acid of interest, and may be complementary in whole or in part with, for example, messenger RNA (mRNA), non-mRNA RNA sequences (e.g., microRNA, piwiRNA, tRNA, rRNA, and hnRNA), or coding or non-coding DNA sequences.

The term "sense strand" as used in the present specification refers to a polynucleotide having the same nucleic acid sequence as a target nucleic acid, such as a polynucleotide that is identical in whole or in part to, for example, messenger RNA (mRNA), non-mRNA RNA sequences (e.g., microRNA, piwiRNA, tRNA, rRNA, and hnRNA), or coding or non-coding DNA sequences.

The term "complementarity" or "complementary" as used in the present specification refers to a generally accepted meaning in the art. This term may generally refer to formation or presence of hydrogen bond(s) between one nucleic acid sequence and another nucleic acid sequence, either by traditional Watson-Crick bonding or other non-traditional types of bonding as described herein. Perfect complementarity may refer that all contiguous residues of a nucleic acid sequence form hydrogen bonds with the same number of contiguous residues in a second nucleic acid sequence. Partial complementarity within a nucleic acid molecule may include various mismatches or non-base-paired nucleotides (e.g., 1, 2, 3, 4, 5, 6, 7, 8, 9, or 10 or more mismatches, e.g., 1 to 3 mismatches, non-nucleotide linkers, or non-base pair nucleotides). The partial complementarity may result in bulges, loops, overhangs, or blunt ends between a sense strand and an antisense strand of a nucleic acid molecule, or between an antisense strand of a nucleic acid molecule and a corresponding target nucleic acid molecule.

The term "blunt end" as used in the present specification refers to a generally accepted meaning in the art. Regarding the RNAi agent or nucleic acid molecule as used in the present specification, the term may refer to an end of a double-stranded siRNA molecule that lacks overhanging nucleotides. In the siRNA molecules described in the present specification, the 5'-end of the antisense strand and the 3'-end of the sense strand may form a blunt end.

*RNAi agent for inhiniting expression of MARC1*

"Mitochondrial amidoxime reducing component 1 (MARC1)" is a mammalian enzyme containing molybdenum, and is also referred to as MTARC1 or MOSC1, and deficiency of the MARC1 enzyme is associated with low blood cholesterol levels and regulation of fat metabolism, making it a potential therapeutic target for obesity. The MARC1 protein may be interpreted to include the naturally occurring wild-type MARC1 and functional variants thereof, and the sequence of the MARC1 protein or a gene encoding the same may be obtained from a known database such as GenBank of the U.S. National Institutes of Health.

The term "expression" as used in the present specification refers to the generally accepted meaning in the art. The term may generally refer to a process by which a gene ultimately produces a protein. The expression includes, but is not limited to, transcription, splicing, post-transcriptional modification, or translation. As used in the present specification, an expression level may be determined or monitored by detection of an mRNA level or a protein level.

The term "inhibition" or "reduction", as used in reference to MARC1 gene expression in a subject, refers to a

statistically significant decrease compared to an untreated or normal control group. The reduction may be, for example, at least 30 %, 35 %, 40 %, 45 %, 50 %, 55 %, 60 %, 60 %, 65 %, 70 %, 70 %, 80 %, 85 %, 90 %, or 95 % or more, but it may be below the detection limit, depending on the detection or measurement method.

In addition, the RNAi agent according to an embodiment may increase the expression or plasma level of fibroblast growth factor 21 (FGF 21). The "FGF 21 (MARC1)" regulates many types of metabolic pathways, including glucose metabolism and energy homeostasis, and it has been reported that administration of FGF 21 to an obese animal model can improve insulin sensitivity and lipoprotein profiles. The increase may be, for example, at least 30 %, 35 %, 40 %, 45 %, 50 %, 55 %, 60 %, 60 %, 65 %, 70 %, 70 %, 80 %, 85 %, 90 %, or 95 % or more relative to a non-treated group, but it may be above a detection level, depending on the detection or measurement method.

siRNA is a small interfering RNA and is involved in RNA interference (RNAi). RNAi is an intracellular gene regulation mechanism first discovered in 1998 in Caenorthabditis elegans, and its mechanism of action is known to induce target gene degradation by an antisense strand, which is one of RNA double strands which are introduced into the cell, that complementarily binds to mRNA of a target gene, and RNAi has recently been one of the most popular candidate technologies for developing new drugs.

However, contrary to this possibility, side effects and disadvantages of siRNA have been continuously reported. For the development of RNAi-based therapeutic agents, it is necessary to overcome barriers such as 1) absence of an effective delivery system, 2) off-target effect, 3) induction of immune response, and 4) saturation of RNAi machinery in cells. Although siRNA is an effective method for directly regulating expression of a target gene, it is difficult to develop a therapeutic agent due to these issues. In this regard, asymmetric shorter duplex siRNA (asiRNA) is an asymmetric RNAi-induced structure having a shorter double helix length compared to the 19+2 structure of siRNA in the art. asiRNA technology overcame issues such as off-target effect, saturation of RNAi mechanism, and immune response by TLR3, which are identified in the existing siRNA structure technology, and accordingly, it is possible to develop new RNAi drugs with low side effects.

Based on this, asymmetric siRNA including a sense strand and an antisense strand complementary to the sense strand is presented in this example, and siRNA according to an example does not cause issues such as off-target effect, saturation of RNAi machinery, etc., and thereby, may inhibit expression of an MARC1 gene to a desired degree while stably maintaining high delivery efficiency.

In an embodiment, the sense strand or the antisense strand of the RNAi agent may include one or more chemical modifications.

General siRNA cannot pass through the cell membrane due to high negative charge and high molecular body weight of the phosphate backbone structure, and is rapidly degraded and eliminated from the blood, making it difficult to deliver a sufficient amount to an actual target site for RNAi induction. Currently, in case of in vitro delivery, many high-efficiency delivery methods using cationic lipids and cationic polymers have been developed, however, in case of in vivo, it is difficult to deliver siRNA with as high efficiency as in vitro, and there is an issue of reduced siRNA delivery efficiency due to interaction with various proteins existing in the living body.

Accordingly, this embodiment suggests an RNAi agent that can be transfected into cells or animal models expressing MARC1, and then targeted with an asymmetric siRNA with high knockdown efficiency (including an antisense strand of SEQ ID NO: 1 (UGAGUAAGCAAUCUGGUCCUU), SEQ ID NO: 2 (AGAUCCAGAGCUGCGCCAC), or SEQ ID NO: 3 (UGAUCCAGAGCUGCGCCAC) and a sense strand of SEQ ID NO: 4 (CCAGAUUGCUUACUCA), SEQ ID NO: 5 (GCGCAGCUCUGGAUCU), or SEQ ID NO: 6 (GCGCAGCUCUGGAUCA), to enable effective and targeted intracellular delivery without a separate carrier by introducing chemical modifications.

In the present disclosure, the chemical modification in the sense strand or the antisense strand may include one or more selected from the group consisting of: binding with an N-acetyl galactosamine (GalNAc) derivative; modification of a nucleotide bond with phosphorothioate, boranophosphate, or methyl phosphonate; substitution of an -OH group at the 2' carbon position of the sugar structure of a nucleotide with $-CH_3$ (methyl), - $OCH_3$ (methoxy), $-NH_2$, -F, -O-2-methoxyethyl-O-propyl, -O-2methylthioethyl, -O-3-aminopropyl, or -O-3-dimethylaminopropyl; and binding of a phosphate group, E-vinylphosphonate, or a cell penetrating peptide.

In an embodiment, the N-acetyl galactosamine (GalNAc) derivative may have a structure of Formula 1 below. The RNAi agent with GalNAc derivatives bonded to the terminus may have improved delivery ability to target cells (e.g., hepatocytes), thereby providing an effective therapeutic effect.

**[Formula 1]**

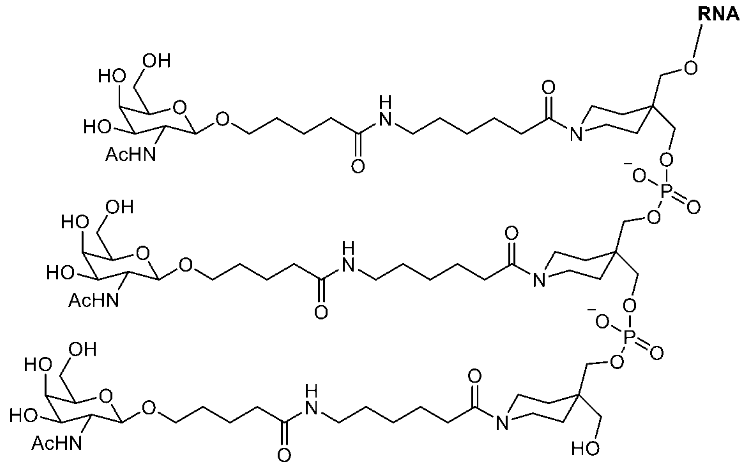

In an embodiment, the sense strand may include one or more chemical modifications selected from: modification of 2 to 4 nucleotide bonds adjacent to the 5' end with phosphorothioate, boranophosphate, or methyl phosphonate; substitution of an -OH group at the 2' carbon position of the sugar structure of at least one nucleotide with $-CH_3$ (methyl), $-OCH_3$ (methoxy), $-NH_2$, -F, -O-2-methoxyethyl-O-propyl, -O-2-methylthioethyl, -O-3-aminopropyl, or -O-3-dimethylaminopropyl; and binding with an N-acetylgalactosamine (GalNAc) derivative, or a cell penetrating peptide at the 3' end.

In an embodiment, the antisense strand may include one or more chemical modifications selected from: modification of 2 to 7 nucleotide bonds adjacent to the 3' end or the 5' end with phosphorothioate, boranophosphate, or methyl phosphonate; substitution of an -OH group at the 2' carbon position of the sugar structure of at least one nucleotide with $-CH_3$ (methyl), $-OCH_3$ (methoxy), $-NH_2$, -F, -O-2-methoxyethyl-O-propyl, - O-2-methyl methylthioethyl, -O-3-aminopropyl, or -O-3-dimethylaminopropyl; and binding with a phosphate group, E-vinylphosphonate, or a cell penetrating peptide at the 5' end.

In another embodiment, the RNAi agent may include at least one modification selected from the group consisting of: modification of 2 to 7 nucleotide bonds adjacent to the 3' end or the 5' end in a sense or an antisense strand to phosphorothioate; modification of an -OH group at the 2' carbon position of the sugar structure of at least one nucleotide in a sense strand or an antisense strand to $-OCH_3$ (methoxy), or -F; binding to an N-acetylgalactosamine (GalNAc) derivative at the 3' end of the sense strand; and binding with a phosphate group, or E-vinylphosphonate at the 5' end of an antisense strand.

In an embodiment, the antisense strand may consist of EVP-mU*fG*mAmGmUmAfAmGfCmAmAmUmCfUmG*fG*mU*mC*mC*mU*mU, P-mA*fG*mAmUmCfCmAfGfAmGmCmUmGfCmG*fC*mC*mA*mC, or EVP-mU*fG*-mAmUmCfCmAfGfAmGmCmUmGfCmG*fC*mC*mA*mC, and the sense strand may consist of mC*mC*mAfGmAfUmUmGmCmUmUmAmCmUmCmA-GalNAc, mG*fC*mGfCfAfGmCfUmCfUmGfGmAfUmCfU-GalNAc, or mG*fC*mGfCfAfGmCfUmCfUmGfGmAfUmCfA-GalNAc. In the sequences above, * represents a phosphorothioated bond, m represents to 2'-O-methyl, P represents 5'-phosphate group bond, f represents 2'-fluoro, EVP represents 5'-E-vinylphosphonate (5' E-VP) modification, and GalNAc represents a trivalent GalNAc derivative of Formula 1.

*Obesity*

The pharmaceutical composition may be utilized for the prevention or treatment of obesity by inhibiting the expression of the MARC1 genes.

Obesity is a disease caused by an energy imbalance due to excessive intake of nutrients compared to energy consumption over a long period of time. According to the World Health Organization (WHO), obesity is defined as an abnormal or excessive accumulation of fat in adipose tissue to the point of impairing health. Obesity is a representative disease of metabolic syndrome, and obesity itself is a disease, and is known to increase the risk of developing diseases caused by metabolic abnormalities, such as type 2 diabetes, hypertension, hyperlipidemia, coronary artery disease, etc.

Medications for obesity can be broadly divided into appetite suppressants, fat absorption inhibitors, and glucagon-like peptide (GLP-1) analogues. As a first approach, appetite suppression works by stimulating nerves in the brain to create a feeling of fullness, and Diethylpropion, Phendimetrazine, and Phentermine have been approved by the U.S. FDA and are commercially available. As a second approach, inhibition of absorption of ingested fat works on the digestive system and prevent the absorption of fat, and a representative example is Orlistat. Lastly, GLP-1 analogues are drugs conventionally used to treat diabetes, and have important physiological activities such as promoting insulin

secretion and inhibiting glucagon secretion. Semaglutide (product name: Wegovy®) as one of GLP-1 analogues has been approved by the U.S. FDA as an anti-obesity drug for long-term body weight management in adults. However, according to the U.S. National Institutes of Health (NIH), as a result of analyzing changes in the body weight of a patient and cardiometabolic risk factors one year after discontinuation of a therapeutic agent, it was reported that patients regained, on average, two-thirds of their body weight loss one year later, and that cardiometabolic risk factors also increased to a similar level as the body weight. Under this technical background, there is a need to develop new drugs that can improve the efficacy or reduce the side effects of existing obesity therapeutic agents.

*Pharmaceutical composition*

The term "effective ingredient" as used in the present specification refers to an appropriate effective amount of an ingredient that brings about a beneficial or desirable clinical or biochemical outcome. Specifically, the term may refer to an agent, an active agent, or an RNAi agent of an effective amount. The effective amount may be administered one or more times, and unlimitedly may be an appropriate amount for preventing a disease, alleviating symptoms, reducing the extent of the disease, stabilizing (i.e., not exacerbating) the disease state, delaying or reducing the rate of disease progression, or improving or temporarily alleviating and ameliorating (partially or fully) the disease state.

The term "prevention" as used in the present specification refers to any action that blocks an occurrence of a disease in advance, suppresses a disease, or delays progression of a disease. For example, the term refers to preventing the obesity or occurrence of characteristics thereof, interrupting the occurrence, or defending or protecting from the obesity or occurrence of characteristics thereof.

The term "treatment" as used in the present specification refers to both therapeutic treatment and preventive or prophylactic measures. Also, it refers to any action in which the symptoms of a disease are ameliorated or beneficially changed. For example, the term refers to preventing, reducing or improving the obesity or characteristics thereof, or delaying (attenuating) progression of the obesity or characteristics thereof in a subject.

The term "effective amount" as used in the present specification refers to a generally accepted meaning in the art. The term generally refers to an amount of a molecule, compound, or construct that elicits an intended biological response (e.g., a beneficial response) in a cell, tissue, a system, an animal, or a human, sought by researchers, veterinarians, physicians, or other clinicians, etc. Specifically, a "therapeutically effective amount" refers to an amount of a molecule, compound, or construct that is capable of eliciting a desirable medical response to the extent that a particular clinical treatment may be considered effective, due to, for example, a therapeutically relevant change in a measurable parameter associated with the disease or disorder. The therapeutically effective amount of a drug for treatment of a disease or a disorder may be an amount necessary to cause a therapeutically relevant change in the parameter.

A method of administering the pharmaceutical composition may be determined by a person skilled in the art based on the symptoms of the patient and the severity of the disease. In addition, the pharmaceutical composition may be formulated in various forms such as powders, tablets, capsules, solutions, injections, ointments, syrups, etc., and may be provided in unit-dose or multi-dose containers, for example, sealed ampoules and bottles. Also, it can be formulated in combination with one or more other agents currently in use for the prevention or treatment of a disease. Here, an effective amount of the other agents may be determined by the amount present in the preparation, the severity of a disease, and other factors.

The pharmaceutical composition of the present disclosure may be administered orally or parenterally. Routes of administration of the composition according to the present disclosure may be, for example, oral, intravenous, intramuscular, intraarterial, intramedullary, intrathecal, intracardiac, transdermal, subcutaneous, intraperitoneal, intestinal, sublingual, or topical, but are not limited thereto. Dosage of the composition according to the present disclosure varies depending on the patient's body weight, age, sex, health status, diet, administration time, method, excretion rate, or severity of disease, etc. and may be readily determined by those of average skill in the art. In addition, the composition of the present disclosure may be formulated into a suitable formulation for clinical administration using known techniques.

The pharmaceutical composition of the present disclosure may be administered in combination with an existing obesity therapeutic agent, for example, a GLP-1 receptor agonist or a GLP-1/GIP receptor dual agonist. Also, the pharmaceutical composition may be administered with the GLP-1 receptor agonist or the GLP-1/GIP receptor dual agonist separately, simultaneously, or sequentially.

One aspect provides a combined preparation for preventing or treating obesity, including: a first active ingredient including a GLP-1 receptor agonist or a GLP-1/GIP receptor dual agonist; and a second active ingredient including a double-stranded RNA agent including a sense strand and an antisense strand, wherein the antisense strand consists of EVP-mU*fG*mAmGmUmAfAmGfCmAmAmUmCfUmG*fG*mU*mC*mC*mU*mU, P-mA*fG*mAmUmCfCmAfGfAmGmCmUmGfCmG*fC*mC*mA*mC, or EVP-mU*fG*mAmUmCfCmAfGfAmGmCmUmGfCmG*fC*mC*mA*mC, each having sequence complementarity with MARC1 mRNA, and the sense strand consists of mC*mC*mAfGmAfU-mUmGmCmUmUmAmCmUmCmA-GalNAc, mG*fC*mGfCfAfGmCfUmCfUmGfGmAfUmCfU-GalNAc, or mG*fC*mGfCfAfGmCfUmCfUmGfGmAfUmCfA-GalNAc.

Another aspect provides: medicinal use of the RNAi agent for preventing or treating obesity through combination with a GLP-1 receptor agonist or a GLP-1/GIP receptor dual agonist; and use of the RNAi agent for preparing a combined preparation with a GLP-1 receptor agonist or a GLP-1/GIP receptor dual agonist for preventing or treating obesity.
Another aspect provides a kit for preventing or treating obesity, including the combined preparation, wherein the combined preparation is provided to a subject in the form that the first active ingredient is contained in a first compartment and the second active ingredient is contained in a second compartment.
Since the combined preparation for preventing or treating obesity and the kit for preventing or treating obesity may include or use the aforementioned RNAi agent or pharmaceutical composition, the common contents between them are omitted to avoid undue complexity herein.

*Combined preparation and kit including combined preparation*

The term "combined preparation" as used in the present specification refers to two or more drugs/preparations that are used simultaneously or nearly simultaneously (i.e., one after the other, or administered on the same day). The combined preparation is a medicinal composition for achieving a combined therapeutic effect by administering two or more drugs individually, simultaneously, or sequentially, or by administering the same at regular or undetermined intervals. Although not limited thereto, the combined treatment effect may be defined as efficacy as measured by, for example, degree of response, rate of response, time for disease progression, or survival period, that is therapeutically superior to and synergistic with efficacy that would be obtained by administering one or more substances of the combined therapy at usual doses.
The terms "synergy," "therapeutic synergy," and "synergistic effect" as used in the present specification refer to a phenomenon that treatment of a patient with a combination of therapeutic agents (e.g., a GLP-1 receptor agonist or GLP-1/GIP receptor dual agonist used in combination with the RNAi agent according to an embodiment) results in a therapeutically superior outcome to the outcome achieved by each of the individual components of the combination when used alone. In this regard, the therapeutically superior outcome may include at least one of the following: (a) an increase in therapeutic response greater than either or both of the individual effects of each agent alone at the same dose in the combination; (b) a reduction of the dose of one or more agents in the combination without reducing therapeutic efficacy; (c) a reduction in the incidence of side effects while providing the same or greater therapeutic benefit than monotherapy of the same dose of each agent as in the combination; (d) a reduction of dose-limiting toxicity while providing a greater therapeutic benefit than monotherapy of each agent at the same dose; and (e) a delay or minimization of the induction of drug resistance.
In an embodiment, the combined preparation may include: a first active ingredient including a GLP-1 receptor agonist or a GLP-1/GIP receptor dual agonist; and a second active ingredient including the RNAi agent for preventing or treating obesity. The first active ingredient and the second active ingredient may be administered individually, simultaneously, or sequentially, and such combined therapy may be able to: 1) exhibit better therapeutic efficacy than the individual effect of the GLP-1 receptor agonist or the GLP-1/GIP receptor dual agonist; 2) reduce the dose of the GLP-1 receptor agonist or the GLP-1/GIP receptor dual agonist without reducing therapeutic efficacy; or 3) resolve side effects or problems (e.g., rapid body weight gain after discontinuation of medication) arising from the GLP-1 receptor agonist or the GLP-1/GIP receptor dual agonist.
In an embodiment, in the combined preparation or the kit including the same, the combined preparation may be provided to a subject in the form that the first active ingredient is contained in a first compartment and the second active ingredient is contained in a second compartment.

*GLP-1 receptor agonist*

GLP-1 is a 30 amino acid-long incretin hormone secreted by intestinal L-cells in response to food intake. In healthy individuals, GLP-1 plays an important role in controlling postprandial blood glucose by promoting glucose-dependent insulin secretion by the pancreas. GLP-1 also inhibits glucagon secretion, causing a decrease in hepatic glucose production. Also, GLP-1 delays gastric emptying, slows small intestine motility, and delays food absorption.
GLP-1 receptor agonists or GLP-1 analogues may have functions of promoting synthesis and secretion of insulin, inhibiting glucagon secretion, suppressing gastric hunger, and inhibiting food intake as well as promoting glucose use. The GLP-1 receptor agonist has been developed as a drug for the treatment of type 2 diabetes, but has recently been in the spotlight as an active ingredient for the treatment of obesity. However, it has been reported that the GLP-1 receptor agonist as a treatment for obesity can cause vomiting and nausea when taken, and that the body weight may increase again after discontinuation of medication. The GLP-1 receptor agonist may be selected from the group consisting of semaglutide, liraglutide, exenatide, taspoglutide, albiglutide, lixisenatide, and dulaglutide.

*GLP-1/GIP receptor dual agonist*

GIP (Glucose-dependent insulinotropic polypeptide) is a 42 amino acid-long incretin hormone secreted by K cells in the small intestine after ingestion of glucose or fat. In healthy individuals, GIP performs the functions of promoting insulin secretion from the pancreas and lowering blood sugar concentration, in a blood glucose concentration-dependent manner, and has been reported to increase GLP-1 activity and have antiinflammatory effects, improved lipid metabolism effects, etc.

The GIP receptor agonist or the GIP analogue is a substance that can complement the effects of the GLP-1 receptor agonists, and has been reported to have a greater effect on glucose and body weight when combined with the GLP-1 receptor agonist. Therefore, the GLP-1/GIP receptor dual agonist is being utilized as active ingredients for the treatment obesity. The GLP-1/GIP receptor dual agonist may be selected from the group consisting of tirzepatide, NN9709, SAR-438335, and ZP-DI-70.

Another aspect provides a method of preventing or treating obesity, including administering the RNAi agent to a subject.

Another aspect provides a combined therapy for preventing or treating obesity, including administering to a subject the RNAi agent separately, simultaneously, or sequentially in combination with the GLP-1 receptor agonist or the GLP-1/GIP receptor dual agonist.

Since the method of preventing or treating obesity or the combined therapy may include or utilize any of the aforementioned RNAi agent, the pharmaceutical composition, or the combined preparation as it is, the common contents therebetween are omitted to avoid undue complexity herein.

The term "subject", used herein, refers to a subject in need of treatment for a disease, specifically, liver disease, and more specifically, the term may include all mammals such as a human or a non-human primate, a mouse, a dog, a cat, a horse, a cow, sheep, a pig, a goat, a camel, and an antelope.

Hereinafter, the present disclosure will be described in more detail through examples. However, these examples are intended to illustrate the present disclosure, and the scope of the present disclosure is not limited to these examples.

**[Examples]**

**Example 1. Synthesis of RNAi agent**

In this example, a nucleic acid molecule for inducing RNAi targeting MARC1 was synthesized. In detail, an RNAi agent according to one example was synthesized by introducing various chemical modifications (2'OMe, P, fluoro, EVP) and binding a derivative denoted as "trivalent GalNAc" to the 3' end of the sense strand. The synthesis method in this example was a method known in the art, and the sequence information of the synthesized RNAi agent is shown in Table 1 below.

**[Table 1]**

| Name | Sequence(5' → 3') | |
|---|---|---|
| OLX702A-031-1 | Sense | mG*fC*mGfCfAfGmCfUmCfUmGfGmAfUmCfU-GalNAc |
| | Antisense | P-mA*fG*mAmUmCfCmAfGfAmGmCmUmGfCmG*fC*mC*mA*mC |
| OLX702A-031-2 | Sense | mG*fC*mGfCfAfGmCfUmCfUmGfGmAfUmCfA-GalNAc |
| | Antisense | EVP-mU*fG*mAmUmCfCmAfGfAmGmCmUmGfCmG*fC*mC*mA*mC |
| OLX702A-075-16 | Sense | mC*mC*mAfGmAfUmUmGmCmUmUmAmCmUmCmA-GalNAc |
| | Antisense | EVP-<br>mU*fG*mAmGmUmAfAmGfCmAmAmUmCfUmG*fG*mU*mC*mC*mU*mU |

In detail, chemical modifications denoted by "*", "m", "f", "P", and "GalNAc" in Table 1 are as shown in Table 2.

**[Table 2]**

| Notation | Chemical modification |
|---|---|
| * | Phosphorothioate bond |
| m | 2'-O-methyl |

(continued)

| Notation | Chemical modification |
|---|---|
| f | 2'-fluoro |
| P | 5'-phosphate group |
| EVP | E-vinylphosphonate |
| "GalNAc" | Trivalent GalNAc derivative of Formula 1 |

In detail, in Table 2, "*" represents a form in which an existing phosphodiester bond is substituted with a phosphorothioate bond, and "m" represents a form in which an existing 2'-OH is substituted with 2'-O-methyl. Also, "f" represents, for example, in case of fG, a form in which 2'-OH of existing guanine (G) is substituted with fluorine, and "P" represents a form in which a phosphate group is bonded to the 5'-end (a phosphate group is bonded to an oxygen bonded to the 5th carbon in the 5' end base). Also, "EVP" represents a form in which E-vinylphosphonate is bonded to the 5'-end of the antisense strand, and "GalNAc" represents a form in which a trivalent GalNAc derivative of Formula 1 is bonded to the 3'-end of the sense strand.

Meanwhile, the structure of the trivalent GalNAc derivative is as shown in Formula 1.

## [Formula 1]

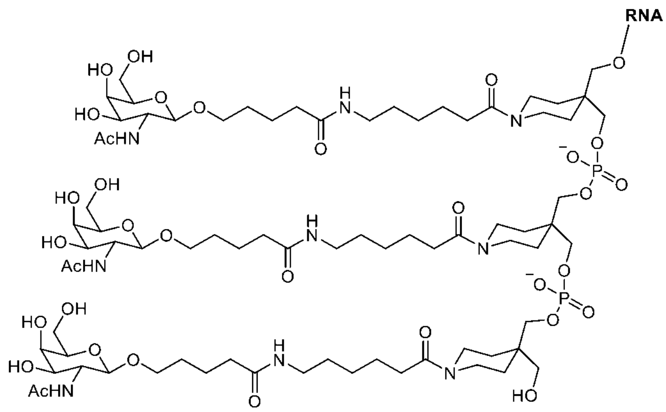

**[Experimental Examples]**

**I. Confirmation of anti-obesity effect by administration of RNAi agent according to embodiment**

**Experimental Example 1. Anti-obesity effects after administration of OLX702A-031-1 or OLX702A-031-2**

In this Experimental Example, targeting an animal model with high-fat diet-induced obesity (DIO), anti-obesity effects were evaluated by measuring changes in the body weight after administration of OLX702A-031-1 or OLX702A-031-2.

**1-1. Confirmation of body weight loss effect after administration of OLX702A-031-1**

Targeting C57BL/6 mice (8 weeks old, male, Koatech), the animal model groups were divided into a group provided with a normal diet (NCD) and a group provided with a high-fat diet for 32 weeks (HFD), and depending on a substance to be administered, the animal model groups provided with a high-fat diet (having a fat content of 60 %, i.e., 60 % HFD) were divided into a group administered with 1X PBS (HFD-VC), a group administered with OLX700A-001-8 targeting Factor IX (HFD-NC), and a group administered with OLX702A-031-1 according to an example (HFD-031-1). Specific experimental conditions for the animal model groups are as shown in Table 3.

**[Table 3]**

| Group | Dose (mpk, mg/kg) | Route | Interval | Treatment | | Number |
|---|---|---|---|---|---|---|
| 1 | - | SC | QW | NCD | 1X PBS | 1 |

(continued)

| Group | Dose (mpk, mg/kg) | Route | Interval | Treatment | | Number |
|---|---|---|---|---|---|---|
| 2 | - | SC | QW | 60% HFD | 1X PBS | 3 |
| 3 | 10 | SC | QW | | OLX700A-001-8 | 4 |
| 4 | 10 | SC | QW | | OLX702A-031-1 | 5 |
| Number: Number of animals subject to the treatment | | | | | | |

On Week 28 from the time the high-fat diet was provided, OLX702A-031-1 according to an embodiment was administered, with 10 mpk doses administered subcutaneously a total of 4 times at 1-week intervals. This experiment was conducted for 4 weeks, and the body weight was measured three times a week along with the administration of the test substance. Also, about 4 weeks from the day the test substance was administered, changes in the body weight in each animal model group were evaluated with the naked eye. Meanwhile, a control group was administered with 1X PBS (HFD-VC), and a negative control group was administered subcutaneously with OLX700A-001-8 at a dose of 10 mpk at 1-week intervals (HFD-NC).

As a result, as shown in FIG. 1, the group administered with OLX702A-031-1 according to an embodiment (HFD-031-1) showed a significant body weight loss effect from about 10 days of the administration of the test substance, and the body weight loss of about 15 % was maintained throughout the experiment period. Also, as shown in FIG. 2, in the HFD-031-1 group according to an embodiment, a significant body weight loss effect was confirmed at a level that could be identified with the naked eye.

**1-2. Confirmation of body weight loss effect after administration of OLX702A-031-2**

Targeting C57BL/6 mice (8 weeks old, male, Koatech) fed with a high-fat diet (having a fat content of 60 %, i.e., 60 % HFD) for 8 weeks, depending on a substance to be administered, the animal model groups were divided into a group administered with 1X PBS (Vehicle), a group administered with semaglutide, and a group administered with OLX702A-031-2 according to an embodiment (OLX702A-031-2). Specific experimental conditions for the animal model groups are as shown in Table 4.

**[Table 4]**

| Group | Dose (mpk) | Route | Interval | Treatment | | Number |
|---|---|---|---|---|---|---|
| 1 | - | SC | QW | 60% HFD | 1X PBS | 12 |
| 2 | 10 | SC | Q2W | | OLX702A-031-2 | 12 |
| 3 | 0.42 | SC | QW | | semaglutide | 12 |

At Week 8 from the time the high-fat diet was provided, OLX702A-031-2 according to an embodiment was administered subcutaneously at a dose of 10 mpk for a total of 4 times at 2-week intervals. This experiment was conducted for 8 weeks, and the body weight (FX-2000i, A&D company, 0.01 g to 2200 g) and the dietary intake (CSG201F, OHAUS, 0.1 g to 200 g) were measured twice a week along with the administration of test substance. The dietary intake was calculated by measuring the difference between the supply and the balance for each individual. Meanwhile, a group administered with 1X PBS was used as a control group (Vehicle), and a group administered with semaglutide subcutaneously at a dose of 0.42 mpk for a total of 8 doses at 1-week intervals was used as semaglutide. Here, mpk refers to mg (drug)/kg (target animal).

As a result, as shown in FIG. 3 and Table 5, a significant body weight loss effect was confirmed in the group administered with OLX702A-031-2 according to an embodiment (OLX702A-031-2).

**[Table 5]**

| Group | Day | Body change from baseline (% of VC) | | | | | | | | | | | | | | | | |
|---|---|---|---|---|---|---|---|---|---|---|---|---|---|---|---|---|---|---|
| | | Day 0 | Day 3 | Day 7 | Day 10 | Day 14 | Day 17 | Day 21 | Day 24 | Day 28 | Day 31 | Day 34 | Day 38 | Day 42 | Day 45 | Day 48 | Day 52 | Day 55 |
| Vehicle | Mean | 0.00 | 0.00 | 0.00 | 0.00 | 0.00 | 0.00 | 0.00 | 0.00 | 0.00 | 0.00 | 0.00 | 0.00 | 0.00 | 0.00 | 0.00 | 0.00 | 0.00 |
| | SE | 0.00 | 0.55 | 0.64 | 0.56 | 0.96 | 1.04 | 0.82 | 0.94 | 1.03 | 0.97 | 1.11 | 1.26 | 1.27 | 1.28 | 1.23 | 1.28 | 1.50 |
| Semaglutide | Mean | 0.00 | -7.19 | 4.97 | 13.3 2 | 7.79 | 16.8 5 | 13.9 9 | 20.8 3 | 17.8 1 | 25.2 4 | 21.0 3 | 26.4 6 | 23.0 4 | 30.8 9 | 27.1 7 | 31.1 2 | 27.1 9 |
| | SE | 0.00 | 0.47 | 0.48 | 0.92 | 0.67 | 1.08 | 1.22 | 1.43 | 1.64 | 1.83 | 2.00 | 1.66 | 1.86 | 1.81 | 2.06 | 1.97 | 2.07 |
| OLX702A-031-2 | Mean | 0.00 | 0.49 | 2.10 | 5.27 | 6.61 | 8.57 | 12.3 1 | 14.6 5 | 17.1 3 | 18.0 2 | 19.2 4 | 20.3 0 | 21.3 6 | 22.4 3 | 24.1 5 | 24.9 6 | 26.3 8 |
| | SE | 0.00 | 0.49 | 0.65 | 0.65 | 0.86 | 1.13 | 1.37 | 1.48 | 1.26 | 1.46 | 1.36 | 1.46 | 1.69 | 2.03 | 1.89 | 1.67 | 1.61 |

Also, as shown in FIG. 4 and Table 6, the OLX702A-031-2 group showed a small reduction in the dietary intake compared to the positive control group, indicating that the body weight loss effect did not simply result from a decrease in the dietary intake.

[Table 6]

| | | Cumulative food consumption (g/mouse) | | | | | | | | | | | | | | |
|---|---|---|---|---|---|---|---|---|---|---|---|---|---|---|---|---|
| Group | Da y | Day 3 | Day 7 | Day 10 | Day 14 | Day 17 | Day 21 | Day 24 | Day 28 | Day 31 | Day 34 | Day 38 | Day 42 | Day 45 | Day 48 | Day 52 | Day 55 |
| Vehicle | Me an | 8.1 | 17.0 | 25.4 | 32.8 | 41.8 | 51.5 | 61.1 | 71.0 | 79.7 | 87.9 | 97.4 | 107. 1 | 115. 9 | 124. 4 | 131. 4 | 139. 9 |
| | SE | 0.52 | 0.40 | 0.70 | 1.05 | 1.40 | 1.19 | 1.60 | 1.47 | 1.49 | 1.57 | 2.03 | 1.87 | 1.99 | 2.01 | 2.04 | 2.16 |
| Semaglut ide | Me an | 3.5 | 13.3 | 17.3 | 27.7 | 33.3 | 44.1 | 50.0 | 61.3 | 66.1 | 75.7 | 81.6 | 93.3 | 98.1 | 107. 9 | 112. 4 | 122. 1 |
| | SE | 0.27 | 0.43 | 0.68 | 0.84 | 1.41 | 1.74 | 1.84 | 2.21 | 2.29 | 2.63 | 2.70 | 2.99 | 3.06 | 3.32 | 3.41 | 3.61 |
| OLX702A -031-2 | Me an | 7.8 | 15.4 | 22.3 | 29.7 | 38.7 | 47.3 | 56.1 | 65.5 | 74.1 | 82.4 | 92.1 | 102. 3 | 111. 2 | 119. 4 | 126. 5 | 134. 8 |
| | SE | 0.47 | 0.49 | 0.79 | 0.60 | 0.93 | 0.92 | 1.24 | 1.13 | 1.32 | 1.49 | 1.84 | 1.95 | 2.12 | 2.24 | 2.21 | 2.32 |

### Experimental Example 2. Confirmation of increased basal metabolism and fat oxidation effects

In this experimental example, targeting the animal model with high-fat DIO, changes in the basal metabolism levels and the fat oxidation levels by the administration of OLX702A-031-2 were measured to determine key factors in the anti-obesity effect.

Dietary intake, energy consumption, and locomotor activity for the animal group models of Experimental Example 1-2 were measured by using a comprehensive animal metabolic monitoring system (CLAMS: Columbus instruments, Columbus, OH, USA). In detail, in the afternoon of Day 12 from the time the test substance was first administration, the animal model groups were placed in the CLAMS and adapted to the changed environment. On the next day (i.e., Day 13 after the first administration of the test substance), starting at 9 a.m., oxygen uptake ($VO_2$), carbon dioxide consumption ($VCO_2$), activity levels (measured by using infrared beams), food intake, and water intake were measured every 10 minutes for a total of 48 hours. Afterwards, the respiration exchange rate (RER) was calculated according to the ratio ($VCO_2/VO_2$) of carbon dioxide consumption to oxygen, and the energy expenditure was calculated according to Equation 1.

## Equation 1

$$\text{Energy expenditure} = (3.815 + 1.232 \times \text{RER}) \times VO_2$$

As a result, as shown in FIGS. 5 and 6 and Tables 7 and 8, there was no difference in the activity levels among each animal model group, and the energy expenditure of the semaglutide-administered group was confirmed to be similar to that of the control group. On the other hand, the energy expenditure of the group administered with OLX702A-031-2 according to an embodiment (OLX702A-031-2) was confirmed to be significantly increased regardless of the changes in the activity level.

## [Table 7]

| Activity (counts/hr) | | | | | | | | | | | | | | | | | | | | | | | | | |
|---|---|---|---|---|---|---|---|---|---|---|---|---|---|---|---|---|---|---|---|---|---|---|---|---|---|
| Group | Hour | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 |
| Vehicle | | 160.7 | 63.2 | 435.9 | 322.5 | 254.2 | 218.6 | 294.2 | 405.0 | 684.0 | 793.5 | 622.8 | 930.3 | 2883.9 | 3128.3 | 2547.3 | 1669.8 | 840.0 | 1648.6 | 1288.4 | 1259.3 | 1355.7 | 1296.8 | 350.0 | 353.3 |
| Semaglutide | | 146.8 | 151.4 | 178.9 | 355.4 | 255.6 | 419.5 | 354.7 | 577.0 | 440.5 | 521.4 | 646.4 | 1963.8 | 2034.1 | 2441.1 | 1640.3 | 1497.8 | 1920.4 | 1015.8 | 1476.0 | 737.8 | 1006.6 | 1249.1 | 321.8 | 211.5 |
| OLX702A-031-2 | | 207.3 | 160.9 | 399.4 | 372.6 | 341.9 | 333.0 | 448.9 | 335.1 | 208.3 | 653.3 | 727.4 | 567.0 | 2204.8 | 1942.0 | 1668.6 | 2992.8 | 1522.0 | 921.4 | 1314.0 | 774.6 | 726.9 | 2237.5 | 455.8 | 396.2 |
| Group | Hour | 25 | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 |
| Vehicle | | 107.3 | 103.9 | 153.7 | 330.3 | 439.8 | 218.6 | 289.6 | 435.7 | 467.7 | 735.5 | 467.3 | 907.2 | 1621.1 | 3203.5 | 2560.9 | 1559.8 | 1628.3 | 1082.2 | 1121.9 | 1063.7 | 1521.1 | 1238.3 | 584.5 | 410.8 |
| Semaglutide | | 274.8 | 192.6 | 311.8 | 335.6 | 281.6 | 362.3 | 459.4 | 303.3 | 662.5 | 178.3 | 442.0 | 818.7 | 1364.9 | 1881.7 | 2290.4 | 1310.6 | 1339.3 | 1442.9 | 961.8 | 850.4 | 1236.8 | 1394.9 | 567.4 | 260.3 |
| OLX702A-031-2 | | 122.9 | 230.3 | 205.3 | 231.5 | 250.7 | 471.6 | 256.3 | 415.9 | 287.8 | 354.3 | 248.5 | 973.4 | 867.4 | 1969.9 | 2192.1 | 1765.8 | 1192.3 | 1477.4 | 1354.4 | 1084.3 | 993.3 | 1869.1 | 563.8 | 412.5 |

## [Table 8]

| Energy expenditure (kcal/mouse/hr) | | | | | | | | | | | | | | | | | | | | | | | | | |
|---|---|---|---|---|---|---|---|---|---|---|---|---|---|---|---|---|---|---|---|---|---|---|---|---|---|
| Group | Hour | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 |
| Vehicle | | 0.455 | 0.450 | 0.457 | 0.455 | 0.446 | 0.454 | 0.460 | 0.457 | 0.461 | 0.474 | 0.490 | 0.524 | 0.574 | 0.556 | 0.572 | 0.547 | 0.547 | 0.542 | 0.532 | 0.532 | 0.533 | 0.517 | 0.451 | 0.475 |
| Semaglutide | | 0.466 | 0.453 | 0.456 | 0.464 | 0.453 | 0.465 | 0.465 | 0.474 | 0.465 | 0.476 | 0.507 | 0.535 | 0.543 | 0.564 | 0.550 | 0.549 | 0.556 | 0.532 | 0.536 | 0.526 | 0.526 | 0.514 | 0.467 | 0.474 |
| OLX702A-031-2 | | 0.505 | 0.501 | 0.507 | 0.510 | 0.494 | 0.492 | 0.493 | 0.453 | 0.452 | 0.511 | 0.532 | 0.535 | 0.579 | 0.597 | 0.611 | 0.626 | 0.599 | 0.592 | 0.590 | 0.552 | 0.593 | 0.594 | 0.542 | 0.526 |
| Group | Hour | 25 | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 |
| Vehicle | | 0.455 | 0.449 | 0.457 | 0.467 | 0.457 | 0.449 | 0.461 | 0.467 | 0.463 | 0.455 | 0.455 | 0.531 | 0.567 | 0.603 | 0.553 | 0.555 | 0.564 | 0.553 | 0.551 | 0.553 | 0.561 | 0.529 | 0.522 | 0.503 |
| Semaglutide | | 0.450 | 0.455 | 0.461 | 0.463 | 0.454 | 0.464 | 0.464 | 0.456 | 0.472 | 0.453 | 0.455 | 0.515 | 0.544 | 0.555 | 0.567 | 0.532 | 0.542 | 0.547 | 0.531 | 0.536 | 0.536 | 0.532 | 0.501 | 0.475 |
| OLX702A-031-2 | | 0.506 | 0.499 | 0.496 | 0.452 | 0.500 | 0.511 | 0.493 | 0.494 | 0.492 | 0.506 | 0.514 | 0.567 | 0.551 | 0.597 | 0.611 | 0.613 | 0.596 | 0.617 | 0.597 | 0.556 | 0.575 | 0.592 | 0.565 | 0.536 |

Also, as shown in FIGS. 7 and 8 and Table 9, the RER of the OLX702A-031-2 group showed a value closer to 0.7 compared to the semaglutide-administered group (generally, it is known that the RER is about 0.7 when fats are broken down, and is about 1.0 when carbohydrate is broken). The value close to 0.7 shows that fat oxidation, i.e., fat burning, was actively conducted. Furthermore, this trend was observed in both light and dark conditions, and was more prevalent under the dark condition.

## [Table 9]

| Respiratory exchange rate ($VCO_2/VO_2$) | | | | | | | | | | | | | | | | | | | | | | | | |
|---|---|---|---|---|---|---|---|---|---|---|---|---|---|---|---|---|---|---|---|---|---|---|---|---|
| Group / Hour | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 |
| Vehicle | 0.716 | 0.720 | 0.728 | 0.717 | 0.730 | 0.720 | 0.726 | 0.733 | 0.732 | 0.731 | 0.731 | 0.730 | 0.720 | 0.714 | 0.706 | 0.711 | 0.706 | 0.710 | 0.704 | 0.711 | 0.711 | 0.710 | 0.707 | 0.713 |
| Semaglutide | 0.730 | 0.729 | 0.718 | 0.732 | 0.731 | 0.742 | 0.736 | 0.741 | 0.739 | 0.748 | 0.746 | 0.746 | 0.732 | 0.737 | 0.726 | 0.724 | 0.730 | 0.725 | 0.721 | 0.724 | 0.733 | 0.725 | 0.729 | 0.742 |
| OLX702A-031-2 | 0.697 | 0.695 | 0.694 | 0.702 | 0.699 | 0.711 | 0.701 | 0.702 | 0.712 | 0.714 | 0.716 | 0.718 | 0.722 | 0.715 | 0.712 | 0.707 | 0.706 | 0.702 | 0.699 | 0.695 | 0.697 | 0.696 | 0.693 | 0.713 |
| Group / Hour | 25 | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 |
| Vehicle | 0.714 | 0.712 | 0.720 | 0.721 | 0.724 | 0.715 | 0.730 | 0.727 | 0.726 | 0.730 | 0.728 | 0.736 | 0.725 | 0.725 | 0.716 | 0.713 | 0.712 | 0.706 | 0.710 | 0.704 | 0.705 | 0.707 | 0.701 | 0.710 |
| Semaglutide | 0.731 | 0.732 | 0.735 | 0.741 | 0.742 | 0.751 | 0.747 | 0.739 | 0.745 | 0.751 | 0.756 | 0.765 | 0.753 | 0.753 | 0.743 | 0.738 | 0.733 | 0.732 | 0.729 | 0.736 | 0.732 | 0.731 | 0.722 | 0.734 |
| OLX702A-031-2 | 0.705 | 0.697 | 0.703 | 0.705 | 0.708 | 0.706 | 0.710 | 0.713 | 0.714 | 0.724 | 0.727 | 0.733 | 0.722 | 0.719 | 0.721 | 0.720 | 0.712 | 0.706 | 0.706 | 0.700 | 0.701 | 0.705 | 0.712 | 0.712 |

Referring to the results above, it was confirmed that the body weight loss effect caused by the RNAi agent according to an embodiment was due to an increase in the energy expenditure, that is, the basal metabolism, and that an excellent fat oxidation effect was exhibited as well.

### Experimental Example 3. Confirmation of mechanism of anti-obesity effect

In this experimental example, targeting the animal model with high-fat DIO, the fibroblast growth factor 21 (FGF 21) level by administration of OLX702A-031-2 was evaluated, and the FGF 21-related effect was compared with the semaglutide-administered group.

### 3-1. Effect of increasing expression of FGF 21

Targeting C57BL/6 mice (8 weeks old, male, Koatech) provided with a high-fat diet for 10 weeks, the animal model groups were divided into a group provided with 1X PBS (Vehicle) and a group provided with OLX702A-031-2 according to an embodiment (OLX702A-031-2), depending on a substance to be administered. Specific experimental conditions for the animal model groups are as shown in Table 10.

**[Table 10]**

| Group | Dose (mpk) | Route | Interval | Treatment | | Number |
|---|---|---|---|---|---|---|
| 1 | - | SC | Single injection | 60% HFD | 1X PBS | 4 |
| 2 | 10 | SC | Single injection | | OLX702A-031-2 | 5 |

At Week 10 from the time the high-fat diet was provided, OLX702A-031-2 according to an embodiment was administered subcutaneously at a dose of 10 mpk for a total of 1 time. Then, on Day 12 after the subcutaneous administration, each animal model group was sacrificed, and the expression level of FGF 21 mRNA in liver tissue and the level of FGF 21 in serum were measured. Meanwhile, a group administered with 1X PBS (Vehicle) was used as a control group.

As a result, as shown in FIG. 9, the expression level of FGF 21 mRNA in liver tissue and the level of FGF 21 in serum of the group administered with OLX702A-031-2 according to one embodiment (OLX702A-031-2) were all confirmed to be significantly increased compared to the control group.

### 3-2. Comparison with semaglutide-administered group

Targeting C57BL/6 mice provided with a high-fat diet for 8 weeks, the animal model groups were divided into a group administered with 1X PBS (Vehicle),a group administered with semaglutide (Semaglutide), and a group administered with OLX702A-031-2 according to an embodiment (OLX702A-031-2), depending on a substance to be administered. Specific experimental conditions for the animal model groups are as shown in Table 11.

**[Table 11]**

| Group | Dose (mpk) | Route | Interval | Treatment | | Number |
|---|---|---|---|---|---|---|
| 1 | - | SC | QW | 60% HFD | 1X PBS | 12 |
| 2 | 10 | SC | Q2W | | OLX702A-031-2 | 12 |
| 3 | 0.42 | SC | QW | | semaglutide | 12 |

At Week 8 from the time the high-fat diet was provided, OLX702A-031-2 according to an embodiment was administered subcutaneously at a dose of 10 mpk for a total of 4 times at 2-week intervals. Then, at Week 8 after the subcutaneous administration, each animal model group was sacrificed, and the expression level of FGF 21 mRNA in liver tissue and the level of FGF 21 in serum were measured. Meanwhile, a group administered with 1X PBS was used as a control group (VC), and a group administered with semaglutide subcutaneously at a dose of 0.42 mpk for a total of 8 doses at 1-week intervals was used as a comparison group.

As a result, as shown in FIG. 10, it was confirmed that the expression level of FGF 21 mRNA in liver tissue and the level of FGF 21 in serum of the semaglutide-administered group were similar to those of the control group or showed a tendency to decrease, whereas the expression level of FGF 21 in the group administered with OLX702A-031-2 according to an embodiment (OLX702A-031-2) administered group was significantly increased, consistent with the aforementioned experimental results. Referring to the results above, it was confirmed that OLX702A-031-2 according to an embodiment, unlike semaglutide, exhibited an effect of enhancing the expression of FGF 21 due to an additional mechanism of operation for the body weight loss.

Summarizing the experimental results above, the body weight loss effect of the RNAi agent targeting MARC1 according to an embodiment was based on the increase in the basal metabolism/energy metabolism, unlike the existing GLP-1 receptor agonist-based therapeutic agents. Also, in terms of mechanism of operation, this effect could be further distinguished by additionally enhancing the expression of FGF 21. Therefore, as confirmed in this experimental example, the RNAi agent according to an embodiment is expected to exhibit not only an anti-obesity effect by a single administration, but also an improved anti-obesity effect when administered in combination with a GLP-1 receptor agonist-based therapeutic agent.

## II. Confirmation of improved anti-obesity effect by combined administration with GLP-1 receptor agonist or GLP-1/GIP dual agonist

To confirm the efficacy of a combination agent containing the RNAi agent targeting MARC1 according to an embodiment, the anti-obesity effect of combined use with semaglutide, a GLP-1 receptor agonist, and combined use with tirzepatide, a GLP-1/GIP receptor dual agonist was to be confirmed.

### Experimental Example 1. Confirmation of body weight loss by combined administration of OLX702A-031-2 and semaglutide

In this experimental example, targeting the animal model with high-fat DIO, changes in the body weight and food intake by the combined administration of OLX702A-031-2 with semaglutide, a GLP-1 receptor agonist, were measured to evaluate the anti-obesity effect.

### 1-1. Comparison of body weight changes

Targeting C57BL/6 mice (6 weeks old, male, Koatech) provided with a high-fat diet for 8 weeks, the animal model groups were divided into a group provided with a normal diet (NCD) and a group provided with a high-fat diet for 8 weeks (HFD), and depending on a substance to be administered, the animal model groups provided with a high-fat diet were divided into a group administered with 1X PBS (HFD VC), a group administered with only semaglutide (Semaglutide), and a group administered with OLX702A-031-2 according to an embodiment in combination with semaglutide (OLX702A-031-2+sema). Specific experimental conditions for the animal model groups are as shown in Table 12.

**[Table 12]**

| Group | Dose (mpk) | Route | Interval | Treatment | | Number |
|---|---|---|---|---|---|---|
| 1 | - | SC | | NCD | 1X PBS | 3 |

(continued)

| Group | Dose (mpk) | Route | Interval | Treatment | | Number |
|---|---|---|---|---|---|---|
| 2 | - | SC | | 60% HFD | 1X PBS | 4 |
| 3 | 0.42 | SC | BIW | | semaglutide | 5 |
| 4 | 10/0.42 | SC | Q2W/BIW | | OLX702A-031-2/ semaglutide | 5 |

At Week 8 from the time the high-fat diet was provided, OLX702A-031-2 according to an embodiment was administered in combination with semaglutide. Specifically, the semaglutide was administered subcutaneously twice a week at a dose of 0.42 mpk for a total of 16 times, and then the administration was discontinued for the remainder of the experiment period. The OLX702A-031-2 was administered subcutaneously at a dose of 10 mpk for a total of 8 times at 2-week intervals for 16 weeks in total. This experiment was conducted for 16 weeks, and along with the administration of the test substance, each subject was weighed 5 times a week from the start of the experiment (D0) until the 66th day, and then 3 times a week until the end of the experiment. Meanwhile, a group in which 1X PBS was administered to an obese animal model (VC) was used as a control group, and a group in which only semaglutide was administered subcutaneously 16 times in total at a dose of 0.42 mpk, twice a week for a total of 8 weeks (Semaglutide) was used as a comparison group.

As a result, as shown in FIG. 11 and Table 13, the group administered with only semaglutide (Semaglutide) showed a significant body weight loss effect during the administration period, but the body weight rapidly increased after the administration of semaglutide was discontinued (on Week 8 after the administration of the test substance). Meanwhile, the OLX702A-031-2+sema group according to an embodiment showed an improved body weight loss effect of about 1.5 times compared to the Semaglutide group, not only during a period of the administration with semaglutide, but also during a period that the administration of semaglutide was stopped.

## [Table 13]

| Relative Body Weight to HFD VC (%) | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|
| Group | DAY | D0 | D3 | D7 | D9 | D10 | D13 | D14 | D15 | D16 | D17 | D20 | D21 | D22 | D23 | D24 | D27 | D28 | D29 | D31 | D34 | D35 | D36 | D38 | D42 | D43 | D44 | D48 | D50 | D51 |
| Normal Chow VC | Mean | 77.9 | 78.7 | 79.2 | 78.4 | 78.4 | 79.2 | 79.6 | 79.6 | 79.6 | 78.7 | 76.8 | 75.2 | 77.7 | 77.0 | 76.0 | 74.6 | 75.0 | 74.3 | 74.1 | 73.7 | 74.0 | 72.9 | 72.4 | 70.2 | 71.7 | 72.2 | 69.5 | 70.3 | 70.0 |
| | SE | 3.8 | 3.3 | 2.9 | 2.4 | 2.0 | 2.6 | 2.3 | 2.5 | 2.5 | 2.3 | 2.4 | 4.5 | 2.0 | 2.4 | 2.5 | 2.6 | 2.1 | 2.3 | 2.1 | 2.0 | 1.8 | 1.9 | 1.3 | 1.4 | 0.7 | 1.6 | 1.6 | 1.8 | 1.8 |
| HFD VC | Mean | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 |
| | SE | 4.7 | 5.3 | 5.7 | 5.7 | 5.7 | 5.4 | 5.4 | 5.6 | 5.7 | 5.6 | 5.3 | 5.9 | 5.8 | 6.3 | 6.4 | 6.3 | 6.2 | 6.5 | 6.6 | 6.1 | 5.5 | 6.1 | 5.8 | 5.4 | 5.8 | 5.7 | 5.7 | 5.8 | 5.8 |
| Semaglutide | Mean | 101.4 | 93.6 | 91.7 | 84.3 | 86.0 | 83.5 | 86.4 | 79.5 | 79.6 | 84.2 | 81.6 | 84.1 | 77.3 | 77.3 | 80.2 | 78.4 | 82.3 | 75.6 | 78.9 | 77.6 | 81.6 | 74.6 | 78.0 | 80.9 | 75.1 | 76.2 | 80.2 | 75.9 | 79.3 |
| | SE | 4.1 | 3.8 | 3.4 | 3.3 | 3.0 | 3.3 | 3.1 | 3.2 | 3.1 | 3.0 | 2.8 | 2.8 | 2.8 | 3.0 | 2.7 | 2.9 | 3.0 | 3.1 | 2.8 | 2.7 | 2.9 | 3.0 | 2.9 | 3.2 | 3.3 | 3.2 | 3.5 | 3.4 | 3.4 |
| OLX702A-031-2+sema | Mean | 101.9 | 93.4 | 89.4 | 80.4 | 79.9 | 75.0 | 76.7 | 69.9 | 69.7 | 72.8 | 70.0 | 73.2 | 65.8 | 67.1 | 69.4 | 69.1 | 71.7 | 64.7 | 69.6 | 68.3 | 72.0 | 63.4 | 67.7 | 69.6 | 63.6 | 64.1 | 68.9 | 63.4 | 67.0 |
| | SE | 6.0 | 5.7 | 4.8 | 4.0 | 3.4 | 2.0 | 1.7 | 1.6 | 1.6 | 1.4 | 1.2 | 1.5 | 1.2 | 1.3 | 1.6 | 1.2 | 1.4 | 1.5 | 1.3 | 1.4 | 1.3 | 1.8 | 1.4 | 1.6 | 1.5 | 1.8 | 2.0 | 2.1 | 2.0 |
| Group | DAY | D52 | D55 | D57 | D58 | D59 | D62 | D63 | D64 | D65 | D66 | D70 | D71 | D73 | D77 | D78 | D80 | D83 | D85 | D87 | D90 | D92 | D94 | D97 | D99 | D101 | D104 | D106 | D108 | D111 |
| Normal Chow VC | Mean | 70.2 | 67.9 | 68.7 | 68.5 | 67.6 | 67.9 | 68.4 | 68.9 | 68.6 | 68.6 | 66.7 | 68.7 | 69.2 | 68.7 | 69.2 | 66.9 | 67.6 | 68.3 | 68.0 | 67.5 | 68.4 | 68.3 | 68.0 | 68.1 | 67.2 | 67.1 | 66.8 | 68.1 | 68.6 |
| | SE | 1.5 | 1.7 | 1.7 | 1.2 | 1.3 | 1.7 | 2.5 | 2.3 | 1.9 | 1.9 | 1.5 | 1.6 | 2.1 | 2.2 | 2.2 | 1.3 | 2.1 | 1.7 | 2.0 | 2.3 | 1.8 | 1.8 | 2.4 | 2.6 | 2.4 | 2.2 | 2.2 | 2.2 | 2.1 |
| HFD VC | Mean | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 |
| | SE | 5.9 | 6.2 | 6.0 | 6.2 | 6.3 | 5.8 | 5.7 | 5.9 | 6.2 | 6.1 | 5.6 | 5.7 | 5.4 | 5.0 | 5.0 | 5.2 | 5.3 | 4.9 | 5.1 | 4.6 | 5.2 | 5.1 | 4.8 | 4.8 | 5.0 | 4.7 | 4.9 | 4.8 | 4.8 |
| Semaglutide | Mean | 74.9 | 81.3 | 76.3 | 80.0 | 83.4 | 88.3 | 89.8 | 90.0 | 91.1 | 92.7 | 96.3 | 96.9 | 99.0 | 100.8 | 100.3 | 101.2 | 101.9 | 102.4 | 102.9 | 104.8 | 104.8 | 105.3 | 105.9 | 105.3 | 105.5 | 105.8 | 105.4 | 105.1 | 105.0 |
| | SE | 3.4 | 3.5 | 3.6 | 3.4 | 3.9 | 4.0 | 4.2 | 3.9 | 3.7 | 3.7 | 3.0 | 2.9 | 2.7 | 2.5 | 2.0 | 1.8 | 1.5 | 1.5 | 1.6 | 1.4 | 1.0 | 1.4 | 1.3 | 1.4 | 1.6 | 1.7 | 1.5 | 1.6 | 1.9 |
| OLX702A-031-2+sema | Mean | 62.0 | 68.6 | 63.5 | 66.6 | 69.7 | 72.4 | 74.0 | 74.3 | 76.0 | 76.4 | 78.0 | 78.4 | 80.5 | 81.2 | 80.8 | 80.3 | 80.4 | 82.2 | 82.1 | 82.4 | 81.8 | 82.4 | 82.5 | 83.4 | 83.6 | 83.6 | 82.4 | 81.6 | 82.0 |
| | SE | 2.0 | 2.2 | 1.9 | 2.1 | 2.3 | 3.1 | 3.0 | 3.4 | 3.5 | 3.3 | 3.0 | 3.2 | 3.0 | 3.4 | 3.5 | 3.3 | 3.7 | 4.0 | 3.9 | 3.7 | 3.7 | 3.8 | 3.8 | 3.9 | 4.0 | 3.9 | 3.8 | 3.8 | 3.7 |

### 1-2. Comparison of dietary intake

Targeting the animal model groups of Experimental Example 1-1, measuring the dietary intake was conducted for 16 weeks, and along with the administration of the test substance, the dietary intake of each subject was measured twice a week from the start of the experiment (D0) until the 9$^{th}$ day, and then 5 times a week until the end of the experiment. The dietary intake was calculated by measuring the difference between the supply amount and the remaining amount for each subject, and the cumulative dietary intake during the entire experiment period, the average daily dietary intake during the period before discontinuation of the administration of semaglutide, and the average daily dietary intake during the entire experiment period were evaluated.

As a result, as shown in FIG. 12 and Table 14, in terms of the cumulative dietary intake, the group administered with OLX702A-031-2 according to an embodiment in combination with semaglutide (OLX702A-031-2+sema) showed no

difference from the control group and the group administered with only semaglutide (Semaglutide). Additionally, in terms of the average daily dietary intake, both the Semaglutide group and the OLX702A-031-2+sema group had reduced dietary intake compared to the control group until discontinuation of administration of semaglutide, and the dietary intake of the OLX702A-031-2+sema group showed similar levels regardless of discontinuous of the administration of semaglutide.

## [Table 14]

| Cumulative Food Intake (g/mouse) | | | | | | | | | | | | | | | | | | | | | | | | |
|---|---|---|---|---|---|---|---|---|---|---|---|---|---|---|---|---|---|---|---|---|---|---|---|---|
| Group | DAY | D0 | D3 | D7 | D9 | D10 | D13 | D14 | D15 | D16 | D17 | D20 | D21 | D22 | D23 | D24 | D27 | D28 | D29 | D30 | D31 | D34 | D35 | D36 | D37 |
| HFD VC | Mean | 0.0 | 6.7 | 15.2 | 20.0 | 22.0 | 28.7 | 30.9 | 33.2 | 35.3 | 37.5 | 44.9 | 47.3 | 50.0 | 52.7 | 55.3 | 63.0 | 65.7 | 68.3 | 70.8 | 73.3 | 81.0 | 83.3 | 85.9 | 88.2 |
| | SE | 0.0 | 0.6 | 0.9 | 1.1 | 1.2 | 1.4 | 1.5 | 1.6 | 1.7 | 1.8 | 1.9 | 2.1 | 2.2 | 2.4 | 2.5 | 2.7 | 2.8 | 2.9 | 2.9 | 3.1 | 3.2 | 3.0 | 3.1 | 3.1 |
| Semaglutide | Mean | 0.0 | 2.2 | 8.5 | 10.6 | 12.7 | 17.9 | 21.3 | 21.9 | 23.4 | 26.5 | 32.2 | 35.9 | 36.7 | 38.6 | 41.8 | 48.3 | 52.3 | 53.4 | 55.5 | 58.9 | 65.2 | 69.1 | 70.1 | 72.2 |
| | SE | 0.0 | 0.2 | 0.6 | 0.8 | 0.9 | 1.2 | 1.4 | 1.4 | 1.5 | 1.8 | 2.1 | 2.3 | 2.3 | 2.3 | 2.6 | 3.2 | 3.3 | 3.4 | 3.5 | 3.7 | 4.1 | 4.0 | 4.0 | 4.1 |
| OLX702A-031-2+sema | Mean | 0.0 | 2.5 | 7.9 | 9.5 | 11.1 | 15.3 | 18.1 | 19.1 | 20.8 | 24.0 | 30.3 | 34.5 | 35.4 | 37.8 | 40.8 | 47.8 | 51.6 | 52.5 | 55.0 | 58.4 | 65.0 | 68.8 | 69.6 | 71.8 |
| | SE | 0.0 | 0.2 | 0.5 | 0.7 | 0.8 | 1.5 | 1.7 | 1.7 | 1.8 | 2.0 | 2.4 | 2.5 | 2.5 | 2.7 | 2.8 | 3.2 | 3.2 | 3.1 | 3.3 | 3.3 | 3.5 | 3.5 | 3.5 | 3.6 |
| Group | DAY | D38 | D42 | D43 | D44 | D48 | D49 | D50 | D51 | D52 | D55 | D57 | D58 | D59 | D62 | D63 | D64 | D65 | D66 | D70 | D71 | D72 | D73 | D77 | D78 |
| HFD VC | Mean | 90.5 | 99.6 | 101.9 | 104.2 | 113.7 | 116.0 | 118.4 | 120.8 | 123.1 | 130.1 | 135.2 | 137.3 | 140.0 | 147.0 | 149.3 | 151.8 | 154.0 | 156.3 | 165.0 | 167.2 | 169.1 | 170.8 | 180.1 | 182.6 |
| | SE | 3.1 | 3.1 | 3.2 | 3.2 | 3.4 | 3.6 | 3.6 | 3.6 | 3.8 | 4.1 | 4.2 | 4.2 | 4.4 | 4.5 | 4.5 | 4.7 | 4.8 | 4.9 | 5.0 | 5.1 | 5.2 | 5.2 | 5.3 | 5.5 |
| Semaglutide | Mean | 75.0 | 85.1 | 86.1 | 88.2 | 98.6 | 99.6 | 101.8 | 105.0 | 106.1 | 115.1 | 118.3 | 121.5 | 125.9 | 137.3 | 140.8 | 144.2 | 147.6 | 150.9 | 163.1 | 166.1 | 169.2 | 171.8 | 183.8 | 186.5 |
| | SE | 4.2 | 4.2 | 4.2 | 4.3 | 4.5 | 4.5 | 4.5 | 4.5 | 4.5 | 4.7 | 4.8 | 4.8 | 4.9 | 5.2 | 5.2 | 5.3 | 5.5 | 5.5 | 5.7 | 5.8 | 6.0 | 6.0 | 6.2 | 6.4 |
| OLX702A-031-2+sema | Mean | 74.7 | 85.1 | 86.0 | 88.2 | 99.4 | 100.2 | 102.5 | 106.0 | 106.9 | 116.5 | 119.8 | 122.8 | 127.2 | 137.7 | 141.4 | 144.7 | 148.2 | 151.3 | 162.8 | 165.8 | 168.9 | 171.9 | 183.7 | 186.4 |
| | SE | 3.7 | 4.2 | 4.3 | 4.4 | 4.6 | 4.6 | 4.7 | 4.8 | 4.9 | 5.3 | 5.5 | 5.5 | 5.5 | 5.5 | 5.5 | 5.6 | 5.6 | 5.9 | 6.0 | 6.1 | 6.2 | 6.3 | 6.5 | 6.5 |
| Group | DAY | D79 | D80 | D83 | D84 | D85 | D86 | D87 | D90 | D91 | D92 | D93 | D94 | D97 | D98 | D99 | D100 | D101 | D104 | D105 | D106 | D107 | D108 | D111 |
| HFD VC | Mean | 184.9 | 187.2 | 194.0 | 195.9 | 198.5 | 200.7 | 203.1 | 210.4 | 212.8 | 215.1 | 217.3 | 219.6 | 227.0 | 229.6 | 232.3 | 234.9 | 237.6 | 245.2 | 247.6 | 250.8 | 253.2 | 255.5 | 263.1 |
| | SE | 5.5 | 5.7 | 6.2 | 6.2 | 6.3 | 6.5 | 6.5 | 6.8 | 7.1 | 7.3 | 7.4 | 7.5 | 7.7 | 7.8 | 7.8 | 7.9 | 8.0 | 8.1 | 8.2 | 8.3 | 8.3 | 8.3 | 8.3 |
| Semaglutide | Mean | 188.9 | 191.9 | 200.7 | 203.4 | 206.3 | 209.1 | 212.0 | 220.9 | 223.7 | 226.6 | 229.2 | 232.0 | 240.6 | 243.2 | 246.0 | 248.9 | 251.7 | 260.3 | 263.0 | 266.3 | 269.0 | 271.4 | 279.7 |
| | SE | 6.2 | 6.3 | 6.5 | 6.6 | 6.7 | 6.8 | 6.9 | 7.1 | 7.1 | 7.3 | 7.3 | 7.3 | 7.4 | 7.4 | 7.5 | 7.6 | 7.6 | 7.8 | 7.9 | 7.9 | 8.0 | 8.1 | 8.3 |
| OLX702A-031-2+sema | Mean | 189.1 | 191.7 | 200.6 | 203.6 | 207.0 | 210.0 | 212.9 | 221.7 | 224.5 | 227.4 | 230.4 | 233.4 | 242.4 | 245.4 | 248.9 | 252.2 | 255.3 | 264.4 | 267.1 | 270.4 | 273.2 | 275.7 | 284.7 |
| | SE | 6.5 | 6.7 | 6.8 | 6.9 | 7.0 | 7.1 | 7.2 | 7.4 | 7.4 | 7.5 | 7.5 | 7.7 | 7.6 | 7.6 | 7.7 | 7.8 | 7.9 | 8.3 | 8.4 | 8.5 | 8.6 | 8.7 | 8.9 |

Referring to the experimental results above, it was found that the improved body weight loss effect by combined administration with semaglutide, a GLP-1 receptor agonist, resulted from an increase in the energy consumption, not from a decrease in the food intake.

### Experimental Example 2. Confirmation of effect of combined administration of OLX702A-031-2 according to changes in the number of administration of semaglutide

In this experimental example, targeting the animal models with high-fat DIO, in the combined administration of OLX702A-031-2 and semaglutide, it was to be confirmed whether the number of administration of semaglutide affected the anti-obesity effect by the combined administration.

### 2-1. Comparison of changes in body weight in once-weekly administration group

Targeting C57BL/6 mice (8 weeks old, male, Koatech) provided with a high-fat diet for 8 weeks, the animal model groups were divided into, depending on a substance to be administered, a group administered with 1X PBS at 1-week intervals (Vehicle), a group administered with only semaglutide at 1-week intervals (Sema QW), a group administered with only OLX702A-031-2 according to an embodiment at 2-week intervals (OLX702A-031-2 Q2W), and a group administered with OLX702A-031-2 according to an embodiment in combination with semaglutide (OLX702A-031-2 Q2W+sema QW). Specific experimental conditions for the animal model groups are as shown in Table 15.

[Table 15]

| Group | Dose (mpk) | Route | Interval | Treatment | | Number |
|---|---|---|---|---|---|---|
| 1 | - | SC | QW | 60% HFD | 1X PBS | 8 |
| 2 | 0.42 | SC | QW | | semaglutide | 8 |
| 3 | 10 | SC | Q2W | | OLX702A-031-2 | 8 |
| 4 | 10/0.42 | SC | Q2W/QW | | OLX702A-031-2/ semaglutide | 8 |

On Week 8 after the high-fat diet was provided, the test substance was administered. In detail, the semaglutide was administered subcutaneously at a dose of 0.42 mpk a total of 6 times at 1-week intervals, and the OLX702A-031-2 was administered subcutaneously at a dose of 10 mpk a total of 3 times at 2-week intervals. This experiment was conducted for 6 weeks, and the body weight and the dietary intake were measured every day along with the administration of the test substance. The dietary intake was calculated by measuring the difference between the supply and the balance for each subject. Meanwhile, a group in which 1X PBS was administered to an obese animal model (Vehicle) was used as a control group, and a group in which semaglutide was administered subcutaneously at a dose of 0.42 mpk a total of 6 times at 1-week intervals (Sema QW) and a group in which OLX702A-031-2 was administered subcutaneously at a dose of 10 mpk a total of 3 times at 2-week intervals (OLX702A-031-2 Q2W) were used as positive control groups.

As a result, as shown in FIG. 13 and Table 16, under the condition of administering semaglutide once a week, a group administered with OLX702A-031-2 according to an embodiment in combination with semaglutide (OLX702A-031-2 +Semga QW) showed a significantly improved body weight loss effect compared to a single administration group.

## [Table 16]

Body weight change (%) from baseline/VC

| Group | Day | 0 | 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|---|---|---|
| Vehicle | Mean | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 |
| | SE | 0.00 | 0.16 | 0.25 | 0.45 | 0.63 | 0.90 | 0.77 | 0.55 | 0.55 | 0.93 | 1.14 | 1.29 | 1.16 | 1.00 | 1.22 | 1.06 | 1.24 | 1.26 | 1.22 | 1.25 | 1.39 |
| Sema QW | Mean | 0.0 | -7.5 | -9.4 | -7.9 | -6.3 | -4.5 | -4.4 | -4.0 | -12.2 | -12.9 | -12.2 | -9.5 | -5.7 | -7.6 | -7.0 | -14.6 | -17.1 | -16.8 | -13.2 | -12.5 | -12.3 |
| | SE | 0.00 | 0.13 | 0.32 | 0.35 | 0.25 | 0.45 | 0.48 | 0.47 | 0.47 | 0.54 | 0.72 | 0.75 | 0.50 | 0.67 | 1.25 | 0.90 | 1.04 | 0.95 | 0.90 | 1.06 | 1.17 |
| OLX702A-031-2 Q2W | Mean | 0.0 | -1.0 | -1.4 | -1.9 | -2.6 | -2.6 | -2.9 | -2.9 | -3.7 | -4.2 | -6.4 | -6.5 | -7.7 | -8.9 | -10.2 | -10.0 | -10.5 | -12.3 | -12.6 | -13.4 | -14.1 |
| | SE | 0.00 | 0.29 | 0.32 | 0.50 | 0.42 | 0.35 | 0.59 | 0.55 | 0.50 | 0.55 | 0.62 | 0.77 | 0.71 | 0.91 | 0.95 | 0.94 | 1.05 | 1.07 | 1.06 | 1.03 | 1.11 |
| OLX702A-031-2 Q2W + Sema QW | Mean | 0.0 | -7.7 | -9.5 | -9.0 | -6.7 | -6.5 | -6.3 | -7.5 | -16.9 | -17.5 | -19.5 | -15.1 | -19.0 | -19.6 | -20.4 | -29.5 | -33.2 | -31.1 | -25.4 | -25.7 | -25.7 |
| | SE | 0.00 | 0.26 | 0.36 | 0.41 | 0.54 | 0.66 | 0.62 | 0.90 | 0.51 | 0.90 | 1.13 | 1.40 | 1.23 | 1.51 | 1.54 | 1.56 | 1.62 | 1.63 | 1.71 | 1.65 | 1.75 |

| Group | Day | 21 | 22 | 23 | 24 | 25 | 26 | 27 | 28 | 29 | 30 | 31 | 32 | 33 | 34 | 35 | 36 | 37 | 38 | 39 | 40 | 41 |
|---|---|---|---|---|---|---|---|---|---|---|---|---|---|---|---|---|---|---|---|---|---|---|
| Vehicle | Mean | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 |
| | SE | 1.55 | 1.43 | 1.63 | 1.45 | 1.75 | 1.65 | 2.00 | 1.70 | 1.75 | 1.75 | 1.90 | 1.85 | 1.96 | 2.09 | 2.07 | 2.03 | 2.15 | 2.37 | 2.47 | 2.54 | 2.57 |
| Sema QW | Mean | -11.5 | -19.9 | -21.4 | -16.7 | -17.8 | -16.5 | -17.0 | -16.5 | -21.5 | -22.9 | -20.5 | -19.6 | -17.6 | -16.5 | -16.9 | -24.2 | -24.5 | -22.3 | -19.0 | -19.1 | -17.6 |
| | SE | 1.37 | 1.45 | 1.61 | 1.45 | 1.31 | 1.43 | 1.44 | 1.57 | 1.63 | 2.01 | 1.74 | 1.55 | 1.55 | 1.94 | 2.05 | 2.41 | 2.47 | 2.16 | 2.26 | 2.35 | 2.35 |
| OLX702A-031-2 Q2W | Mean | -16.1 | -16.1 | -17.7 | -17.6 | -19.2 | -20.0 | -21.0 | -21.2 | -21.5 | -22.0 | -22.2 | -22.5 | -22.6 | -23.2 | -23.7 | -23.4 | -24.1 | -23.3 | -23.3 | -23.7 | -23.9 |
| | SE | 1.12 | 1.24 | 1.36 | 1.47 | 1.49 | 1.65 | 1.77 | 2.09 | 2.23 | 2.16 | 2.43 | 2.25 | 2.29 | 2.09 | 1.92 | 1.76 | 1.55 | 1.43 | 1.47 | 1.39 | 1.19 |
| OLX702A-031-2 Q2W + Sema QW | Mean | -28.4 | -37.6 | -39.5 | -35.4 | -33.7 | -33.7 | -34.0 | -32.5 | -42.1 | -43.2 | -35.6 | -37.7 | -37.0 | -38.9 | -36.7 | -45.9 | -46.0 | -41.3 | -35.2 | -39.5 | -35.5 |
| | SE | 1.74 | 1.77 | 1.42 | 1.45 | 1.54 | 1.54 | 1.66 | 1.75 | 1.59 | 1.56 | 1.55 | 1.97 | 2.42 | 2.52 | 2.79 | 2.50 | 2.13 | 2.34 | 2.02 | 2.49 | 2.64 |

Also, as shown in FIG. 14 and Table 17, in terms of the cumulative dietary intake, the OLX702A-031-2+semaglutide QW group showed no difference from the other groups, indicating that the body weight loss effect resulted from an increase in the energy consumption.

## [Table 17]

Cumulative food intake (g/mouse)

| Group | Day | 0 | 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|---|---|---|
| Vehicle | Mean | 0.0 | 2.3 | 4.5 | 6.5 | 8.7 | 11.1 | 13.5 | 15.8 | 18.3 | 20.7 | 22.9 | 25.3 | 27.8 | 30.1 | 32.6 | 35.0 | 37.7 | 40.0 | 42.1 | 44.7 | 47.0 |
| | SE | 0.00 | 0.08 | 0.14 | 0.29 | 0.41 | 0.57 | 0.63 | 0.70 | 0.74 | 0.82 | 0.95 | 1.08 | 1.10 | 1.10 | 1.19 | 1.19 | 1.29 | 1.35 | 1.41 | 1.50 | 1.58 |
| Sema QW | Mean | 0.0 | 0.3 | 1.0 | 2.4 | 4.3 | 7.5 | 10.0 | 12.6 | 13.0 | 14.0 | 15.7 | 18.2 | 21.3 | 24.5 | 27.3 | 28.1 | 29.1 | 30.8 | 33.1 | 36.1 | 38.9 |
| | SE | 0.00 | 0.04 | 0.06 | 0.09 | 0.16 | 0.28 | 0.35 | 0.42 | 0.44 | 0.48 | 0.57 | 0.73 | 0.82 | 0.86 | 0.95 | 0.96 | 1.02 | 1.08 | 1.17 | 1.25 | 1.36 |
| OLX702A-031-2 Q2W | Mean | 0.0 | 2.0 | 3.9 | 5.8 | 7.5 | 9.8 | 11.6 | 13.6 | 15.7 | 17.4 | 19.3 | 21.3 | 23.6 | 25.5 | 27.7 | 30.2 | 32.9 | 34.5 | 36.6 | 39.1 | 41.0 |
| | SE | 0.00 | 0.08 | 0.15 | 0.24 | 0.25 | 0.26 | 0.35 | 0.33 | 0.34 | 0.37 | 0.41 | 0.49 | 0.55 | 0.61 | 0.64 | 0.65 | 0.77 | 0.84 | 0.88 | 0.93 | 1.02 |
| OLX702A-031-2 Q2W + Sema QW | Mean | 0.0 | 0.4 | 1.1 | 2.3 | 4.2 | 6.9 | 9.2 | 11.4 | 11.9 | 12.5 | 13.7 | 15.7 | 18.4 | 21.0 | 23.4 | 23.9 | 24.8 | 27.2 | 30.1 | 33.2 | 35.9 |
| | SE | 0.00 | 0.06 | 0.07 | 0.15 | 0.17 | 0.32 | 0.40 | 0.47 | 0.49 | 0.56 | 0.68 | 0.93 | 0.99 | 1.21 | 1.23 | 1.27 | 1.37 | 1.51 | 1.53 | 1.56 | 1.61 |

| Group | Day | 21 | 22 | 23 | 24 | 25 | 26 | 27 | 28 | 29 | 30 | 31 | 32 | 33 | 34 | 35 | 36 | 37 | 38 | 39 | 40 | 41 |
|---|---|---|---|---|---|---|---|---|---|---|---|---|---|---|---|---|---|---|---|---|---|---|
| Vehicle | Mean | 49.2 | 51.9 | 54.7 | 57.0 | 59.3 | 61.8 | 64.4 | 66.8 | 69.1 | 71.8 | 74.1 | 76.8 | 79.2 | 81.7 | 84.1 | 86.8 | 89.4 | 91.5 | 93.9 | 96.6 | 99.1 |
| | SE | 1.70 | 1.75 | 1.88 | 1.87 | 1.86 | 1.94 | 2.09 | 2.17 | 2.24 | 2.31 | 2.43 | 2.50 | 2.55 | 2.59 | 2.62 | 2.69 | 2.83 | 2.93 | 3.06 | 3.14 | 3.21 |
| Sema QW | Mean | 41.8 | 42.5 | 43.7 | 45.8 | 48.1 | 51.2 | 54.2 | 57.3 | 58.1 | 59.6 | 61.8 | 64.2 | 67.5 | 70.8 | 73.8 | 74.6 | 76.2 | 78.4 | 81.0 | 84.2 | 87.7 |
| | SE | 1.48 | 1.47 | 1.54 | 1.60 | 1.74 | 1.87 | 1.92 | 2.00 | 1.99 | 2.13 | 2.20 | 2.41 | 2.54 | 2.58 | 2.72 | 2.78 | 2.84 | 2.89 | 3.06 | 3.20 | 3.28 |
| OLX702A-031-2 Q2W | Mean | 43.0 | 45.2 | 47.5 | 49.7 | 51.7 | 54.0 | 56.4 | 58.5 | 60.7 | 63.2 | 65.5 | 68.1 | 70.5 | 72.9 | 75.3 | 78.2 | 80.4 | 83.0 | 85.7 | 88.5 | 91.1 |
| | SE | 1.00 | 1.11 | 1.19 | 1.21 | 1.28 | 1.37 | 1.47 | 1.54 | 1.61 | 1.69 | 1.77 | 1.87 | 1.96 | 1.91 | 1.90 | 1.91 | 1.93 | 2.01 | 2.09 | 2.25 | 2.31 |
| OLX702A-031-2 Q2W + Sema QW | Mean | 38.9 | 39.3 | 40.6 | 43.3 | 46.2 | 49.4 | 52.8 | 55.7 | 55.9 | 57.1 | 59.7 | 62.6 | 65.7 | 68.7 | 71.6 | 71.9 | 73.5 | 76.4 | 79.5 | 82.8 | 86.1 |
| | SE | 1.71 | 1.76 | 1.77 | 1.70 | 1.63 | 1.72 | 1.79 | 1.77 | 1.79 | 1.89 | 1.88 | 1.87 | 2.00 | 2.10 | 2.23 | 2.28 | 2.34 | 2.43 | 2.46 | 2.58 | 2.70 |

### 2-2. Comparison of changes in body weight in once-daily administration group

Targeting C57BL/6 mice (8 weeks old, male, Koatech) provided with a high-fat diet for 8 weeks, the animal model groups were divided into, depending on a substance to be administered, a group administered with 1X PBS at 1-week intervals (Vehicle), a group administered with only semaglutide at one-day intervals (Sema QD), a group administered with only OLX702A-031-2 according to an embodiment at 2-week intervals (OLX702A-031-2 Q2W), and a group administered with OLX702A-031-2 according to an embodiment in combination with semaglutide (OLX702A-031-2 Q2W+Sema QD).

Specific experimental conditions for the animal model groups are as shown in Table 18.

**[Table 18]**

| Group | Dose (mpk) | Route | Interval | Treatment | | Number |
|---|---|---|---|---|---|---|
| 1 | - | SC | QW | 60% HFD | 1X PBS | 8 |
| 2 | 0.15 | SC | QD | | semaglutide | 8 |
| 3 | 10 | SC | Q2W | | OLX702A-031-2 | 8 |
| 4 | 10/0.15 | SC | Q2W/QD | | OLX702A-031-2/ semaglutide | 8 |

At week 8 after the time the high-fat diet was provided, OLX702A-031-2 according to an embodiment was administered in combination with semaglutide. In detail, the semaglutide was administered subcutaneously at one day-interval in total at a dose of 0.15 mpk for a total of 14 times, and then the administration was discontinued for the remainder of the experiment period. The OLX702A-031-2 was administered subcutaneously at a dose of 10 mpk for a total of 3 times at 2-week intervals. This experiment was conducted for 6 weeks, and the body weight and the dietary intake were measured every day along with the administration of the test substance. The dietary intake was calculated by measuring the difference between the supply and the balance for each subject. Meanwhile, a group in which 1X PBS was administered to an obese animal model (Vehicle) was used as a control group, and a group in which semaglutide was administered subcutaneously at a dose of 0.15 mpk for a total of 14 times at one-day intervals and then stopped (Sema QD) and a group administered with OLX702A-031-2 subcutaneously at a dose of 10 mpk for a total of 3 times at 2-week intervals were used as positive control group.

As a result, as shown in FIG. 15 and Table 19, under the condition of administering semaglutide everyday, a group administered with OLX702A-031-2 according to an embodiment in combination with semaglutide (OLX702A-031-2 +Sema QD) showed an improved body weight loss effect compared to a single administration group. In particular, the group administered with semaglutide at one-day intervals (Sema QD) showed a rapid body weight gain after discontinuation of the administration of semaglutide, whereas the OLX702A-031-2+Sema QD group was confirmed to maintain the body weight loss effect continuously.

## [Table 19]

Body weight change from baseline (% of VC)

| Group | Day | 0 | 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|---|---|---|
| Vehicle | Mean | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 |
| | SE | 0.00 | 0.15 | 0.25 | 0.45 | 0.63 | 0.90 | 0.77 | 0.55 | 0.56 | 0.93 | 1.14 | 1.29 | 1.15 | 1.00 | 1.22 | 1.06 | 1.24 | 1.25 | 1.22 | 1.25 | 1.39 |
| OLX702A-031-2 Q2W | Mean | 0.0 | -1.0 | -1.4 | -1.9 | -2.6 | -2.6 | -2.9 | -2.9 | -3.7 | -4.2 | -6.4 | -6.5 | -7.7 | -5.9 | -10.2 | -10.0 | -10.5 | -12.3 | -12.6 | -13.4 | -14.1 |
| | SE | 0.00 | 0.29 | 0.32 | 0.50 | 0.42 | 0.35 | 0.59 | 0.55 | 0.50 | 0.55 | 0.62 | 0.77 | 0.71 | 0.91 | 0.95 | 0.94 | 1.05 | 1.07 | 1.08 | 1.03 | 1.11 |
| Sema QD 14d | Mean | 0.0 | -0.9 | -3.0 | -6.7 | -9.1 | -11.4 | -11.9 | -15.0 | -16.6 | -17.5 | -20.6 | -20.4 | -21.5 | -22.6 | -23.3 | -24.0 | -22.6 | -19.5 | -17.6 | -16.0 | -14.9 |
| | SE | 0.00 | 0.17 | 0.12 | 0.24 | 0.37 | 0.52 | 0.59 | 0.43 | 0.45 | 0.52 | 0.69 | 0.71 | 0.64 | 0.61 | 0.74 | 0.66 | 0.75 | 0.65 | 0.73 | 1.01 | 0.90 |
| OLX702A-031-2 Q2W + Sema QD 14d | Mean | 0.0 | -0.4 | -2.5 | -7.0 | -9.5 | -13.4 | -15.6 | -19.5 | -23.7 | -26.3 | -30.7 | -31.7 | -35.0 | -37.5 | -39.5 | -41.3 | -40.0 | -37.3 | -33.5 | -31.5 | -30.9 |
| | SE | 0.00 | 0.36 | 0.35 | 0.32 | 0.26 | 0.40 | 0.64 | 0.57 | 1.16 | 1.24 | 1.51 | 1.64 | 1.77 | 1.81 | 2.09 | 2.42 | 2.59 | 2.45 | 2.16 | 2.07 | 1.95 |

| Group | Day | 21 | 22 | 23 | 24 | 25 | 26 | 27 | 28 | 29 | 30 | 31 | 32 | 33 | 34 | 35 | 36 | 37 | 38 | 39 | 40 | 41 |
|---|---|---|---|---|---|---|---|---|---|---|---|---|---|---|---|---|---|---|---|---|---|---|
| Vehicle | Mean | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 |
| | SE | 1.55 | 1.43 | 1.63 | 1.45 | 1.78 | 1.68 | 2.00 | 1.70 | 1.78 | 1.75 | 1.90 | 1.56 | 1.96 | 2.09 | 2.07 | 2.03 | 2.15 | 2.37 | 2.47 | 2.54 | 2.67 |
| OLX702A-031-2 Q2W | Mean | -15.1 | -16.1 | -17.7 | -17.6 | -19.2 | -20.0 | -21.0 | -21.2 | -21.5 | -22.0 | -22.2 | -22.5 | -22.6 | -23.2 | -23.7 | -23.4 | -24.1 | -23.3 | -23.3 | -23.7 | -23.9 |
| | SE | 1.12 | 1.24 | 1.36 | 1.47 | 1.49 | 1.65 | 1.77 | 2.09 | 2.23 | 2.16 | 2.43 | 2.25 | 2.29 | 2.09 | 1.92 | 1.76 | 1.55 | 1.43 | 1.47 | 1.39 | 1.19 |
| Sema QD 14d | Mean | -13.6 | -12.4 | -13.0 | -12.1 | -12.6 | -12.2 | -13.0 | -11.3 | -11.2 | -11.7 | -11.2 | -11.7 | -10.0 | -10.2 | -10.5 | -5.7 | -9.4 | -5.2 | -5.0 | -6.3 | -7.6 |
| | SE | 1.17 | 1.23 | 1.25 | 1.41 | 1.45 | 1.46 | 1.31 | 1.62 | 1.35 | 1.40 | 1.37 | 1.56 | 1.56 | 1.75 | 1.65 | 1.46 | 1.50 | 1.41 | 1.41 | 1.60 | 1.46 |
| OLX702A-031-2 Q2W + Sema QD 14d | Mean | -29.5 | -25.0 | -25.8 | -26.0 | -25.5 | -25.5 | -29.3 | -25.6 | -27.7 | -25.2 | -27.3 | -27.9 | -27.6 | -27.5 | -27.5 | -27.0 | -27.7 | -27.3 | -27.1 | -26.5 | -25.6 |
| | SE | 1.51 | 1.99 | 1.93 | 1.91 | 1.72 | 1.79 | 1.59 | 1.57 | 1.61 | 1.59 | 1.64 | 1.47 | 1.63 | 1.56 | 1.40 | 1.44 | 1.36 | 1.26 | 1.24 | 1.22 | 1.34 |

Also, as shown in FIG. 16 and Table 20, in terms of the cumulative dietary intake, the OLX702A-031-2+Sema QD group showed no difference from the other groups as described above, indicating that the body weight loss effect resulted from an increase in the energy consumption.

## [Table 20]

Cumulative food intake (g/mouse)

| Group | Day | 0 | 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|---|---|---|
| Vehicle | Mean | 0.0 | 2.3 | 4.5 | 6.5 | 8.7 | 11.1 | 13.5 | 15.8 | 18.3 | 20.7 | 22.9 | 25.3 | 27.8 | 30.1 | 32.6 | 35.0 | 37.7 | 40.0 | 42.1 | 44.7 | 47.0 |
| | SE | 0.00 | 0.08 | 0.14 | 0.29 | 0.41 | 0.57 | 0.63 | 0.70 | 0.74 | 0.82 | 0.95 | 1.08 | 1.10 | 1.10 | 1.19 | 1.19 | 1.29 | 1.35 | 1.41 | 1.50 | 1.58 |
| OLX702A-031-2 Q2W | Mean | 0.0 | 2.0 | 3.9 | 5.8 | 7.5 | 9.8 | 11.6 | 13.6 | 15.7 | 17.4 | 19.3 | 21.3 | 23.6 | 25.5 | 27.7 | 30.2 | 32.9 | 34.5 | 36.6 | 39.1 | 41.0 |
| | SE | 0.00 | 0.08 | 0.15 | 0.24 | 0.25 | 0.26 | 0.35 | 0.33 | 0.34 | 0.37 | 0.41 | 0.49 | 0.55 | 0.61 | 0.64 | 0.65 | 0.77 | 0.84 | 0.88 | 0.93 | 1.02 |
| Sema QD 14d | Mean | 0.0 | 1.9 | 3.4 | 4.3 | 4.9 | 5.8 | 6.7 | 7.5 | 8.8 | 9.8 | 11.1 | 12.4 | 13.9 | 15.5 | 17.0 | 18.7 | 21.1 | 23.8 | 27.0 | 30.8 | 33.9 |
| | SE | 0.00 | 0.05 | 0.04 | 0.13 | 0.17 | 0.23 | 0.26 | 0.30 | 0.35 | 0.36 | 0.42 | 0.44 | 0.50 | 0.48 | 0.55 | 0.52 | 0.48 | 0.54 | 0.60 | 0.66 | 0.72 |
| OLX702A-031-2 Q2W + Sema QD 14d | Mean | 0.0 | 2.2 | 3.5 | 4.2 | 4.6 | 5.5 | 6.2 | 6.8 | 7.6 | 8.5 | 9.2 | 10.3 | 11.8 | 13.1 | 14.9 | 16.4 | 18.8 | 21.4 | 25.0 | 28.6 | 31.8 |
| | SE | 0.00 | 0.16 | 0.18 | 0.21 | 0.24 | 0.26 | 0.27 | 0.26 | 0.30 | 0.39 | 0.57 | 0.71 | 0.84 | 1.03 | 1.05 | 1.12 | 1.27 | 1.31 | 1.31 | 1.38 | 1.33 |

| Group | Day | 21 | 22 | 23 | 24 | 25 | 26 | 27 | 28 | 29 | 30 | 31 | 32 | 33 | 34 | 35 | 36 | 37 | 38 | 39 | 40 | 41 |
|---|---|---|---|---|---|---|---|---|---|---|---|---|---|---|---|---|---|---|---|---|---|---|
| Vehicle | Mean | 49.2 | 51.9 | 54.7 | 57.0 | 59.3 | 61.8 | 64.4 | 66.8 | 69.1 | 71.8 | 74.1 | 76.8 | 79.2 | 81.7 | 84.1 | 86.8 | 89.4 | 91.5 | 93.9 | 96.6 | 99.1 |
| | SE | 1.70 | 1.75 | 1.88 | 1.87 | 1.86 | 1.94 | 2.09 | 2.17 | 2.24 | 2.31 | 2.43 | 2.50 | 2.55 | 2.59 | 2.62 | 2.69 | 2.83 | 2.93 | 3.06 | 3.14 | 3.21 |
| OLX702A-031-2 Q2W | Mean | 43.0 | 45.2 | 47.5 | 49.7 | 51.7 | 54.0 | 56.4 | 58.5 | 60.7 | 63.2 | 65.5 | 68.1 | 70.5 | 72.9 | 75.3 | 78.2 | 80.4 | 83.0 | 85.7 | 88.5 | 91.1 |
| | SE | 1.00 | 1.11 | 1.19 | 1.21 | 1.28 | 1.37 | 1.47 | 1.54 | 1.61 | 1.69 | 1.77 | 1.87 | 1.96 | 1.91 | 1.90 | 1.91 | 1.93 | 2.01 | 2.09 | 2.25 | 2.31 |
| Sema QD 14d | Mean | 36.9 | 40.2 | 43.3 | 46.0 | 48.4 | 51.1 | 53.7 | 56.3 | 58.9 | 61.4 | 63.9 | 66.4 | 69.4 | 72.0 | 74.5 | 77.6 | 80.2 | 82.6 | 84.8 | 87.9 | 90.5 |
| | SE | 0.83 | 0.84 | 0.92 | 1.01 | 1.06 | 1.14 | 1.15 | 1.16 | 1.27 | 1.37 | 1.47 | 1.53 | 1.60 | 1.69 | 1.75 | 1.71 | 1.73 | 1.73 | 1.77 | 1.85 | 1.91 |
| OLX702A-031-2 Q2W + Sema QD 14d | Mean | 34.6 | 38.0 | 41.1 | 43.6 | 46.0 | 48.4 | 50.8 | 53.0 | 55.3 | 57.9 | 60.2 | 63.0 | 65.5 | 68.0 | 70.4 | 73.2 | 75.5 | 78.0 | 80.0 | 82.7 | 85.1 |
| | SE | 1.54 | 1.68 | 1.75 | 1.81 | 1.72 | 1.76 | 1.71 | 1.68 | 1.63 | 1.65 | 1.69 | 1.91 | 1.94 | 2.02 | 2.15 | 2.16 | 2.18 | 2.17 | 2.21 | 2.23 | 2.22 |

Referring to the experimental results above, it was found that the improved body weight loss effect by the combined administration confirmed in Experimental Example 1 was consistently exhibited, regardless of the number of doses of semaglutide administered.

### Experimental Example 3. Confirmation of body weight loss by combined administration of OLX702A-031-2 and tirzepatide

In this Experimental Example, targeting the animal model with high-fat DIO, the anti-obesity effect was evaluated by measuring changes in the body weight and food intake by combined administration of OLX702A-031-2 and tirzepatide, a GLP-1/GIP receptor dual agonist.

#### 3-1. Comparison of body weight changes

Targeting C57BL/6 mice (7 weeks old, male, Koatech) provided with a high-fat diet for 9 weeks, the animal model groups were divided into a group provided with a normal diet (NCD) and a group provided with a high-fat diet for 9 weeks (HFD), and depending on a substance to be administered, the animal model groups provided with a high-fat diet were divided into a group administered with 1X PBS (HFD VC), a group administered with only tirzepatide at a dose of 10 nmol/kg (Tirzepatide 10 nmol), a group administered with only tirzepatide at a dose of 100 nmol/kg (Tirzepatide 100 nmol), and a group administered with OLX702A-031-2 according to an embodiment at a dose of 5 mpk in combination with tirzepatide (OLX702A-031-2+TZP 10 nmnol). Specific experimental conditions for the animal model groups are as shown in Table 21.

**[Table 21]**

<table>
<tr><th>Group</th><th>Dose</th><th>Route</th><th>Interval</th><th colspan="2">Treatment</th><th>Number</th></tr>
<tr><td>1</td><td>-</td><td>SC</td><td>BIW</td><td>NCD</td><td>1X PBS</td><td>4</td></tr>
<tr><td>2</td><td>-</td><td>SC</td><td>BIW</td><td rowspan="4">60% HFD</td><td>1X PBS</td><td>5</td></tr>
<tr><td>3</td><td>10 nmol/kg</td><td>SC</td><td>BIW</td><td>tirzepatide</td><td>4</td></tr>
<tr><td>4</td><td>100 nmol/kg</td><td>SC</td><td>BIW</td><td>tirzepatide</td><td>4</td></tr>
<tr><td>5</td><td>5 mpk/ 10 nmol/kg</td><td>SC</td><td>Q2W/BIW</td><td>OLX702A-031-2/ tirzepatide</td><td>4</td></tr>
</table>

At week 9 from the time the high-fat diet was provided, OLX702A-031-2 according to an embodiment was administered in combination with tirzepatide. Specifically, the tirzepatide was administered subcutaneously twice a week at a dose of 10 nmol/kg for a total of 8 times, and then the administration was discontinued for the remainder of the experiment period. The OLX702A-031-2 was administered subcutaneously at a dose of 5 nmol/kg for a total of 4 times at 2-week intervals. This experiment was conducted for 9 weeks, and the body weight of each subject was measured 5 times a week along with the administration of the test substance. Meanwhile, a group in which 1X PBS was administered to an obese animal model (HFD VC) was used as a control group, and a group in which tirzepatide was administered subcutaneously at a dose of 10 nmol/kg twice a week for a total of 8 times (Tirzepatide 10 nmol ) and a group in which tirzepatide was administered subcutaneously at a dose of 100 nmol/kg twice a week for a total of 8 times (tirzepatide 100 nmol) were used as comparison groups.

As a result, as shown in FIG. 17 and Table 22, the groups administered with only tirzepatide (Tirzepatide 10 nmol and Tirzepatide 100 nmol) showed a significant body weight loss effect during the administration period, but showed a rapid increase in body weight after discontinuation of the administration of tirzepatide. Meanwhile, the group administered

with OLX702A-031-2 according to an embodiment in combination with tirzepatide (OLX702A-031-2+TZP 10 nmol) not only showed a similar level of body weight loss as the group administered with a high dose of tirzepatide (Tirzepatide 100 nmol), but also confirmed that this body weight loss effect was maintained during the administration period of tirzepatide as well as during the period when the administration of tirzepatide was discontinued.

## [Table 22]

| Relative Body Weight to HFD VC (%) | | | | | | | | | | | | | | | | | | | | | | |
|---|---|---|---|---|---|---|---|---|---|---|---|---|---|---|---|---|---|---|---|---|---|---|
| Group | DAY | D0 | D1 | D2 | D3 | D6 | D7 | D8 | D9 | D10 | D13 | D14 | D15 | D16 | D17 | D20 | D21 | D22 | D23 | D24 | D28 | D29 |
| Normal Chow VC | Mean | 62.3 | 61.1 | 62.7 | 63.9 | 60.9 | 62.3 | 61.8 | 61.9 | 61.7 | 61.3 | 61.9 | 61.5 | 61.1 | 60.8 | 58.5 | 60.7 | 59.8 | 59.7 | 59.5 | 58.3 | 59.6 |
| | SE | 1.5 | 1.4 | 1.3 | 0.9 | 1.2 | 1.2 | 1.2 | 1.1 | 1.3 | 1.4 | 1.6 | 1.5 | 1.4 | 1.4 | 1.4 | 1.5 | 1.7 | 1.8 | 1.3 | 1.5 | 1.3 |
| HFD VC | Mean | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 |
| | SE | 2.7 | 3.0 | 3.0 | 3.1 | 3.3 | 3.2 | 3.4 | 3.3 | 3.5 | 3.6 | 3.6 | 3.8 | 3.7 | 3.6 | 3.5 | 3.3 | 3.3 | 3.2 | 3.0 | 3.4 | 3.2 |
| Tirzepatide 10 nmol | Mean | 100.1 | 91.3 | 90.1 | 91.5 | 81.8 | 83.7 | 77.7 | 77.2 | 78.0 | 74.2 | 76.4 | 70.6 | 70.5 | 71.6 | 68.9 | 70.0 | 64.9 | 65.9 | 67.4 | 69.0 | 71.8 |
| | SE | 3.5 | 3.2 | 2.9 | 2.4 | 2.2 | 2.6 | 2.7 | 2.7 | 3.2 | 3.2 | 3.5 | 3.3 | 3.6 | 3.0 | 3.0 | 3.3 | 3.5 | 3.1 | 3.0 | 3.5 | 3.7 |
| Tirzepatide 100 nmol | Mean | 100.1 | 90.7 | 87.5 | 85.2 | 71.3 | 70.6 | 65.8 | 63.5 | 62.4 | 61.6 | 64.5 | 60.2 | 58.3 | 60.5 | 58.8 | 61.4 | 56.9 | 56.8 | 59.4 | 60.6 | 65.1 |
| | SE | 3.0 | 3.2 | 3.1 | 3.2 | 2.5 | 2.4 | 2.0 | 2.1 | 2.9 | 2.0 | 2.0 | 1.7 | 1.8 | 1.9 | 2.3 | 2.0 | 1.2 | 0.9 | 0.8 | 1.4 | 2.0 |
| OLX702A-031-2+TZP 10 nmol | Mean | 100.1 | 91.7 | 90.8 | 91.4 | 78.5 | 78.8 | 71.4 | 68.6 | 67.1 | 59.3 | 60.1 | 55.6 | 56.9 | 59.4 | 59.0 | 61.3 | 55.7 | 57.5 | 59.9 | 61.5 | 64.2 |
| | SE | 3.1 | 2.8 | 3.0 | 3.3 | 3.0 | 2.7 | 2.7 | 2.9 | 2.4 | 3.5 | 4.3 | 3.4 | 3.0 | 2.5 | 1.8 | 1.7 | 2.0 | 1.9 | 2.0 | 2.5 | 1.7 |
| Group | DAY | D31 | D32 | D35 | D36 | D37 | D38 | D41 | D42 | D43 | D44 | D45 | D48 | D49 | D50 | D51 | D52 | D55 | D56 | D57 | D58 | D59 |
| Normal Chow VC | Mean | 58.3 | 58.5 | 58.2 | 58.3 | 57.5 | 58.0 | 57.1 | 57.4 | 57.9 | 57.4 | 57.5 | 57.1 | 56.5 | 56.9 | 57.3 | 57.6 | 57.4 | 56.9 | 57.0 | 57.0 | 57.6 |
| | SE | 1.4 | 1.7 | 1.6 | 1.3 | 1.4 | 1.4 | 1.4 | 1.5 | 1.7 | 1.5 | 1.5 | 1.4 | 1.4 | 1.2 | 1.1 | 1.2 | 1.2 | 1.3 | 1.4 | 1.7 | 1.4 |
| HFD VC | Mean | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 |
| | SE | 3.0 | 2.9 | 2.7 | 2.5 | 2.4 | 2.3 | 2.1 | 2.3 | 2.6 | 2.4 | 2.6 | 2.4 | 3.0 | 2.7 | 2.7 | 2.8 | 2.5 | 2.7 | 2.6 | 2.7 | 2.8 |
| Tirzepatide 10 nmol | Mean | 74.7 | 76.7 | 78.8 | 80.2 | 81.0 | 81.8 | 84.4 | 85.1 | 85.1 | 85.6 | 86.4 | 87.8 | 87.9 | 88.5 | 88.5 | 88.8 | 90.9 | 91.0 | 90.9 | 91.8 | 92.3 |
| | SE | 3.2 | 3.8 | 3.7 | 3.2 | 3.3 | 3.2 | 3.6 | 3.4 | 3.5 | 3.6 | 3.8 | 3.9 | 3.4 | 3.8 | 3.9 | 3.7 | 3.7 | 3.5 | 3.7 | 3.7 | 3.8 |
| Tirzepatide 100 nmol | Mean | 68.9 | 71.7 | 73.4 | 75.1 | 76.4 | 76.9 | 79.5 | 79.6 | 81.0 | 81.6 | 82.2 | 84.2 | 84.9 | 85.5 | 86.1 | 86.0 | 88.7 | 88.7 | 89.5 | 91.5 | 92.1 |
| | SE | 2.8 | 3.1 | 3.6 | 3.6 | 3.8 | 4.2 | 4.6 | 4.7 | 4.7 | 4.8 | 4.9 | 4.8 | 4.9 | 4.7 | 5.1 | 5.2 | 5.3 | 5.2 | 5.3 | 5.4 | 5.4 |
| OLX702A-031-2+TZP 10 nmol | Mean | 66.6 | 68.8 | 69.4 | 70.6 | 71.3 | 72.1 | 72.7 | 73.0 | 73.4 | 73.8 | 74.1 | 74.8 | 74.7 | 75.0 | 75.3 | 75.1 | 75.6 | 75.7 | 76.2 | 77.4 | 77.3 |
| | SE | 1.8 | 2.1 | 2.1 | 2.2 | 2.4 | 2.6 | 2.7 | 2.7 | 2.7 | 2.8 | 2.7 | 2.8 | 3.0 | 3.2 | 3.1 | 3.0 | 3.6 | 3.9 | 3.7 | 4.0 | 4.0 |

### 3-2. Comparison of dietary intake

Targeting the animal model groups of Experimental Example 3-1, measuring the dietary intake was performed for 9 weeks, and along with the administration of the test substance, the dietary intake of each subject was measured 5 times a week. The dietary intake was calculated by measuring the difference between the supply amount and the remaining amount for each subject, and the cumulative dietary intake during the entire experiment period, the average daily dietary intake during the period before discontinuation of the administration of tirzepatide, and the average daily dietary intake during the entire experiment period were evaluated.

As a result, as shown in FIGS. 18A to 18C and Table 23, in terms of the cumulative dietary intake, the group administered with OLX702A-031-2 according to an embodiment in combination with tirzepatide (OLX702A-031-2+TZP 10 nmol) showed no difference from the control group and the groups administered with only tirzepatide (Tirzepatide 10 nmol and Tirzepatide 100 nmol). Also, in terms of the average daily dietary intake, the OLX702A-031-2+TZP 10 nmol group showed a rather higher level than the other groups during the period before the discontinuation of the administration of tirzepatide, and a similar level with the other groups during the entire experiment period.

[Table 23]

Cumulative Food Intake (g)

| Group | DAY | D0 | D1 | D2 | D3 | D6 | D7 | D8 | D9 | D10 | D13 | D14 | D15 | D16 | D17 | D20 | D21 | D22 | D23 | D24 | D28 | D29 | D31 |
|---|---|---|---|---|---|---|---|---|---|---|---|---|---|---|---|---|---|---|---|---|---|---|---|
| HFD VC | Mean | 0.0 | 3.4 | 5.8 | 8.4 | 18.2 | 20.6 | 24.1 | 26.7 | 29.7 | 38.2 | 40.9 | 43.8 | 46.9 | 49.9 | 59.3 | 61.9 | 65.1 | 68.1 | 71.2 | 83.9 | 86.6 | 93.7 |
| | SE | 0.0 | 0.2 | 0.3 | 0.4 | 0.9 | 1.0 | 1.2 | 1.3 | 1.4 | 1.8 | 1.8 | 2.0 | 2.1 | 2.1 | 2.4 | 2.4 | 2.6 | 2.6 | 2.7 | 3.4 | 3.5 | 3.6 |
| Tirzepatide 10 nmol | Mean | 0.0 | 0.4 | 1.2 | 2.7 | 5.7 | 8.6 | 9.7 | 11.8 | 14.9 | 20.2 | 23.5 | 24.4 | 26.5 | 29.4 | 36.0 | 39.2 | 40.1 | 43.1 | 46.5 | 58.7 | 62.5 | 71.7 |
| | SE | 0.0 | 0.1 | 0.3 | 0.4 | 0.8 | 0.8 | 0.8 | 0.9 | 1.2 | 1.3 | 1.4 | 1.5 | 1.8 | 2.1 | 2.8 | 2.8 | 2.8 | 3.5 | 3.7 | 4.2 | 4.2 | 4.3 |
| Tirzepatide 100 nmol | Mean | 0.0 | 0.1 | 0.1 | 0.3 | 0.8 | 2.2 | 2.6 | 3.3 | 5.0 | 10.3 | 13.0 | 14.2 | 16.1 | 18.6 | 24.5 | 27.2 | 28.3 | 30.3 | 33.3 | 43.8 | 47.9 | 57.5 |
| | SE | 0.0 | 0.0 | 0.0 | 0.1 | 0.2 | 0.8 | 1.2 | 1.5 | 1.9 | 1.8 | 1.9 | 1.7 | 1.7 | 1.8 | 1.8 | 1.8 | 1.8 | 1.7 | 1.7 | 1.2 | 1.3 | 1.2 |
| OLX702A-031-2+TZP 10 nmol | Mean | 0.0 | 0.7 | 2.0 | 3.9 | 9.2 | 11.8 | 12.7 | 13.9 | 16.2 | 21.8 | 24.6 | 25.9 | 29.0 | 32.4 | 41.2 | 44.4 | 45.3 | 48.2 | 51.6 | 65.4 | 69.5 | 78.2 |
| | SE | 0.0 | 0.1 | 0.6 | 0.9 | 1.8 | 1.8 | 1.8 | 1.9 | 2.1 | 3.4 | 3.8 | 3.8 | 4.0 | 4.0 | 4.5 | 4.5 | 4.2 | 4.8 | 5.0 | 5.4 | 5.3 | 5.2 |

| Group | DAY | D32 | D35 | D36 | D37 | D38 | D41 | D42 | D43 | D44 | D45 | D48 | D49 | D50 | D51 | D52 | D55 | D56 | D57 | D58 | D59 | D62 | D63 |
|---|---|---|---|---|---|---|---|---|---|---|---|---|---|---|---|---|---|---|---|---|---|---|---|
| HFD VC | Mean | 100.1 | 106.2 | 109.4 | 112.7 | 115.7 | 124.8 | 127.9 | 130.9 | 134.1 | 137.1 | 146.5 | 149.9 | 153.2 | 155.9 | 158.8 | 168.0 | 170.9 | 174.4 | 177.5 | 180.7 | 190.6 | 193.9 |
| | SE | 3.7 | 4.0 | 4.0 | 4.0 | 4.1 | 4.4 | 4.6 | 4.8 | 4.9 | 5.0 | 5.5 | 5.7 | 5.8 | 5.9 | 6.0 | 6.3 | 6.3 | 6.5 | 6.8 | 6.9 | 7.7 | 7.9 |
| Tirzepatide 10 nmol | Mean | 79.8 | 87.1 | 91.2 | 95.1 | 98.6 | 109.3 | 112.6 | 116.0 | 119.5 | 122.8 | 132.3 | 135.7 | 139.0 | 141.8 | 144.8 | 154.0 | 157.2 | 160.3 | 163.5 | 166.7 | 175.8 | 178.6 |
| | SE | 4.2 | 4.1 | 4.0 | 4.0 | 4.0 | 3.9 | 3.9 | 3.9 | 3.9 | 3.8 | 3.6 | 3.6 | 3.6 | 3.5 | 3.5 | 3.6 | 3.7 | 3.6 | 3.6 | 3.6 | 3.8 | 3.9 |
| Tirzepatide 100 nmol | Mean | 66.3 | 74.0 | 78.5 | 82.6 | 86.3 | 97.1 | 100.7 | 104.3 | 108.0 | 111.4 | 121.4 | 125.1 | 128.5 | 131.4 | 134.2 | 143.9 | 147.2 | 150.7 | 154.2 | 157.6 | 167.0 | 170.3 |
| | SE | 1.5 | 2.0 | 2.1 | 2.3 | 2.4 | 3.0 | 3.3 | 3.5 | 3.6 | 3.8 | 4.1 | 4.2 | 4.2 | 4.4 | 4.6 | 4.7 | 4.7 | 4.9 | 5.0 | 5.1 | 5.4 | 5.3 |
| OLX702A-031-2+TZP 10 nmol | Mean | 85.9 | 92.4 | 96.3 | 99.9 | 103.3 | 112.7 | 115.6 | 118.9 | 122.2 | 125.3 | 134.3 | 137.6 | 140.8 | 143.3 | 146.4 | 155.7 | 158.9 | 162.6 | 166.2 | 169.5 | 179.9 | 183.0 |
| | SE | 5.5 | 5.7 | 5.9 | 6.1 | 6.3 | 6.5 | 6.5 | 6.6 | 6.7 | 6.6 | 6.9 | 6.9 | 7.2 | 7.3 | 7.3 | 7.7 | 7.9 | 7.9 | 8.1 | 8.1 | 8.4 | 8.7 |

Referring to the experimental results above, it was found that the improved body weight loss effect by combined administration with tirzepatide, a GLP-1/GIP receptor dual agonist, resulted from an increase in the energy consumption, not from a decrease in the food intake.

### III. Confirmation of body weight loss by combined administration of OLX702A-075-16 with semaglutide using a primate animal model

### Experimental Example 1. Comparison of changes in body weight by combined administration of OLX702A-075-16

In this Experimental Example, the anti-obesity effect was evaluated by measuring changes in the body weight and food intake by combined administration of OLX702A-075-16 and semaglutide, a GLP-1 receptor agonist, targeting a primate animal model in which obesity was induced by a high-fat diet.

#### 1-1. Comparison of body weight changes

Targeting obese monkey models (12 to 23 years old, male) provided with a high-fat diet for more than 2 years, the animal model groups were divided into, depending on a substance to be administered, a group administered with 1X PBS (Vehicle), a group administered with only semaglutide (Semaglutide), a group administered with only OLX702A-075-16 according to an embodiment (OLX702A-075-16), and a group administered with OLX702A-075-16 according to an embodiment in combination of semaglutide (OLX702A -075-16+Sema). Specific experimental conditions for the animal model groups are as shown in Table 24.

**[Table 24]**

| Group | Dose | Route | Interval | Treatment | | Number |
|---|---|---|---|---|---|---|
| 1 | - | SC | QW | HFD | 1X PBS | 2 |
| 2 | 30 μg/kg | SC | QW | | semaglutide | 4 |
| 3 | 10 mpk | SC | Q2W | | OLX702A-075-16 | 4 |
| 4 | 10 mpk/ 30 μg/kg | SC | Q2W/QW | | OLX702A-075-16/ semaglutide | 4 |

Targeting obese monkey animal models provided with a high-fat diet for more than 2 years, OLX702A-075-16 according to an embodiment was administered in combination with semaglutide. In detail, the semaglutide was administered subcutaneously at 1 week-intervals at a dose of 30 μg/kg for a total of 4 times, and then the administration was discontinued for the remainder of the experiment period. The OLX702A-075-16 was administered subcutaneously at a dose of 10 mpk for a total of 3 times at 2-week intervals. This experiment was conducted for 12 weeks, and the body weight of each subject was measured twice a week along with the administration of the test substance. Meanwhile, a group in which 1X PBS was administered to an obese animal model (Vehicle) was used as a control group, and a group in which semaglutide was administered subcutaneously at a dose of 30 μg/kg for a total of 4 times at 1-week intervals (Semaglutide) and a group in which OLX702A-075-16 was administered subcutaneously at a dose of 10 mpk for a total of 2 times at 2-week intervals (OLX702A-075-16) were used as comparison groups.

Table 25 shows the results of relative body weight change (%) over time for each group, compared to the day the test substance was administered (Base).

## [Table 25]

| BW change (% change of baseline) | | | | | | | | | | | | | | | | | | | | | | | | |
|---|---|---|---|---|---|---|---|---|---|---|---|---|---|---|---|---|---|---|---|---|---|---|---|---|
| Group | Day | Base | D3 | D7 | D10 | D14 | D17 | D21 | D24 | D28 | D31 | D35 | D38 | D42 | D45 | D49 | D52 | D56 | D63 | D66 | D70 | D73 | D77 | D80 | D84 |
| Vehicle | Mean | 0.0 | -1.2 | -0.8 | 0.2 | -0.1 | 1.4 | 1.0 | 0.1 | 0.5 | 0.9 | 0.2 | 0.6 | 0.4 | 0.9 | 0.8 | 1.0 | 0.7 | -0.8 | -1.4 | -0.3 | 0.2 | -0.2 | 0.0 | 0.1 |
| | SE | 0.0 | 0.8 | 0.5 | 0.6 | 0.2 | 0.9 | 0.9 | 0.3 | 0.9 | 1.3 | 1.6 | 1.5 | 2.4 | 2.7 | 2.6 | 2.9 | 2.7 | 1.9 | 1.3 | 2.5 | 2.4 | 2.7 | 3.0 | 3.1 |
| semaglutide | Mean | 0.0 | -4.0 | -5.2 | -6.7 | -8.5 | -10.5 | -12.3 | -13.4 | -15.6 | -15.9 | -16.4 | -17.3 | -17.4 | -17.7 | -16.8 | -16.8 | -15.9 | -15.4 | -14.4 | -13.9 | -12.8 | -12.8 | -12.2 | -11.4 |
| | SE | 0.0 | 1.4 | 1.1 | 1.2 | 1.2 | 1.5 | 1.7 | 1.7 | 2.2 | 2.1 | 2.3 | 2.0 | 1.8 | 1.9 | 1.9 | 2.1 | 2.0 | 2.0 | 2.2 | 2.1 | 2.4 | 2.2 | 2.3 | 2.6 |
| OLX702A-075-16 | Mean | 0.0 | 0.4 | -0.2 | -0.3 | -0.6 | 0.0 | -0.2 | -0.7 | -0.9 | -1.0 | -1.6 | -1.3 | -2.7 | -3.0 | -2.9 | -2.9 | -3.1 | -3.6 | -4.0 | -3.3 | -3.4 | -3.7 | -3.2 | -3.4 |
| | SE | 0.0 | 0.2 | 0.2 | 0.7 | 1.1 | 1.1 | 1.6 | 1.9 | 2.2 | 2.1 | 2.7 | 2.5 | 2.5 | 2.5 | 2.6 | 2.6 | 2.9 | 2.7 | 2.9 | 2.9 | 3.2 | 3.2 | 3.1 | 3.4 |
| semaglutide +OLX702A-075-16 | Mean | 0.0 | -3.8 | -5.6 | -7.9 | -9.4 | -11.8 | -13.4 | -15.0 | -17.6 | -18.4 | -19.7 | -20.7 | -21.4 | -21.6 | -22.4 | -22.2 | -21.6 | -21.3 | -20.9 | -19.9 | -19.6 | -19.1 | -18.6 | -17.5 |
| | SE | 0.0 | 1.0 | 0.5 | 0.6 | 0.6 | 0.3 | 0.3 | 0.5 | 1.1 | 0.5 | 0.4 | 0.7 | 1.0 | 1.2 | 1.3 | 1.3 | 1.4 | 1.2 | 1.0 | 0.9 | 0.8 | 0.6 | 0.7 | 0.6 |

As a result, as shown in FIG. 19 and Table 25, the group administered with only semaglutide (Semaglutide) showed a significant body weight loss effect during the administration period, but the body weight regained after the administration of semaglutide was discontinued. Meanwhile, the group administered with OLX702A-075-16 according to an embodiment in combination with semaglutide (OLX702A-075-16+Sema) not only showed an excellent body weight loss effect compared to the Semaglutide group, but also confirmed that this body weight loss effect was maintained during the administration period of semaglutide as well as during the period when the administration of semaglutide was discontinued.

### 1-2. Comparison of dietary intake

Targeting the animal model groups of Experimental Example 1-1, measuring the dietary intake was performed for 12 weeks, and the dietary intake of each subject was measured daily starting 7 days prior to the administration of the test substance. The dietary intake was calculated by measuring the difference between the supply and the balance for each individual, and the cumulative dietary intake and average daily dietary intake during the relevant experimental period were evaluated.

Table 26 shows, for each animal model group, the results of confirming the relative daily average dietary intake change (%) compared to the day the test substance as administered (Base).

[Table 26]

| Daily Food Consumption (% change of baseline) | | | | |
|---|---|---|---|---|
| **Group** | **Vehicle** | **Semaglutide** | **OLX702A-075-16** | **Semaglutide +OLX702A-075-16** |
| **Mean** | -8.8 | -39.5 | -3.4 | -37.1 |
| **SE** | 18.2 | 4.9 | 6.4 | 6.3 |

As a result, as shown in FIG. 20 and Table 26, in terms of the daily average dietary intake, the group administered with OLX702A-075-16 according to an embodiment in combination with semaglutide (OLX702A-075-16+Sema) showed a similar level to the group administered with only semaglutide .

Referring to the experimental results above, it is inferred that the improved body weight loss effect by the combined administration of Experimental Example 1-1 resulted from an increase in the energy consumption, not from a decrease in the dietary intake.

**Experimental Example 2. Comparison of changes in body fat percentage by combined administration of OLX702A-075-16**

Body fat percentage measurements for the animal model groups of Experimental Example 1-1 were performed for 12 weeks, and starting on the day of administration of a test substance, the body fat percentage of each subject was measured at 4-week intervals after anesthetizing the animal model by injecting propofol, the anesthetized animal model was placed onto the GE Lunar Prodigy Primo bone densitometer. Afterwards, the body fat percentage was quantified and evaluated by GE Lunar Prodigy dual energy X-ray absorptiometry (DEXA) scanning.

Table 27 shows the results of changes in the body fat percentage (%) over time for each animal model group.

[Table 27]

| Body Fat Ratio (%) | | | | | | | | | | | | | | | | |
|---|---|---|---|---|---|---|---|---|---|---|---|---|---|---|---|---|
| **Group** | **Vehicle** | | | | **Semaglutide** | | | | **OLX702A-075-16** | | | | **OLX702A-075-16+Sema** | | | |
| **Day** | Base | D28 | D56 | D84 | Base | D28 | D56 | D84 | Base | D28 | D56 | D84 | Base | D28 | D56 | D84 |
| **Mean** | 44.5 | 45.3 | 44.8 | 45.5 | 43.9 | 37.7 | 33.9 | 37.3 | 44.7 | 44.2 | 42.5 | 42.5 | 44.5 | 36.9 | 32.9 | 31.9 |
| **SE** | 9.9 | 6.8 | 5.0 | 5.4 | 4.9 | 5.7 | 5.7 | 5.3 | 2.5 | 2.3 | 2.3 | 2.7 | 2.9 | 3.1 | 3.3 | 3.6 |

As a result, as shown in FIG. 21 and Table 27, the group administrated with OLX702A-075-16 in combination with semaglutide (OLX702A-075-16+Sema) showed a significant effect of reducing the body fat percentage, and this effect was superior to the group administered with semaglutide only.

**Experimental Example 3. Comparison of changes in abdominal circumference by combined administration of OLX702A-075-16**

Abdominal circumference measurements for the animal model groups of Experimental Example 1-1 were performed for 12 weeks, and starting on the day of administration of a test substance, the abdominal circumference of each subject was measured at 4-week intervals. After anesthetizing the animal model by injecting propofol, the anesthetized animal model was placed on its side and the abdominal circumference was measured around the navel and measured.

Table 28 shows the results of changes in the abdominal circumference (in centimeters (cm)) over time for each animal model group.

[Table 28]

| Abdominal Girth (cm) | | | | | | | | | | | | | | | | |
|---|---|---|---|---|---|---|---|---|---|---|---|---|---|---|---|---|
| **Group** | **Vehicle** | | | | **Semaglutide** | | | | **OLX702A-075-16** | | | | **OLX702A-075-16+Sema** | | | |
| **Day** | **Base** | **D28** | **D56** | **D84** | **Base** | **D28** | **D56** | **D84** | **Base** | **D28** | **D56** | **D84** | **Base** | **D28** | **D56** | **D84** |
| **Mean** | 54.5 | 55.0 | 54.0 | 54.0 | 55.9 | 47.0 | 48.0 | 46.8 | 55.4 | 51.3 | 50.4 | 50.1 | 55.0 | 43.6 | 42.0 | 43.8 |
| **SE** | 10.5 | 9.0 | 9.0 | 8.0 | 3.4 | 3.7 | 2.9 | 2.9 | 2.0 | 2.7 | 2.6 | 2.2 | 1.4 | 1.9 | 1.8 | 1.8 |

As a result, as shown in FIG. 22 and Table 28, the group administrated with OLX702A-075-16 in combination with semaglutide (OLX702A-075-16+Sema) showed a significant effect of reducing the abdominal circumference, and this effect was superior to the group administered with semaglutide only.

Thus far, specific portions of the content of the present disclosure have been described in detail, and it will be apparent to those of ordinary skill in the art that these specific descriptions are only preferred embodiments, and that the scope of the present disclosure is not limited thereby. Accordingly, it is intended that the substantial scope of the present disclosure is defined by the appended claims and their equivalents.

## Claims

1. A pharmaceutical composition for preventing or treating obesity, comprising a double-stranded RNAi agent including a sense strand and an antisense strand, as an active ingredient, wherein

   the antisense strand consists of EVP-mU*fG*mAmGmUmAfAmGfCmAmAmUmCfUmG*fG*mU*mC*mC*mU*-mU, P-mA*fG*mAmUmCfCmAfGfAmGmCmUmGfCmG*fC*mC*mA*mC, or EVP-mU*fG*mAmUmCfCmAfGfAmGmCmUmGfCmG*fC*mC*mA*mC, each of which has sequence complementarity with a mitochondrial amidoxime reducing component 1 (MARC1) mRNA sequence, and the sense strand consists of mC*mC*mAfG-mAfUmUmGmCmUmUmAmCmUmCmA-GalNAc, mG*fC*mGfCfAfGmCfUmCfUmGfGmAfUmCfU-GalNAc, or mG*fC*mGfCfAfGmCfUmCfUmGfGmAfUmCfA-GalNAc,
   wherein * indicates a modification to a phosphorothioate bond, m represents a substitution to 2'-O-methyl, P represents binding of a 5'-phosphate group, f represents a substitution to 2'-fluoro, EVP represents binding of 5'-E-vinylphosphonate, and GalNAc represents binding of an N-acetyl-galactosamine derivative.

2. The pharmaceutical composition of claim 1, wherein the N-acetyl-galactosamine derivative has a structure of Formula 1:

[Formula 1]

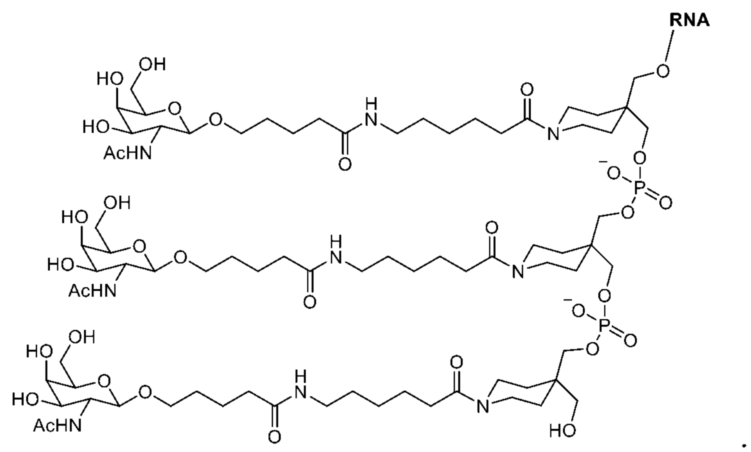

3. The pharmaceutical composition of claim 1, wherein the 5' end of the antisense strand and the 3' end of the sense strand form a blunt end.

4. The pharmaceutical composition of claim 1, wherein the pharmaceutical composition inhibits expression of MARC1.

5. The pharmaceutical composition of claim 1, wherein the pharmaceutical composition increases an expression level or a plasma level of fibroblast growth factor 21 (FGF 21).

6. The pharmaceutical composition of claim 1, wherein the pharmaceutical composition is administered in combination with a glucagon-like peptide-1 receptor (GLP-1) agonist or a glucagon-like peptide-1/glucose-dependent insulinotropic polypeptide (GLP-1/GIP) receptor dual agonist.

7. The pharmaceutical composition of claim 6, wherein the GLP-1 receptor agonist is selected from the group consisting

of semaglutide, liraglutide, exenatide, taspoglutide, albiglutide, lixisenatide, and dulaglutide.

**8.** The pharmaceutical composition of claim 6, wherein the GLP-1/GIP receptor dual agonist is selected from the group consisting of tirzepatide, NN9709, SAR-438335, and ZP-DI-70.

**9.** The pharmaceutical composition of claim 6, wherein the pharmaceutical composition is formulated to be administered with the GLP-1 receptor agonist or the GLP-1/GIP receptor dual agonist separately, simultaneously, or sequentially.

**10.** A combined preparation for preventing or treating obesity, comprising:

a first active ingredient comprising a glucagon-like peptide-1 (GLP-1) receptor agonist or a glucagon-like peptide-1/glucose-dependent insulinotropic polypeptide (GLP-1/GIP) receptor dual agonist; and a second active ingredient comprising a double-stranded RNAi agent comprising a sense strand and an antisense strand, wherein

the antisense strand consists of EVP-mU*fG*mAmGmUmAfAmGfCmAmAmUmCfUmG*fG*mU*mC*mC*mU*-mU, P-mA*fG*mAmUmCfCmAfGfAmGmCmUmGfCmG*fC*mC*mA*mC, or EVP-mU*fG*mAmUmCfCmAfGfAmGmCmUmGfCmG*fC*mC*mA*mC, each of which has sequence complementarity with a mitochondrial amidoxime reducing component 1 (MARC1) mRNA sequence, and the sense strand consists of mC*mC*mAfG-mAfUmUmGmCmUmUmAmCmUmCmA-GalNAc, mG*fC*mGfCfAfGmCfUmCfUmGfGmAfUmCfU-GalNAc, or mG*fC*mGfCfAfGmCfUmCfUmGfGmAfUmCfA-GalNAc,

wherein * indicates a modification to a phosphorothioate bond, m represents a substitution to 2'-O-methyl, P represents binding of a 5'-phosphate group, f represents a substitution to 2'-fluoro, EVP represents binding of 5'-E-vinylphosphonate, and GalNAc represents binding of an N-acetyl-galactosamine derivative.

**11.** The combined preparation of claim 10, wherein the GLP-1 receptor agonist is selected from the group consisting of semaglutide, liraglutide, exenatide, taspoglutide, albiglutide, lixisenatide, and dulaglutide.

**12.** The combined preparation of claim 10, wherein the GLP-1/GIP receptor dual agonist is selected from the group consisting of tirzepatide, NN9709, SAR-438335, and ZP-DI-70.

**13.** The combined preparation of claim 10, wherein the N-acetyl-galactosamine derivative is a compound of Formula 1:

[Formula 1]

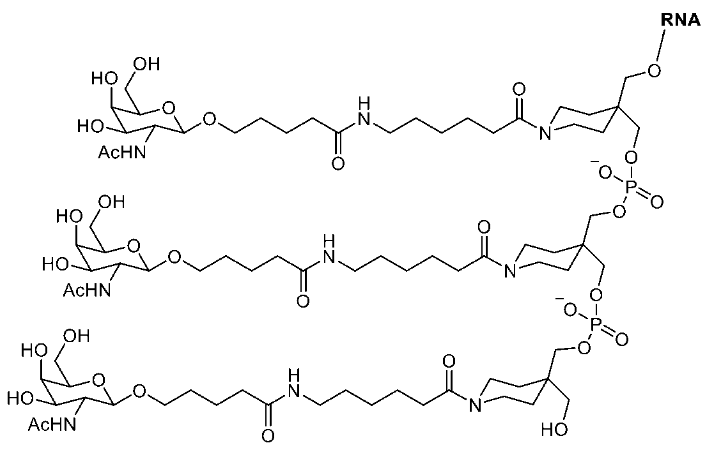

.

**14.** The combined preparation of claim 10, wherein the 5' end of the antisense strand and the 3' end of the sense strand form a blunt end.

**15.** The combined preparation of claim 10, wherein the first active ingredient and the second active ingredient are formulated to be administered individually, simultaneously, or sequentially.

**16.** A kit for preventing or treating obesity, comprising the combined preparation of any one of claims 10 to 15, wherein the combined preparation is provided to a subject in the form that the first active ingredient is contained in a first compartment and the second active ingredient is contained in a second compartment.

FIG. 1

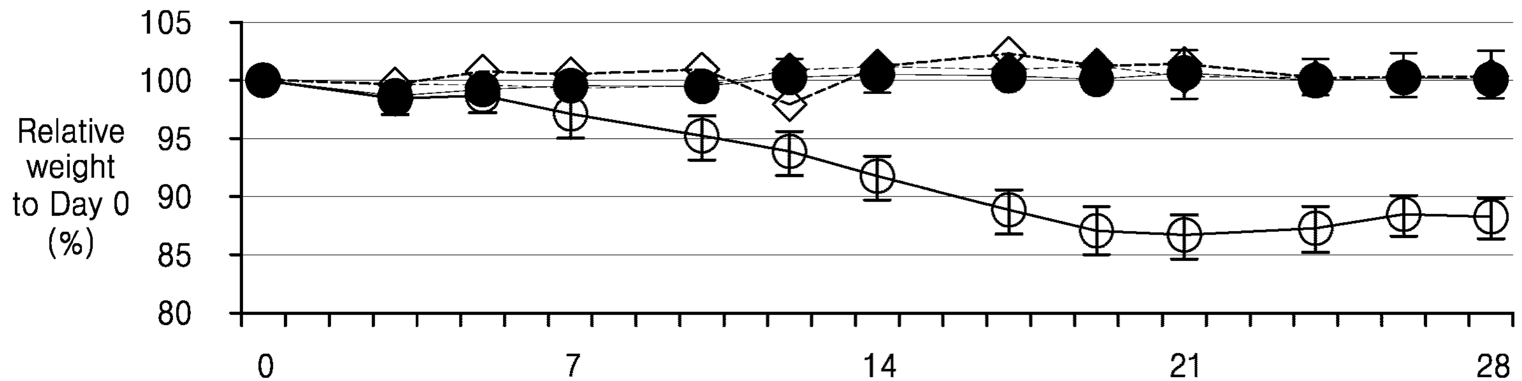


| | 0 | 3 | 5 | 7 | 10 | 12 | 14 | 17 | 19 | 21 | 24 | 25 | 28 |
|---|---|---|---|---|---|---|---|---|---|---|---|---|---|
| G1 | 100.00 | 98.73 | 100.85 | 100.57 | 101.06 | 98.15 | 100.48 | 102.36 | 101.63 | 101.57 | 100.57 | 99.91 | 100.09 |
| G2 | 100.00 | 99.08 | 99.52 | 99.57 | 99.48 | 100.04 | 100.09 | 100.15 | 99.94 | 100.63 | 99.78 | 99.96 | 99.78 |
| G3 | 100.00 | 99.78 | 99.55 | 99.36 | 99.71 | 100.91 | 101.26 | 101.00 | 101.32 | 100.20 | 100.50 | 100.51 | 100.50 |
| G4 | 100.00 | 98.47 | 98.69 | 97.04 | 95.16 | 93.83 | 91.73 | 88.82 | 87.03 | 86.56 | 87.23 | 88.39 | 88.09 |

FIG. 2

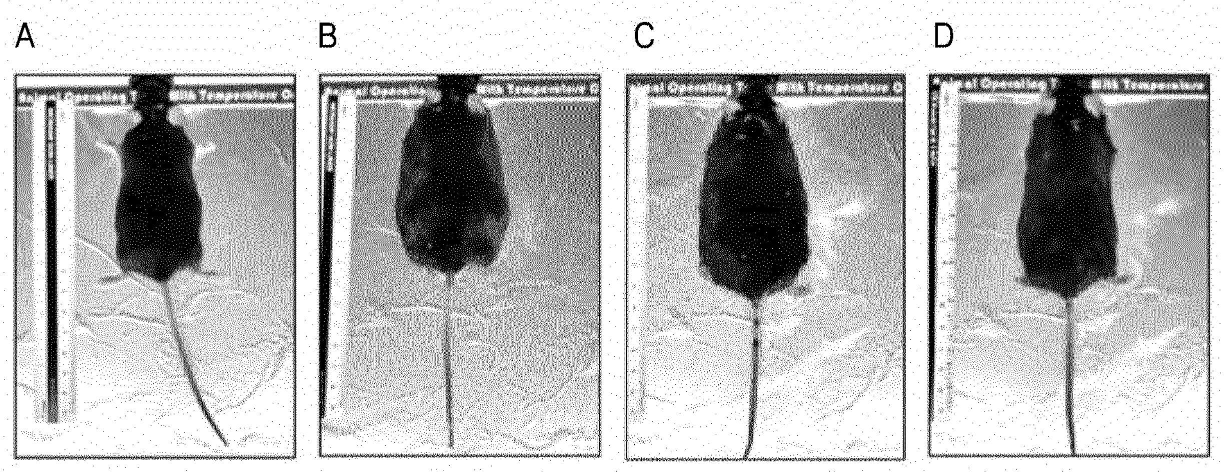

FIG. 3

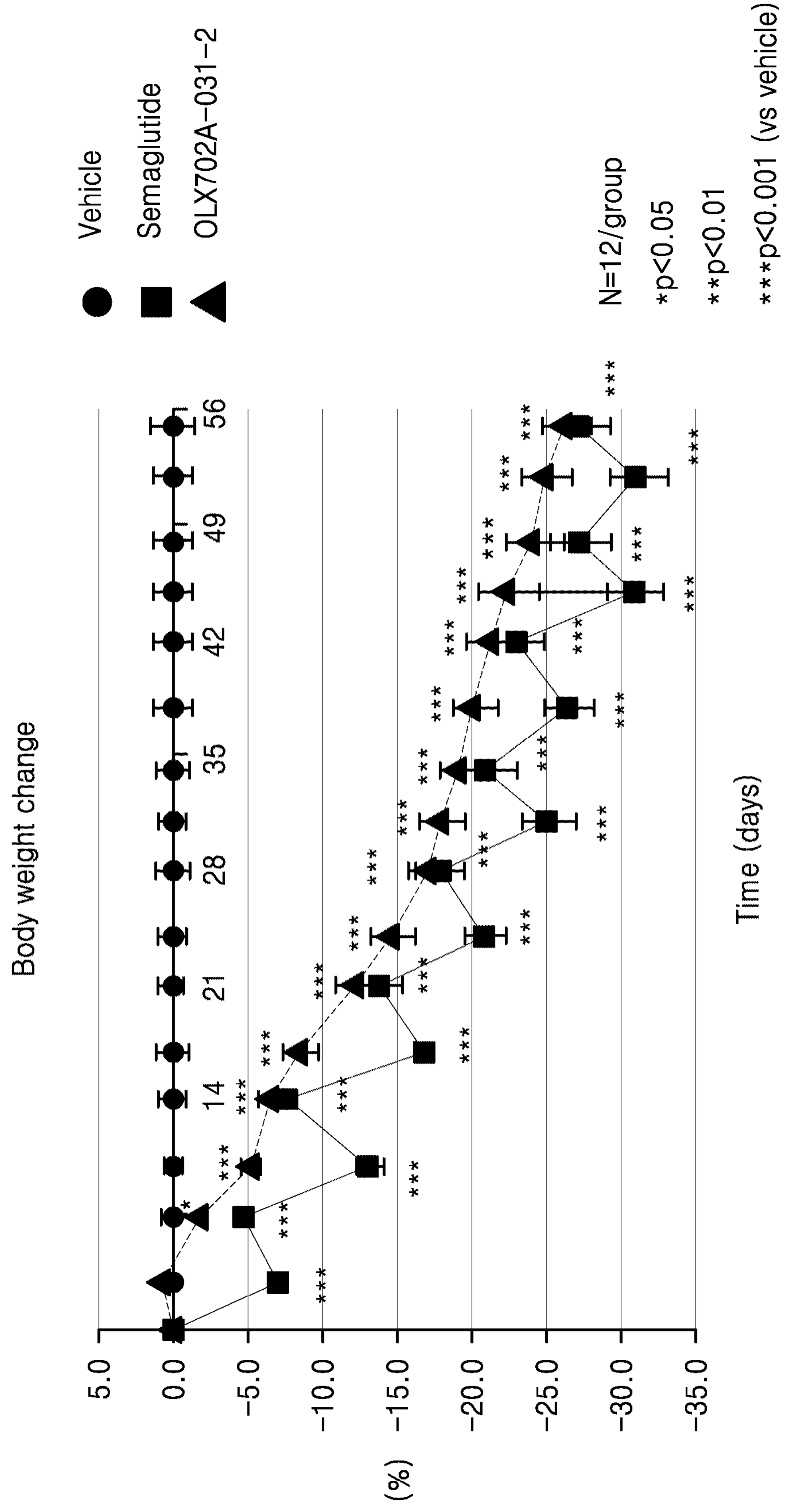
Body weight change
(%)
5.0
0.0
-5.0
-10.0
-15.0
-20.0
-25.0
-30.0
-35.0
14
21
28
35
42
49
56
Time (days)
Vehicle
Semaglutide
OLX702A-031-2
N=12/group
*p<0.05
**p<0.01
***p<0.001 (vs vehicle)

# FIG. 4

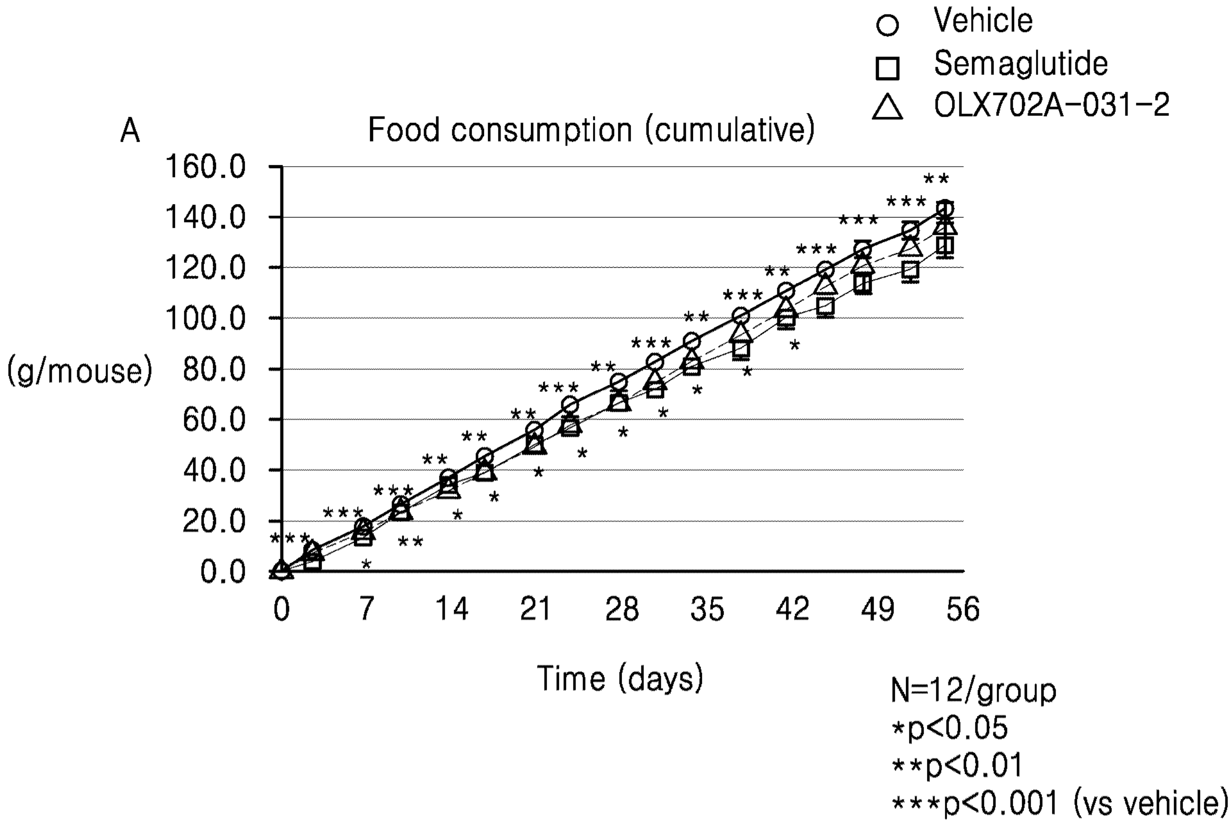
Vehicle
Semaglutide
OLX702A-031-2
A
Food consumption (cumulative)
160.0
140.0
120.0
100.0
(g/mouse) 80.0
60.0
40.0
20.0
0.0
0 7 14 21 28 35 42 49 56
Time (days)
N=12/group
*p<0.05
**p<0.01
***p<0.001 (vs vehicle)

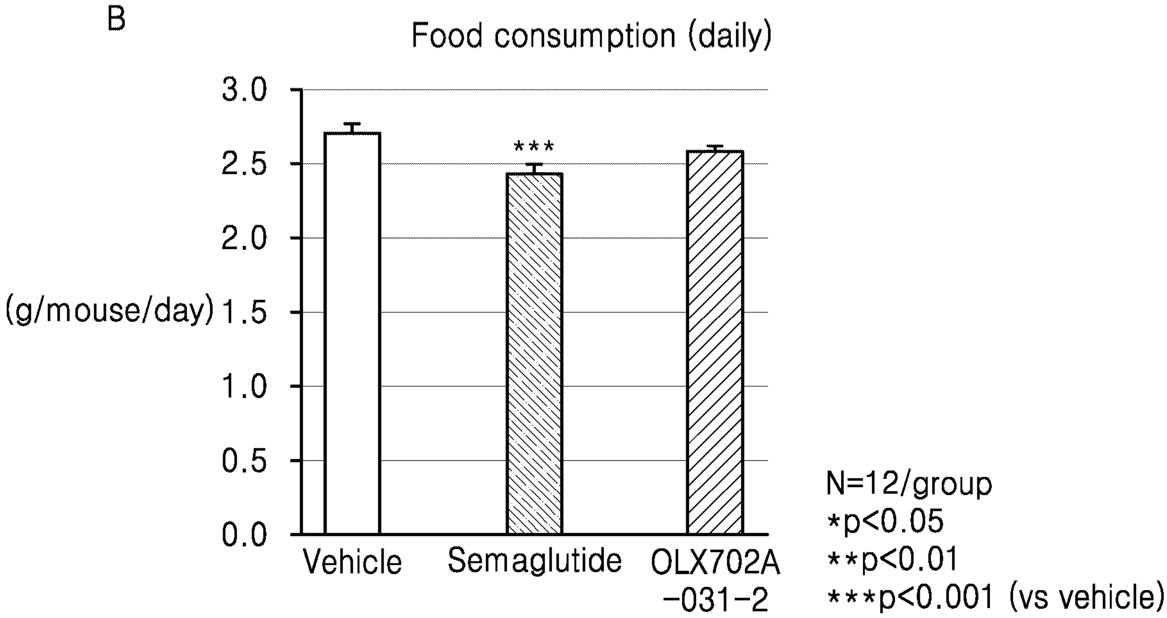
B
Food consumption (daily)
3.0
2.5
2.0
(g/mouse/day) 1.5
1.0
0.5
0.0
***
Vehicle
Semaglutide
OLX702A -031-2
N=12/group
*p<0.05
**p<0.01
***p<0.001 (vs vehicle)

# FIG. 5

A

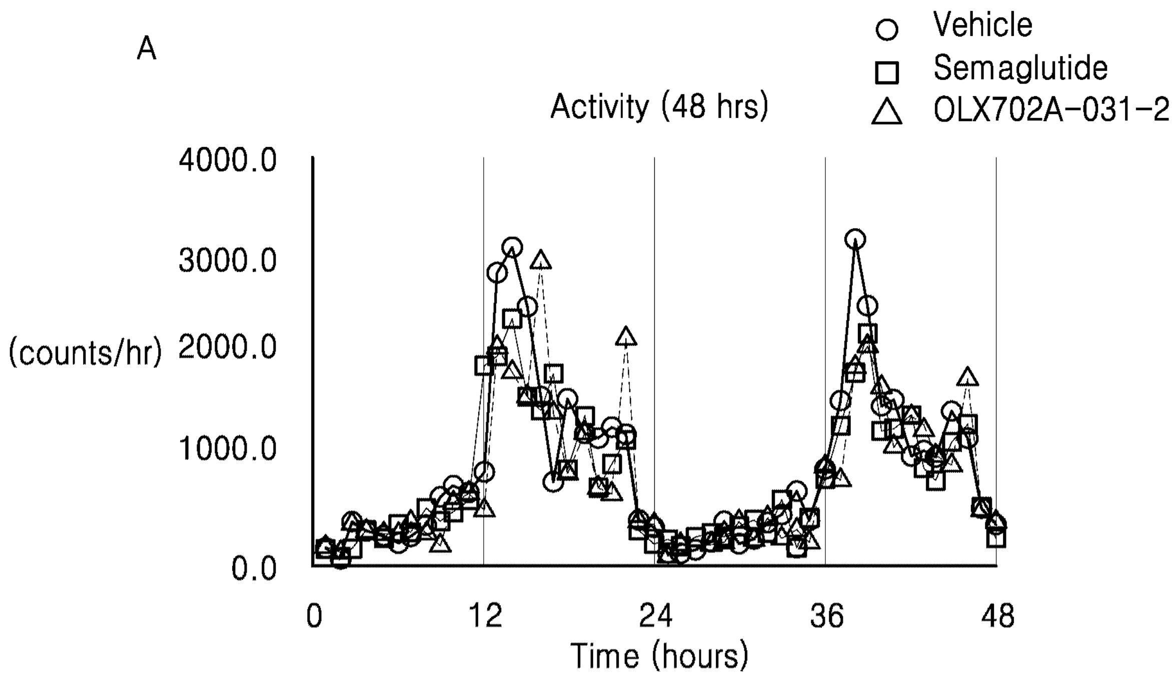
Vehicle
Semaglutide
OLX702A-031-2
Activity (48 hrs)
4000.0
3000.0
(counts/hr) 2000.0
1000.0
0.0
0
12
24
36
48
Time (hours)

B

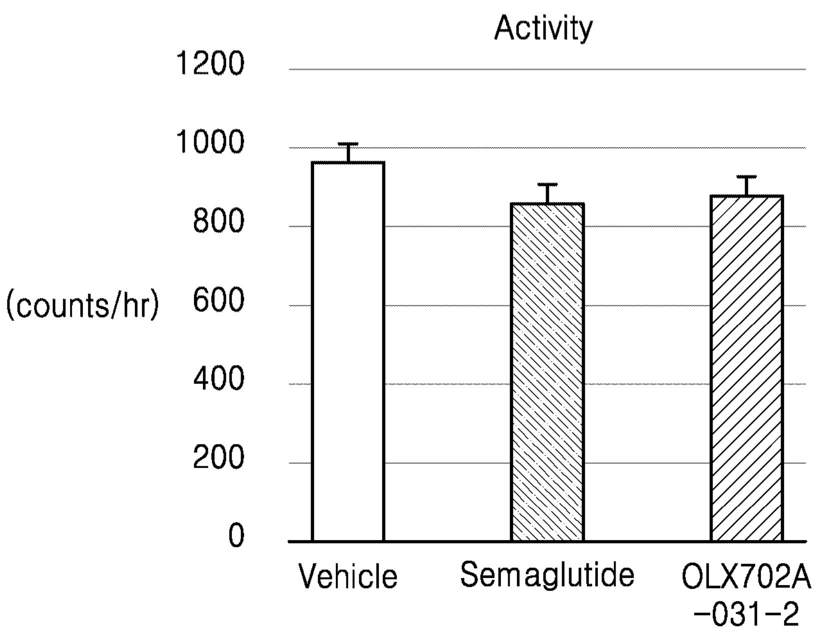
Activity
1200
1000
800
(counts/hr) 600
400
200
0
Vehicle
Semaglutide
OLX702A
-031-2

# FIG. 6

A

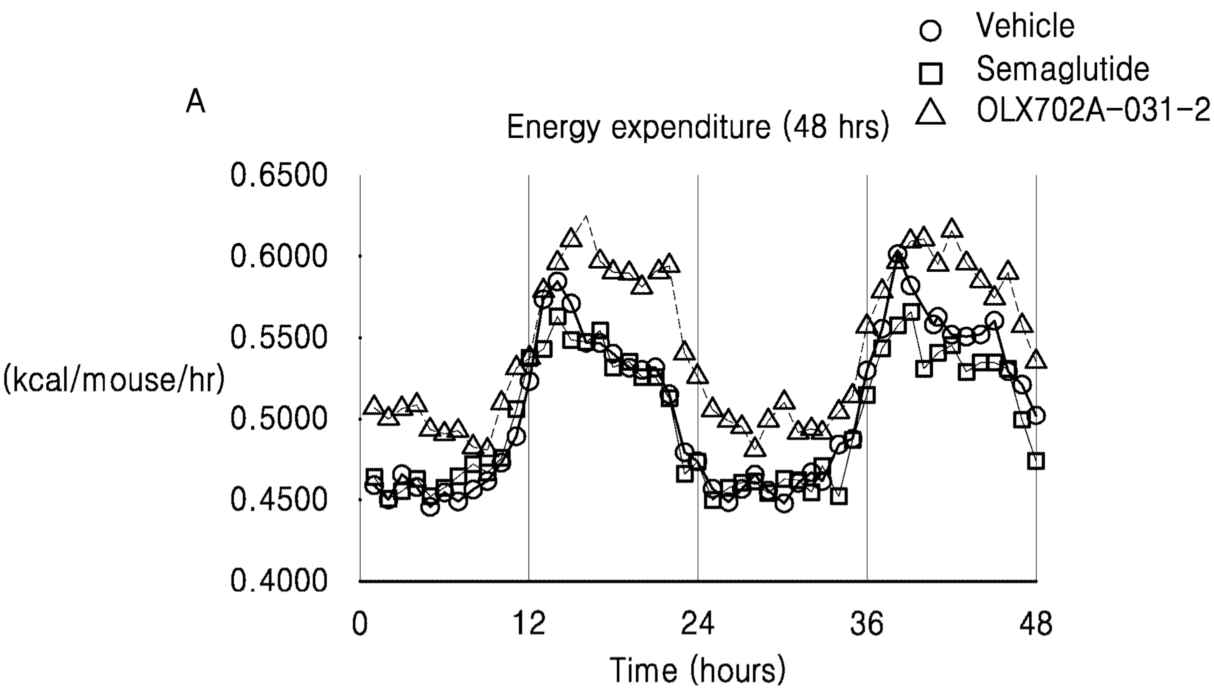
Vehicle
Semaglutide
OLX702A-031-2
Energy expenditure (48 hrs)
0.6500
0.6000
0.5500
(kcal/mouse/hr)
0.5000
0.4500
0.4000
0
12
24
36
48
Time (hours)

B

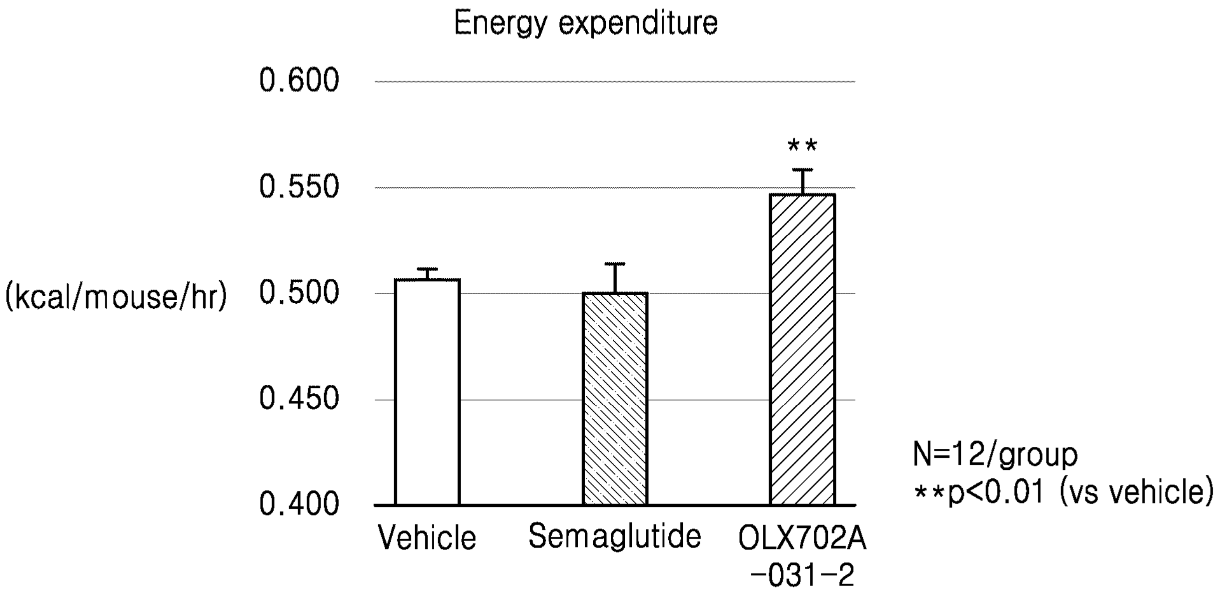
Energy expenditure
0.600
0.550
(kcal/mouse/hr)
0.500
0.450
0.400
**
Vehicle
Semaglutide
OLX702A
-031-2
N=12/group
**p<0.01 (vs vehicle)

# FIG. 7

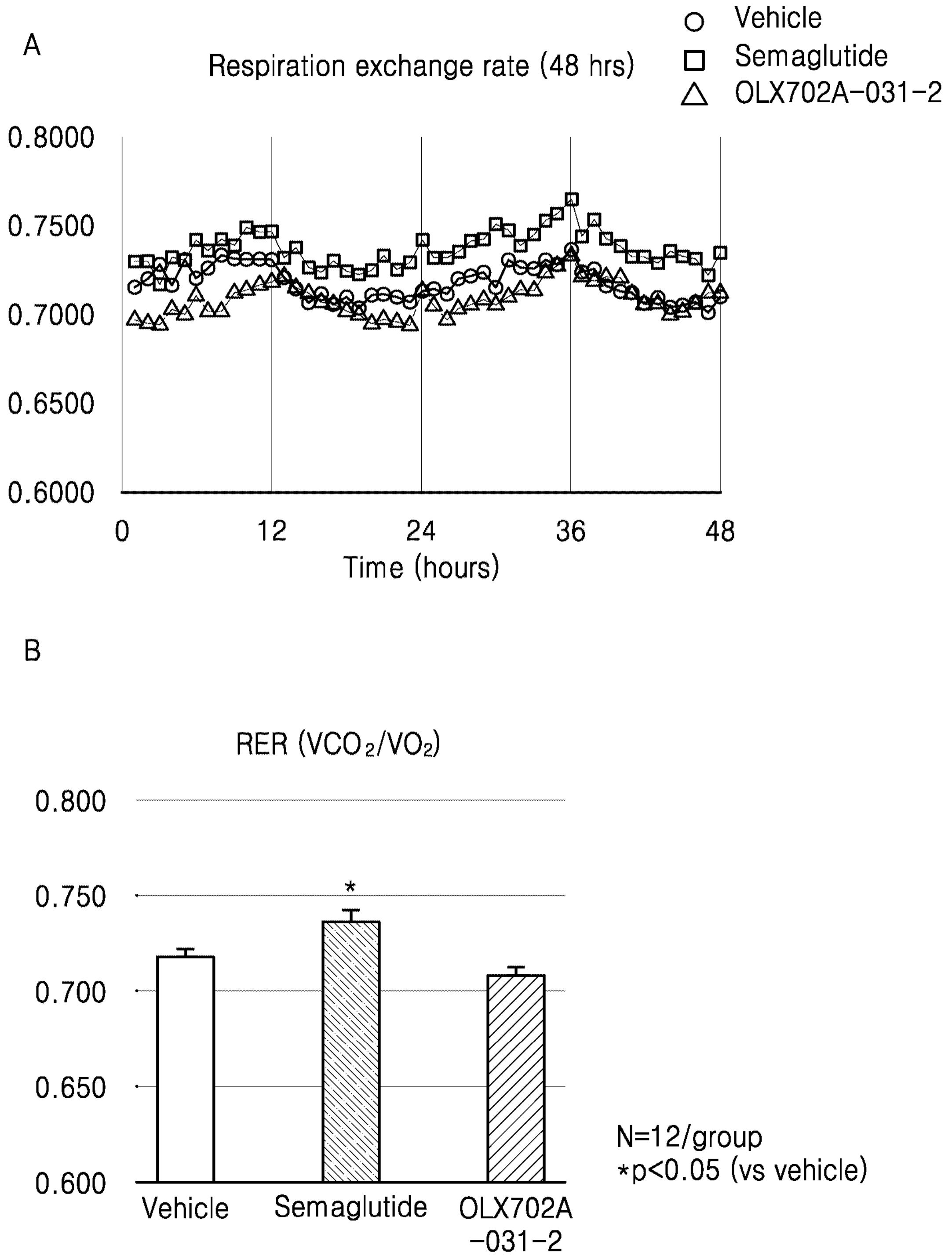
A
Respiration exchange rate (48 hrs)
Vehicle
Semaglutide
OLX702A-031-2
0.8000
0.7500
0.7000
0.6500
0.6000
0
12
24
36
48
Time (hours)
B
RER ($VCO_2/VO_2$)
0.800
0.750
0.700
0.650
0.600
*
Vehicle
Semaglutide
OLX702A
-031-2
N=12/group
*p<0.05 (vs vehicle)

FIG. 8

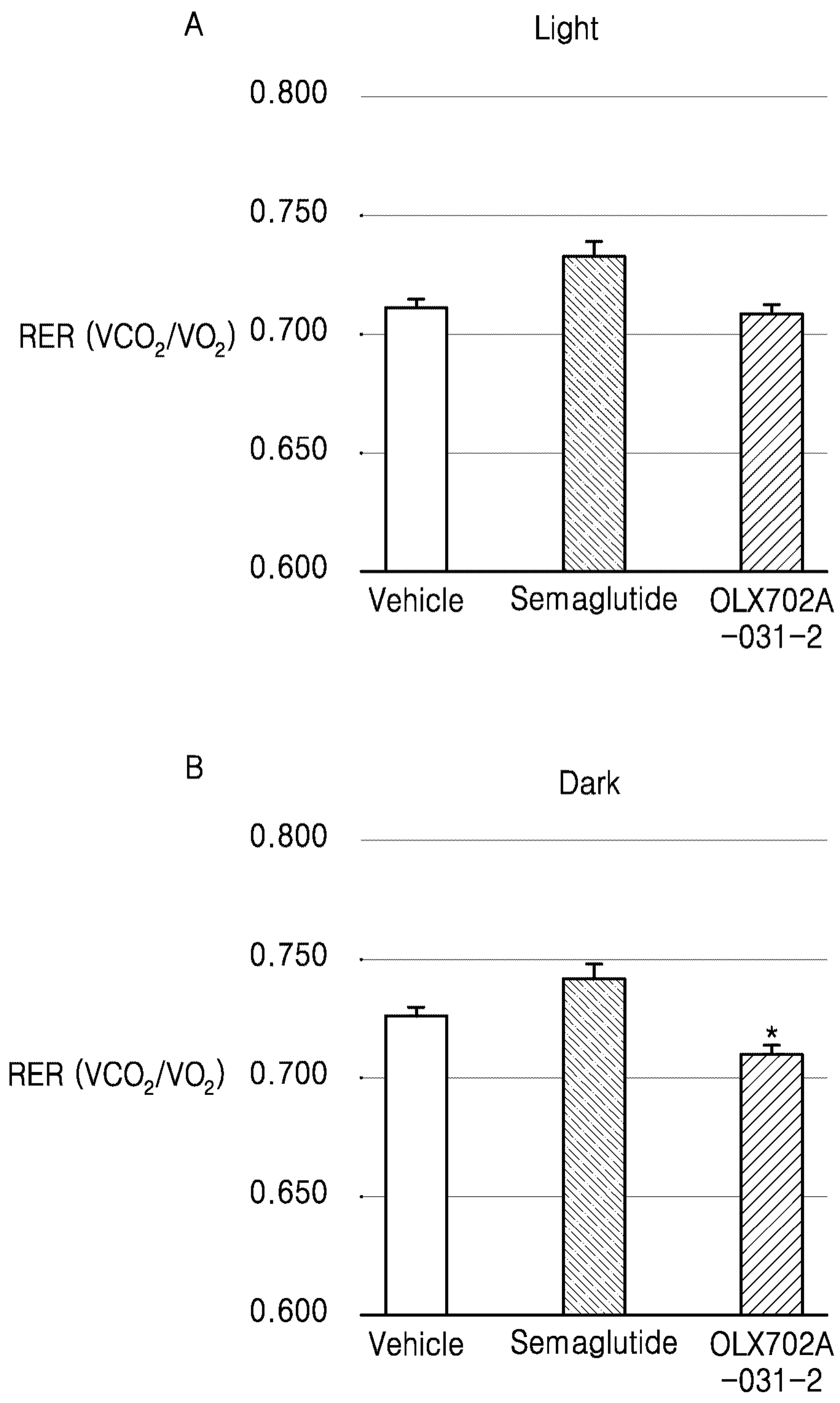
A
Light
RER (VCO2/VO2)
0.800
0.750
0.700
0.650
0.600
Vehicle
Semaglutide
OLX702A
-031-2
B
Dark
RER (VCO2/VO2)
0.800
0.750
0.700
0.650
0.600
*
Vehicle
Semaglutide
OLX702A
-031-2

# FIG. 9

A

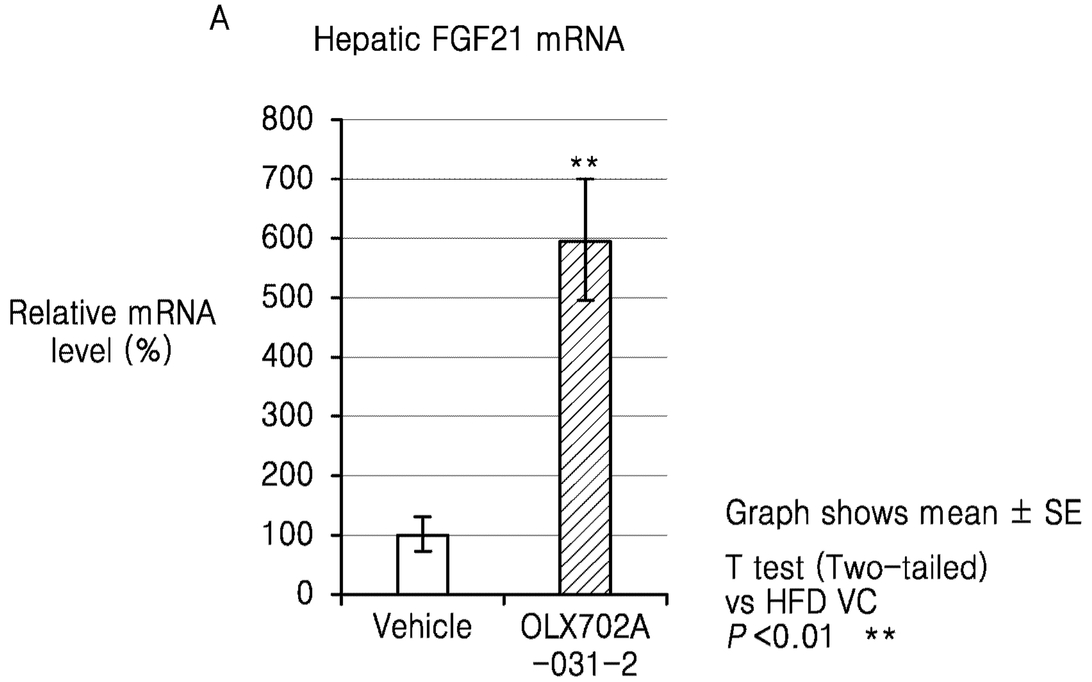
Hepatic FGF21 mRNA
800
700
600
500
400
300
200
100
0
Relative mRNA level (%)
**
Vehicle
OLX702A -031-2
Graph shows mean ± SE
T test (Two-tailed)
vs HFD VC
P<0.01 **

B

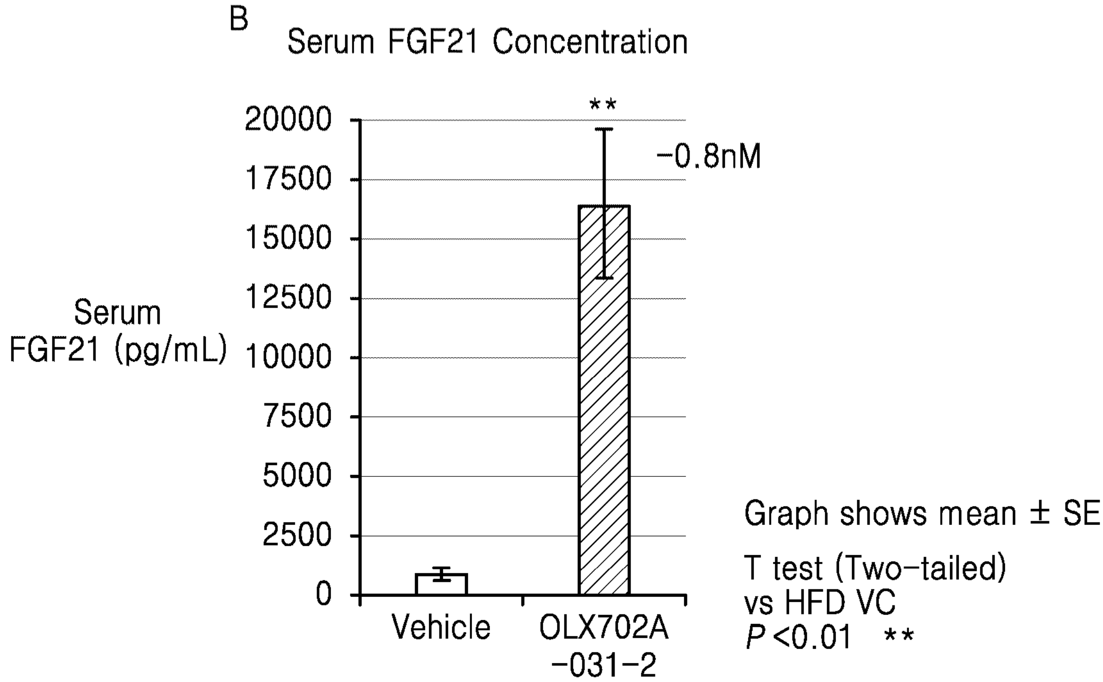
Serum FGF21 Concentration
20000
17500
15000
12500
10000
7500
5000
2500
0
Serum FGF21 (pg/mL)
**
~0.8nM
Vehicle
OLX702A -031-2
Graph shows mean ± SE
T test (Two-tailed)
vs HFD VC
P<0.01 **

FIG. 10

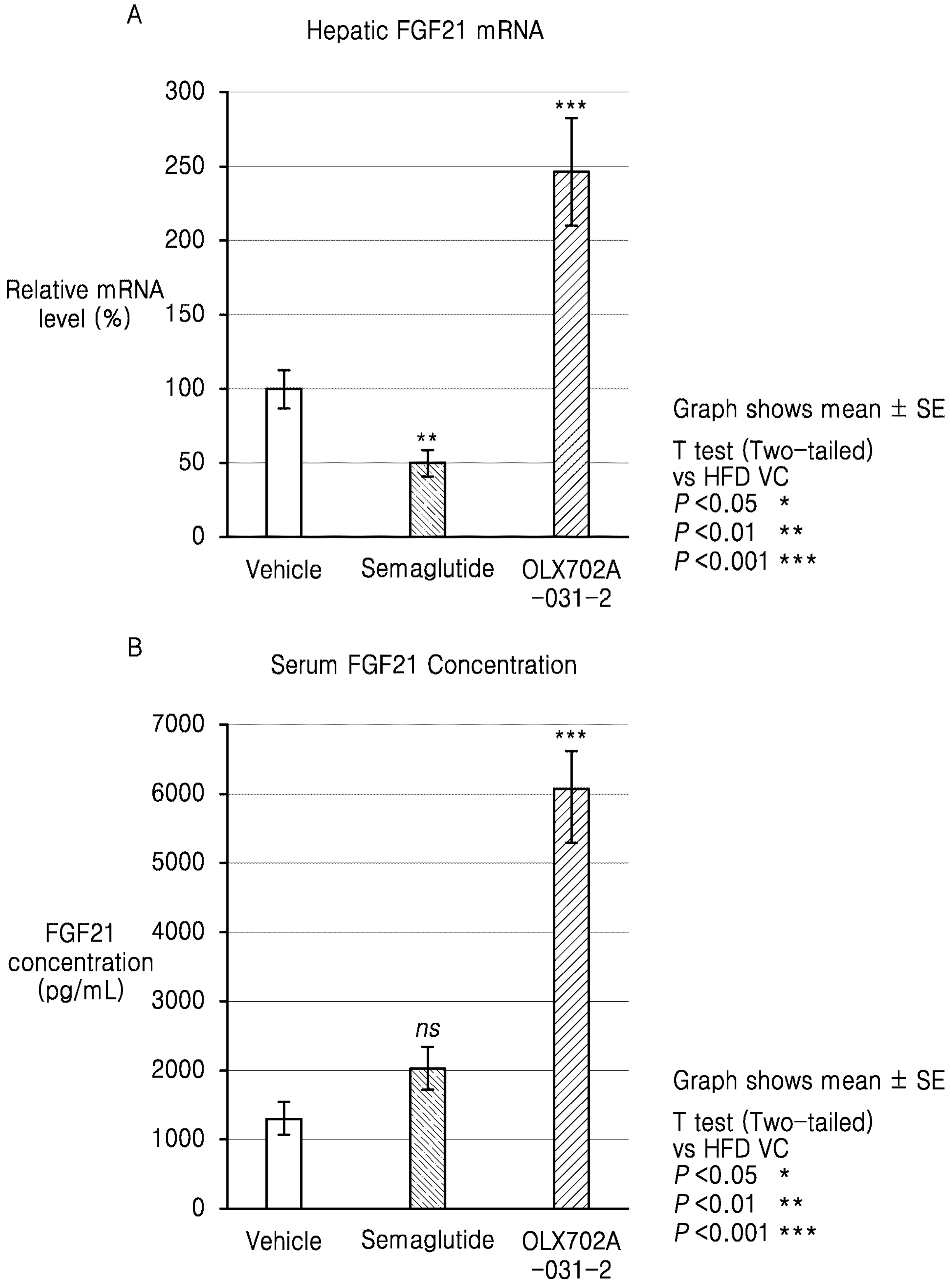
A
Hepatic FGF21 mRNA
Relative mRNA level (%)
300
250
200
150
100
50
0
***
**
Vehicle
Semaglutide
OLX702A -031-2
Graph shows mean ± SE
T test (Two-tailed)
vs HFD VC
P <0.05 *
P <0.01 **
P <0.001 ***
B
Serum FGF21 Concentration
FGF21 concentration (pg/mL)
7000
6000
5000
4000
3000
2000
1000
0
***
ns
Vehicle
Semaglutide
OLX702A -031-2
Graph shows mean ± SE
T test (Two-tailed)
vs HFD VC
P <0.05 *
P <0.01 **
P <0.001 ***

FIG. 11

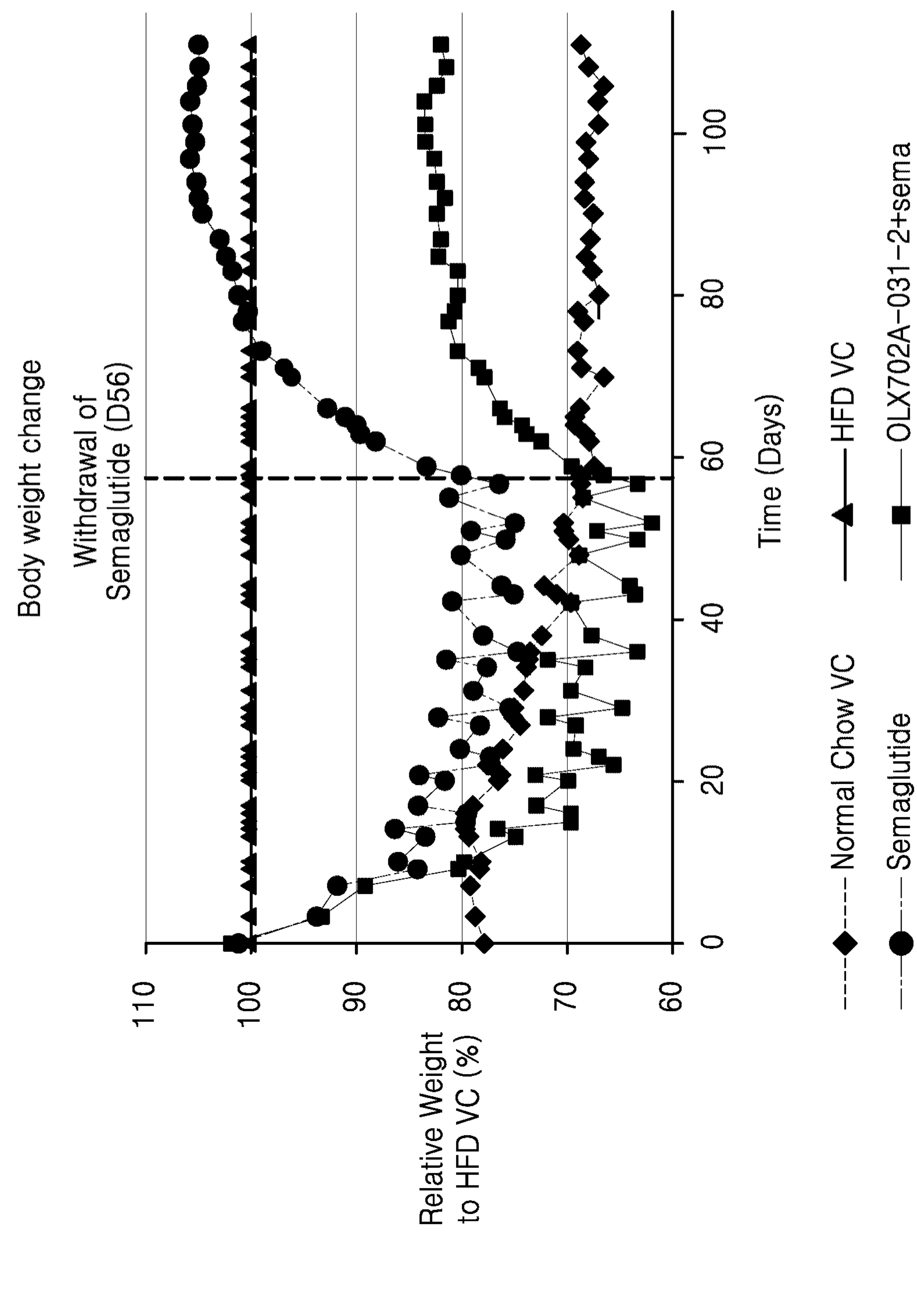
Body weight change
Withdrawal of
Semaglutide (D56)
Relative Weight
to HFD VC (%)
110
100
90
80
70
60
0
20
40
60
80
100
Time (Days)
Normal Chow VC
HFD VC
Semaglutide
OLX702A-031-2+sema

FIG. 12

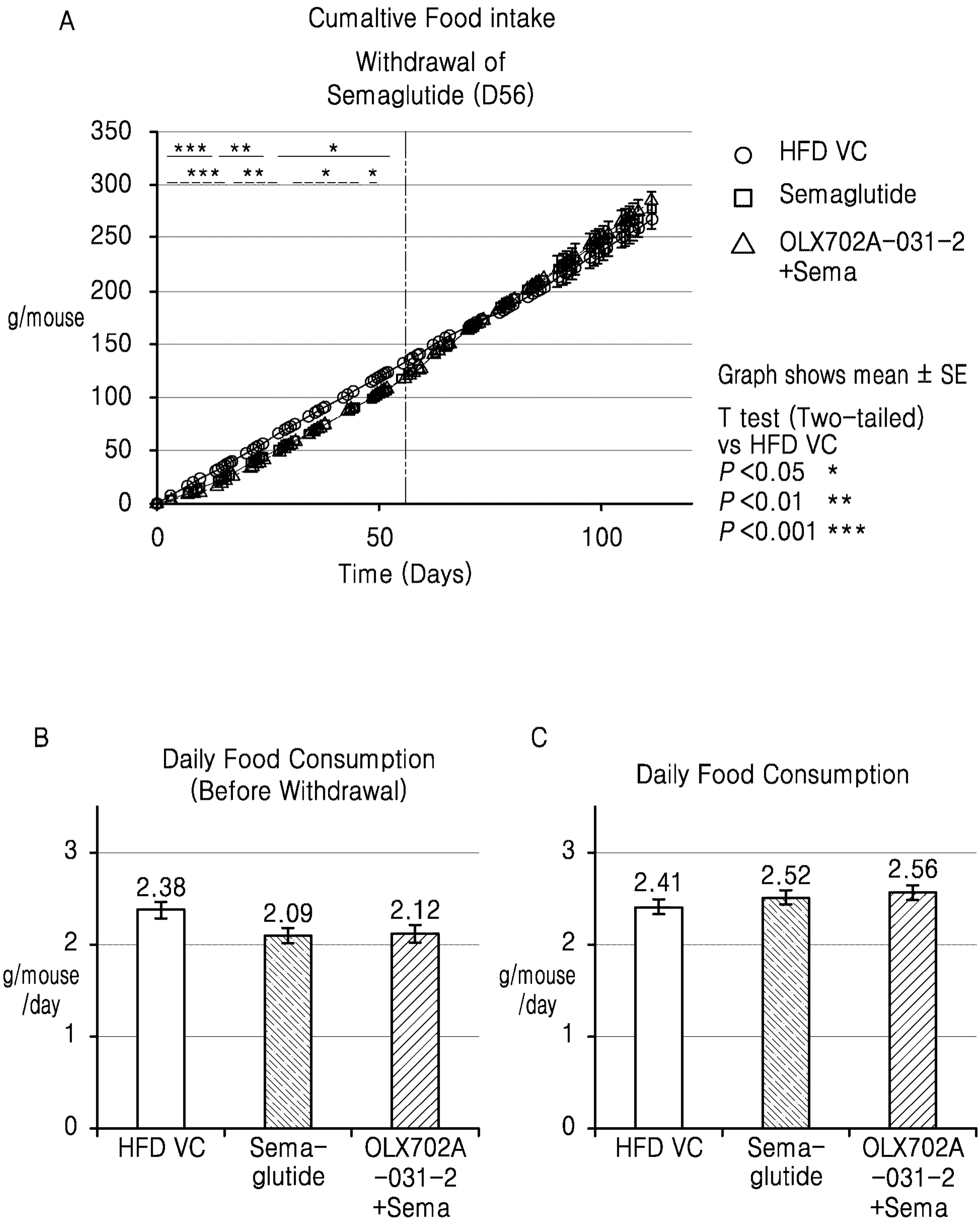
A
Cumaltive Food intake
Withdrawal of
Semaglutide (D56)
350
300
250
200
150
100
50
0
g/mouse
0
50
100
Time (Days)
*** ** *
*** ** * *
HFD VC
Semaglutide
OLX702A-031-2
+Sema
Graph shows mean ± SE
T test (Two-tailed)
vs HFD VC
P<0.05 *
P<0.01 **
P<0.001 ***
B
Daily Food Consumption
(Before Withdrawal)
g/mouse
/day
3
2
1
0
2.38
2.09
2.12
HFD VC
Sema-
glutide
OLX702A
-031-2
+Sema
C
Daily Food Consumption
g/mouse
/day
3
2
1
0
2.41
2.52
2.56
HFD VC
Sema-
glutide
OLX702A
-031-2
+Sema

FIG. 13

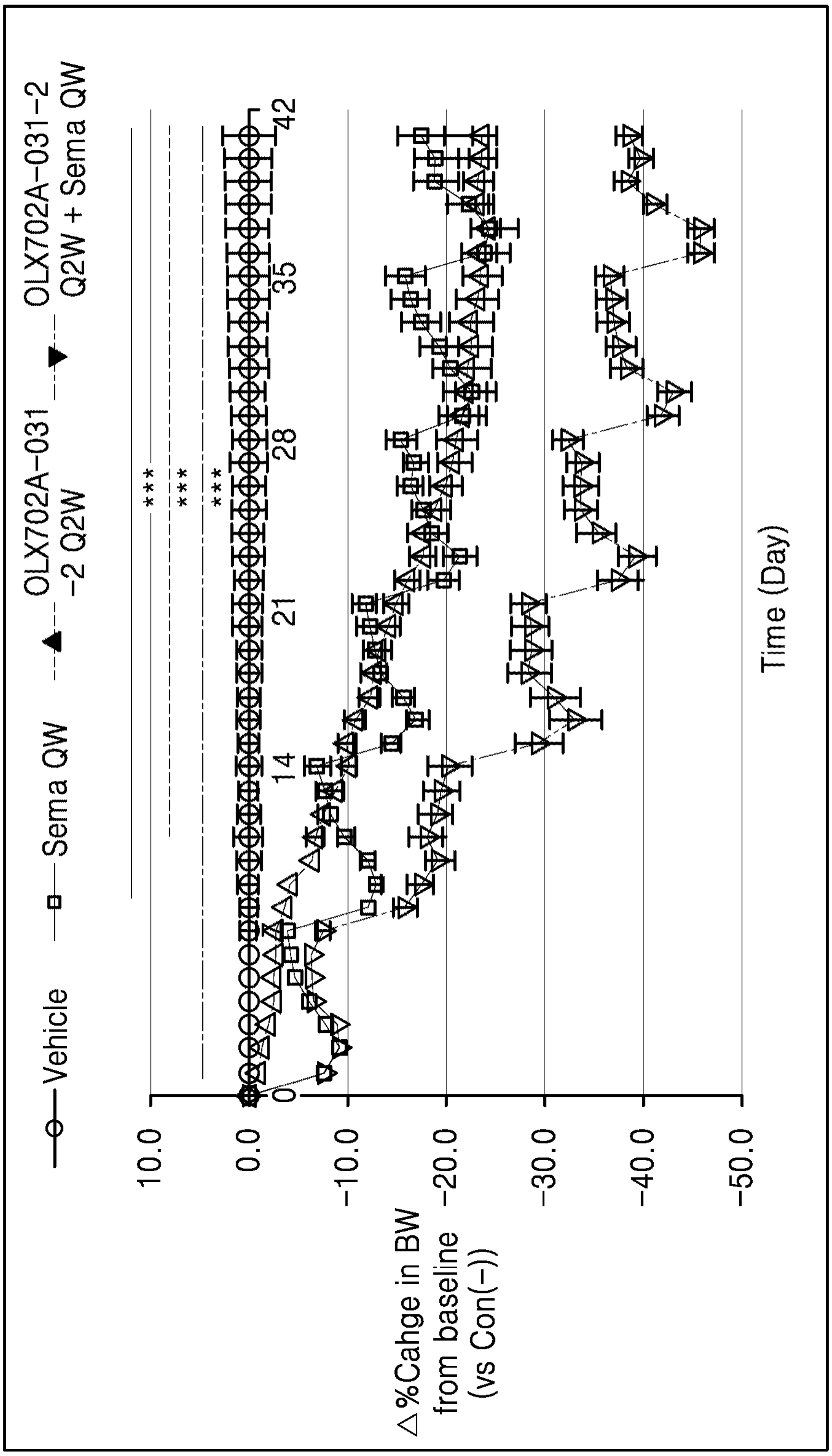

△BW changes from baseline (adjusted by vehicle)
Vehicle
Sema QW
OLX702A-031-2 Q2W
OLX702A-031-2 Q2W + Sema QW
***
***
***
△%Cahge in BW from baseline (vs Con(-))
10.0
0.0
-10.0
-20.0
-30.0
-40.0
-50.0
0
14
21
28
35
42
Time (Day)

FIG. 14

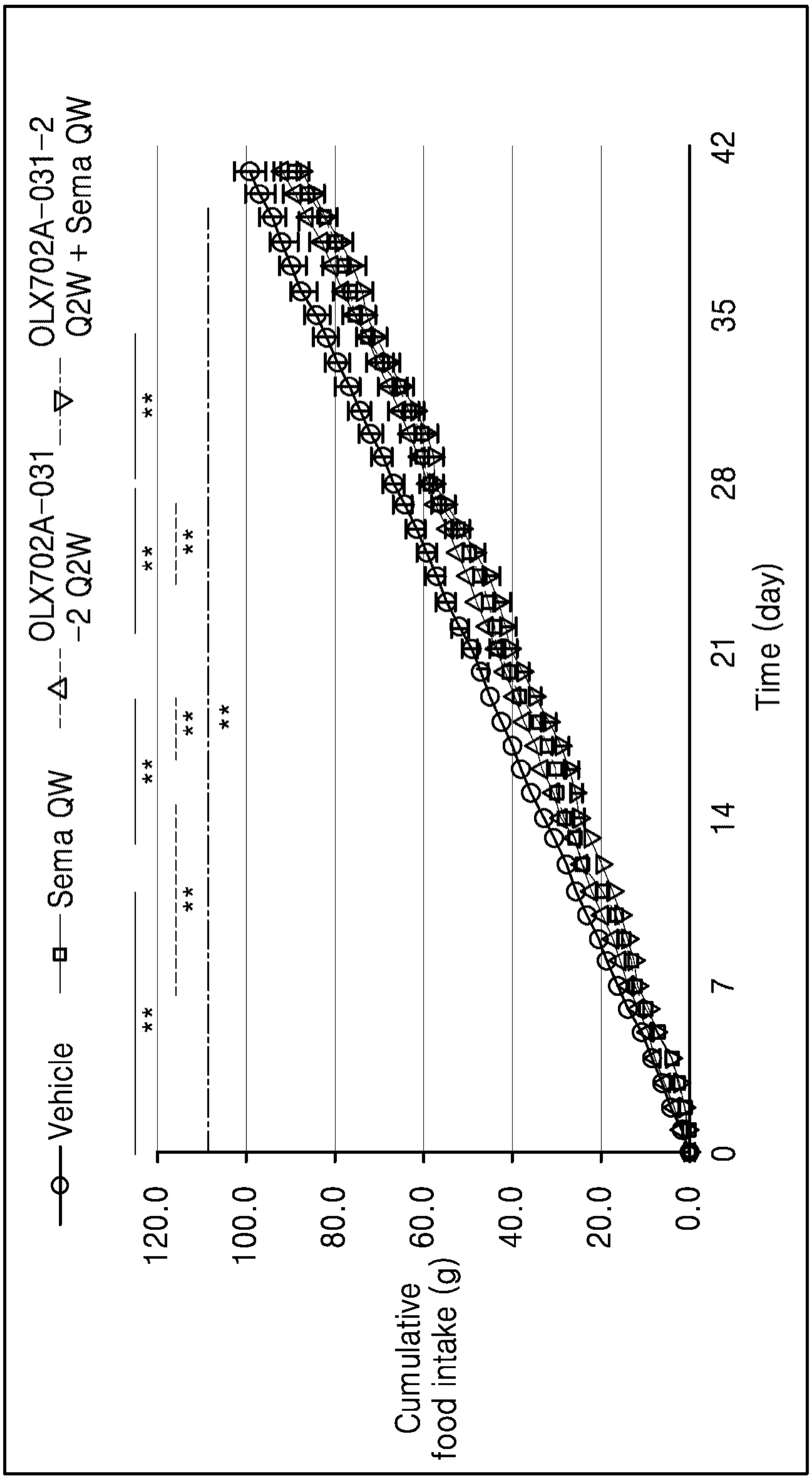
food intake (accumulated)
Vehicle
Sema QW
OLX702A-031-2 Q2W
OLX702A-031-2 Q2W + Sema QW
**
Cumulative food intake (g)
0.0
20.0
40.0
60.0
80.0
100.0
120.0
0
7
14
21
28
35
42
Time (day)

FIG. 15

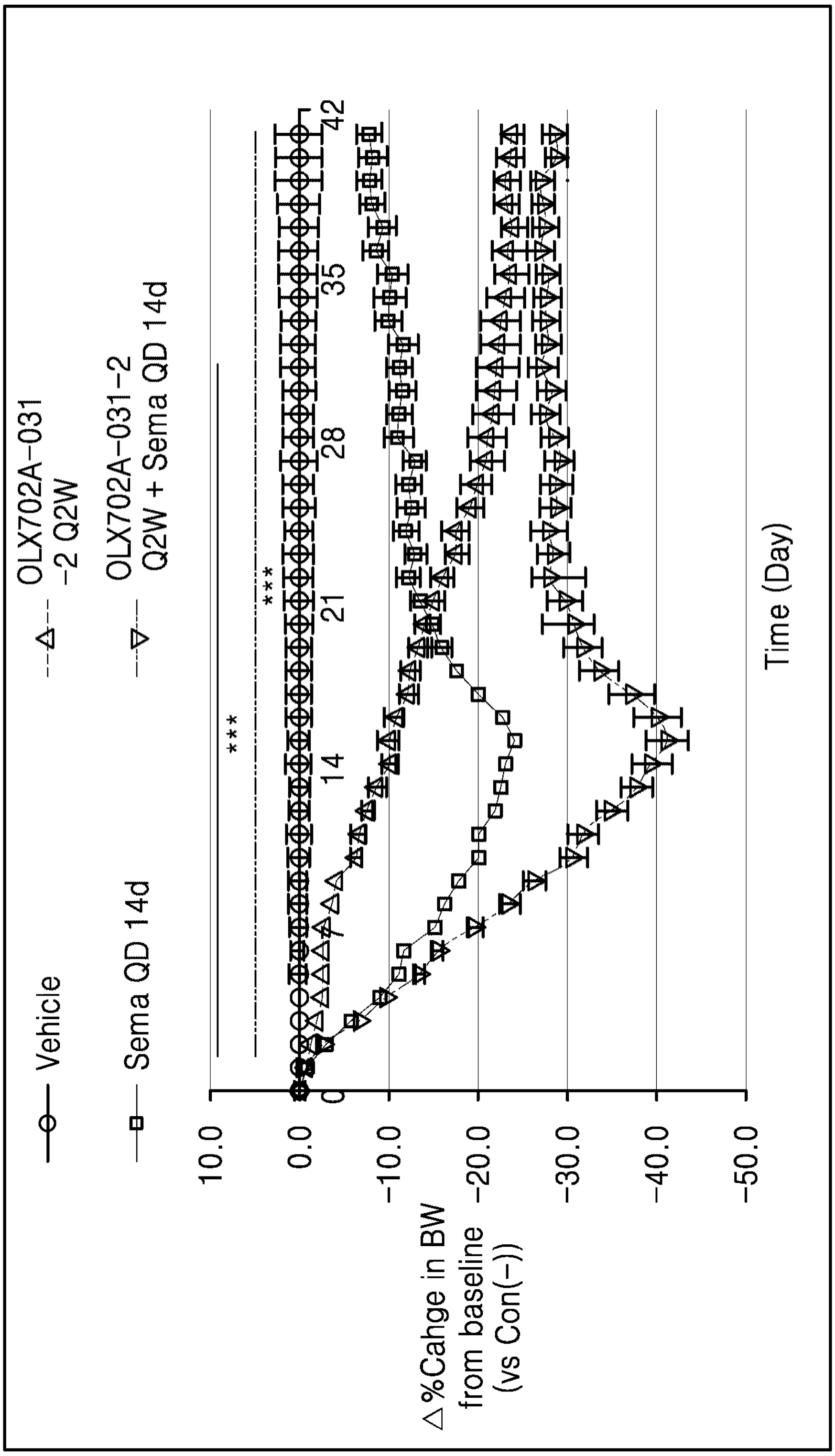
△BW changes from baseline (adjusted by vehicle)
Vehicle
Sema QD 14d
OLX702A-031-2 Q2W
OLX702A-031-2 Q2W + Sema QD 14d
△%Cahge in BW from baseline (vs Con(-))
10.0
0.0
-10.0
-20.0
-30.0
-40.0
-50.0
0
7
14
21
28
35
42
***
***
Time (Day)

FIG. 16

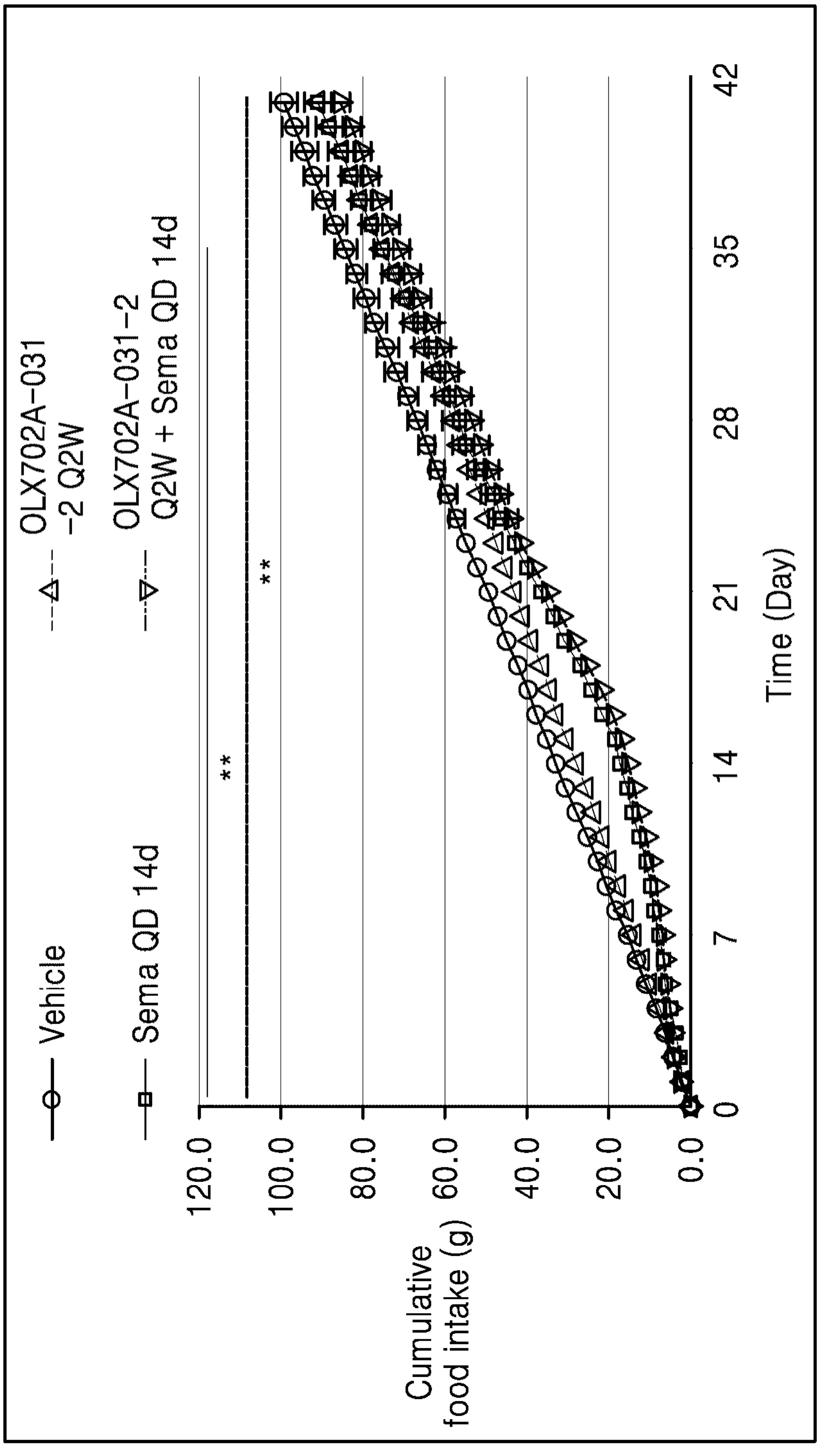
food intake (accumulated)
Vehicle
Sema QD 14d
OLX702A-031-2 Q2W
OLX702A-031-2 Q2W + Sema QD 14d
**
**
Cumulative food intake (g)
120.0
100.0
80.0
60.0
40.0
20.0
0.0
0
7
14
21
28
35
42
Time (Day)

FIG. 17

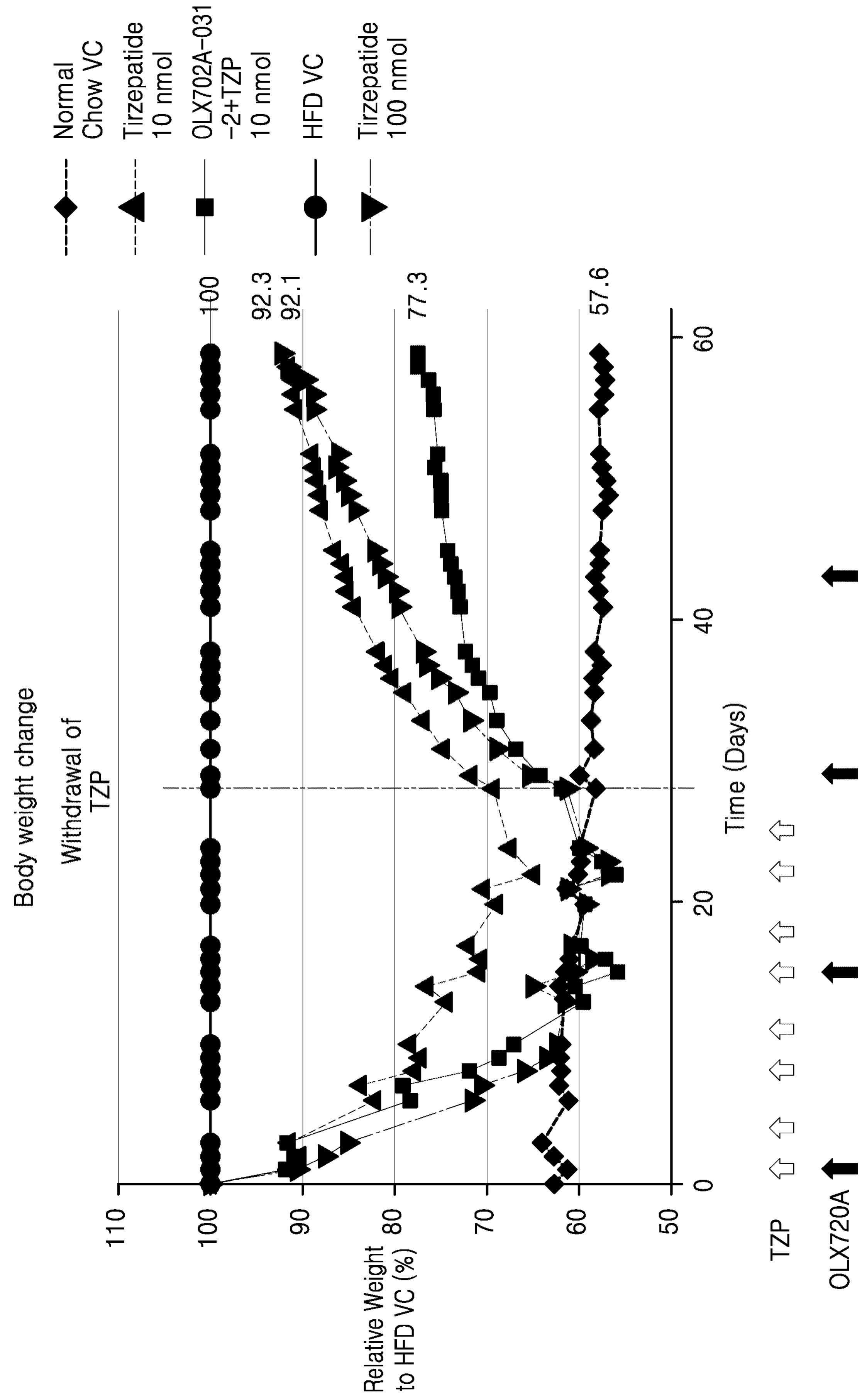
Body weight change
Withdrawal of TZP
Relative Weight to HFD VC (%)
110
100
90
80
70
60
50
0
20
40
60
Time (Days)
100
92.3
92.1
77.3
57.6
TZP
OLX720A
Normal Chow VC
Tirzepatide 10 nmol
OLX702A-031 -2+TZP 10 nmol
HFD VC
Tirzepatide 100 nmol

FIG. 18A

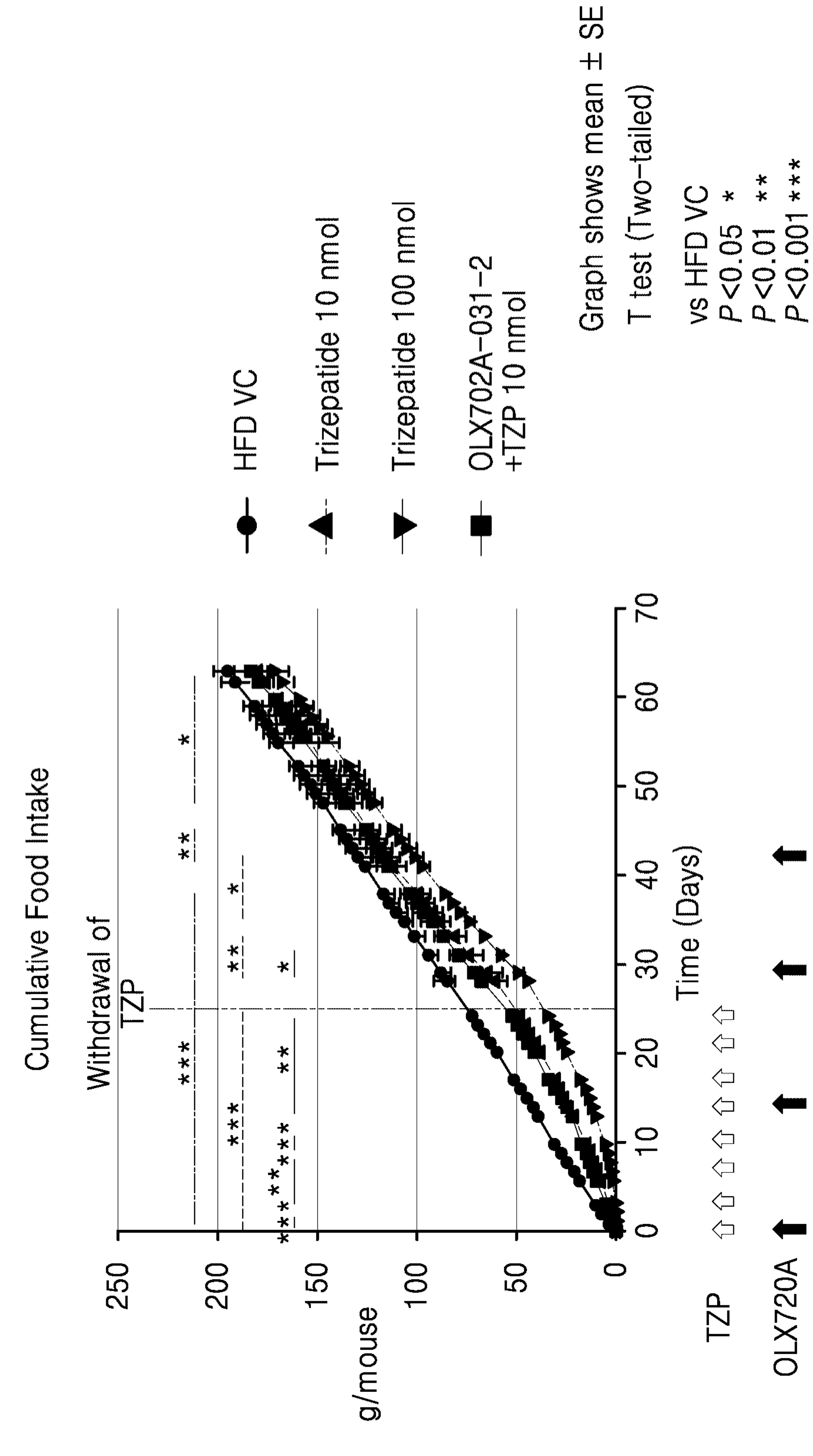

Cumulative Food Intake
Withdrawal of TZP
g/mouse
250
200
150
100
50
0
0
10
20
30
40
50
60
70
Time (Days)
TZP
OLX720A
***
**
*
HFD VC
Trizepatide 10 nmol
Trizepatide 100 nmol
OLX702A-031-2 +TZP 10 nmol
Graph shows mean ± SE
T test (Two-tailed)
vs HFD VC
P<0.05 *
P<0.01 **
P<0.001 ***

# FIG. 18B

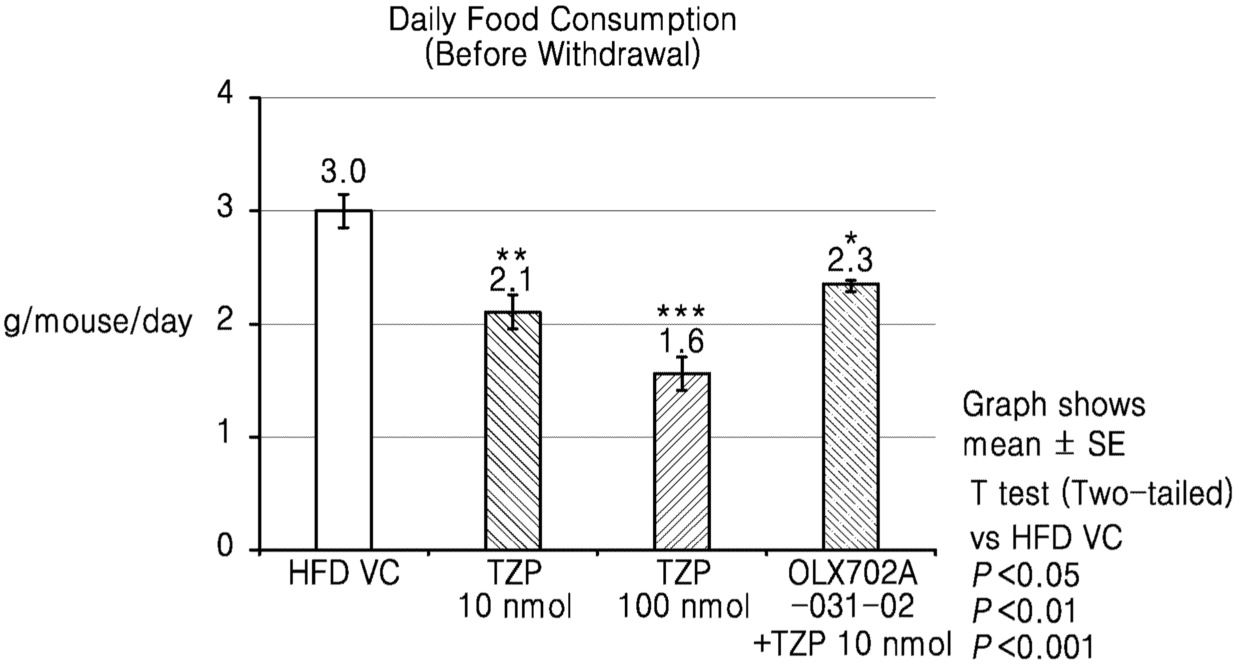


# FIG. 18C

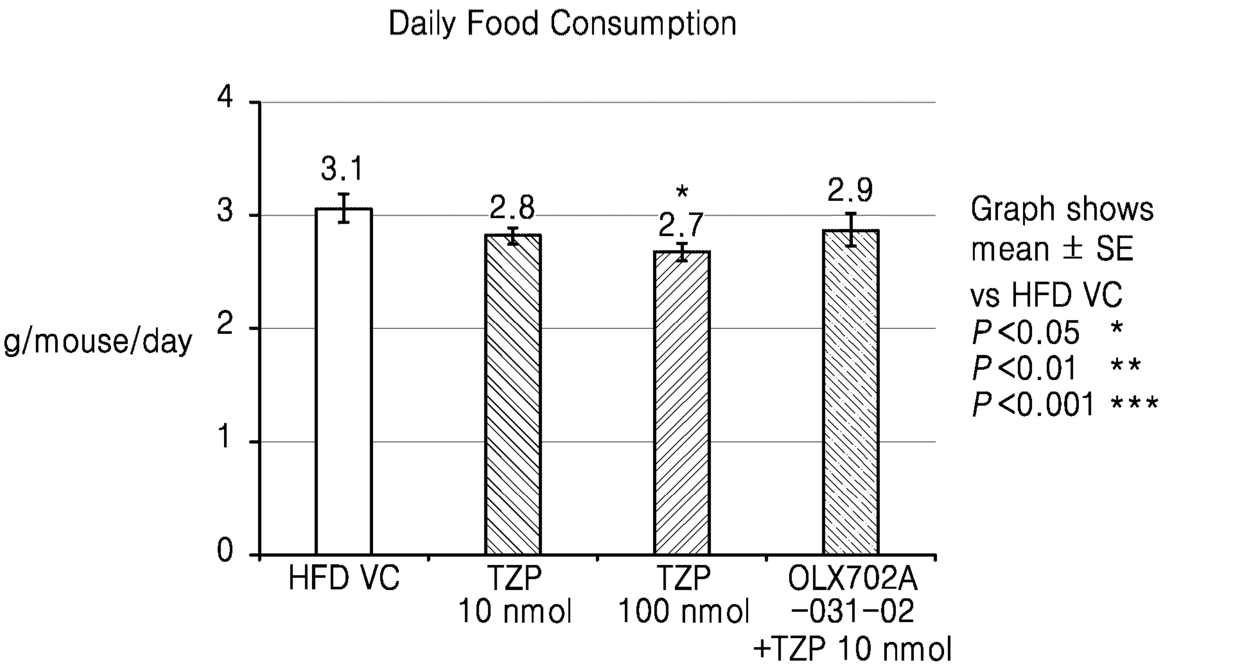

FIG. 19

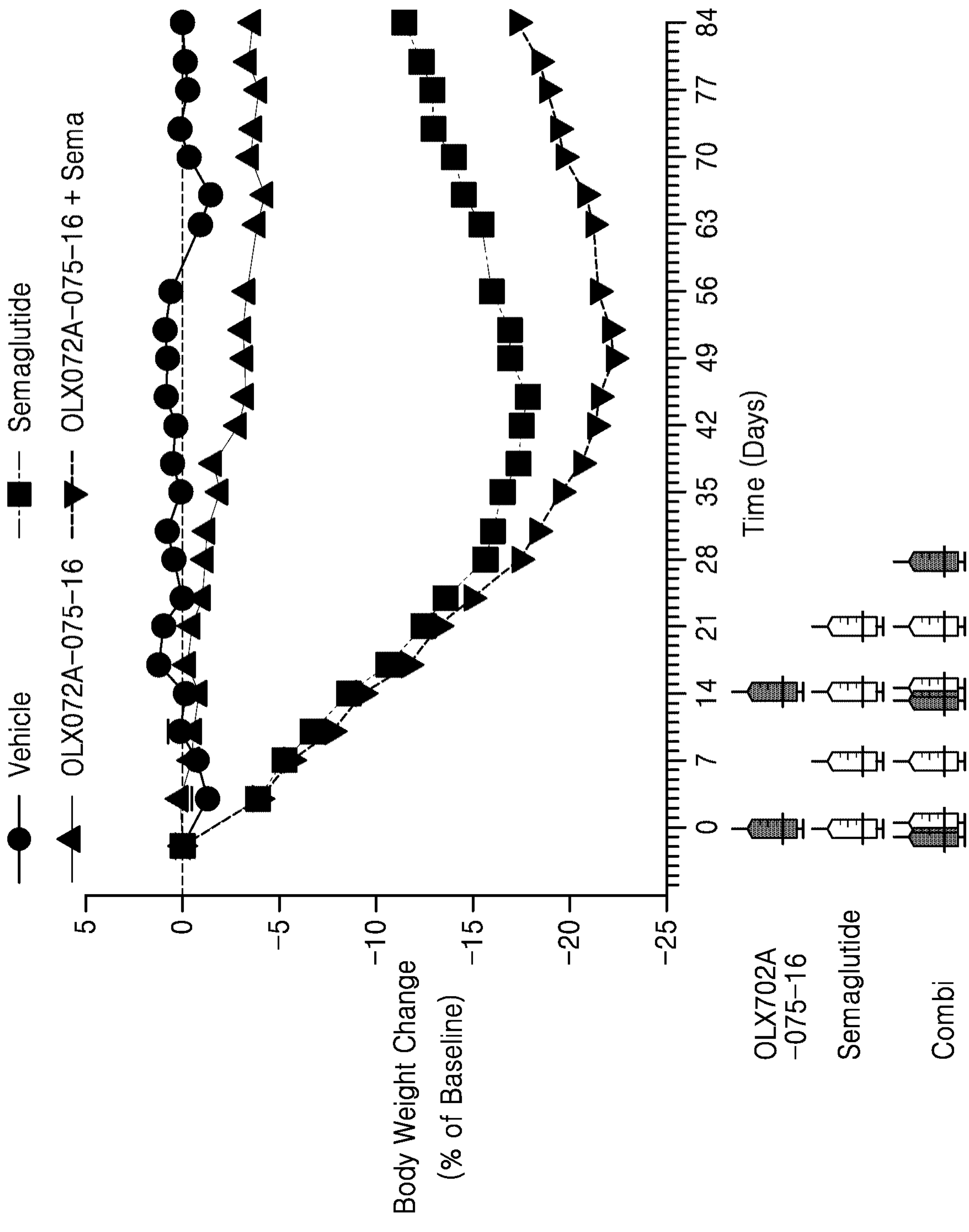
Vehicle
Semaglutide
OLX072A-075-16
OLX072A-075-16 + Sema
Body Weight Change
(% of Baseline)
5
0
-5
-10
-15
-20
-25
0
7
14
21
28
35
42
49
56
63
70
77
84
Time (Days)
OLX702A
-075-16
Semaglutide
Combi

FIG. 20

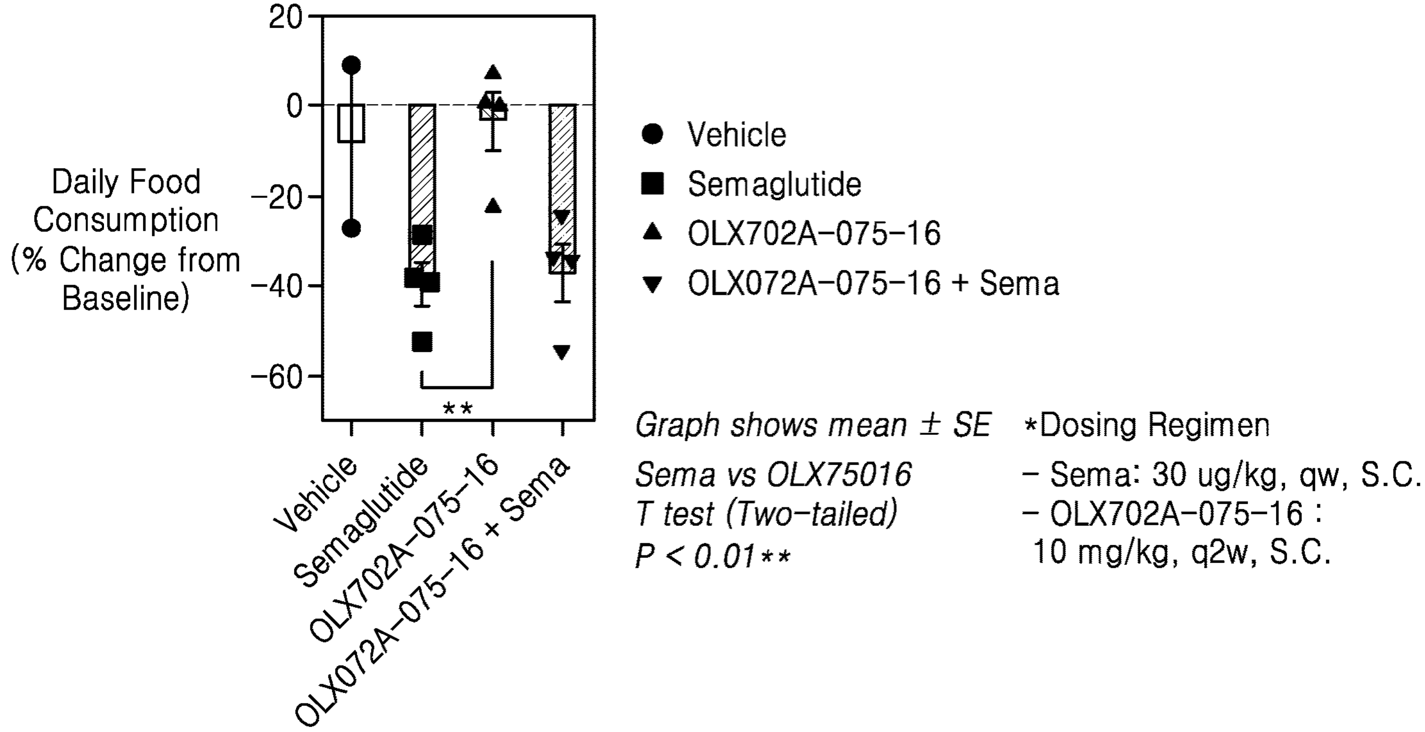
Daily Food Consumption (% Change from Baseline)
20
0
-20
-40
-60
**
Vehicle
Semaglutide
OLX702A-075-16
OLX072A-075-16 + Sema
Vehicle
Semaglutide
OLX702A-075-16
OLX072A-075-16 + Sema
Graph shows mean ± SE
Sema vs OLX75016
T test (Two-tailed)
P < 0.01**
*Dosing Regimen
- Sema: 30 ug/kg, qw, S.C.
- OLX702A-075-16 :
10 mg/kg, q2w, S.C.

FIG. 21

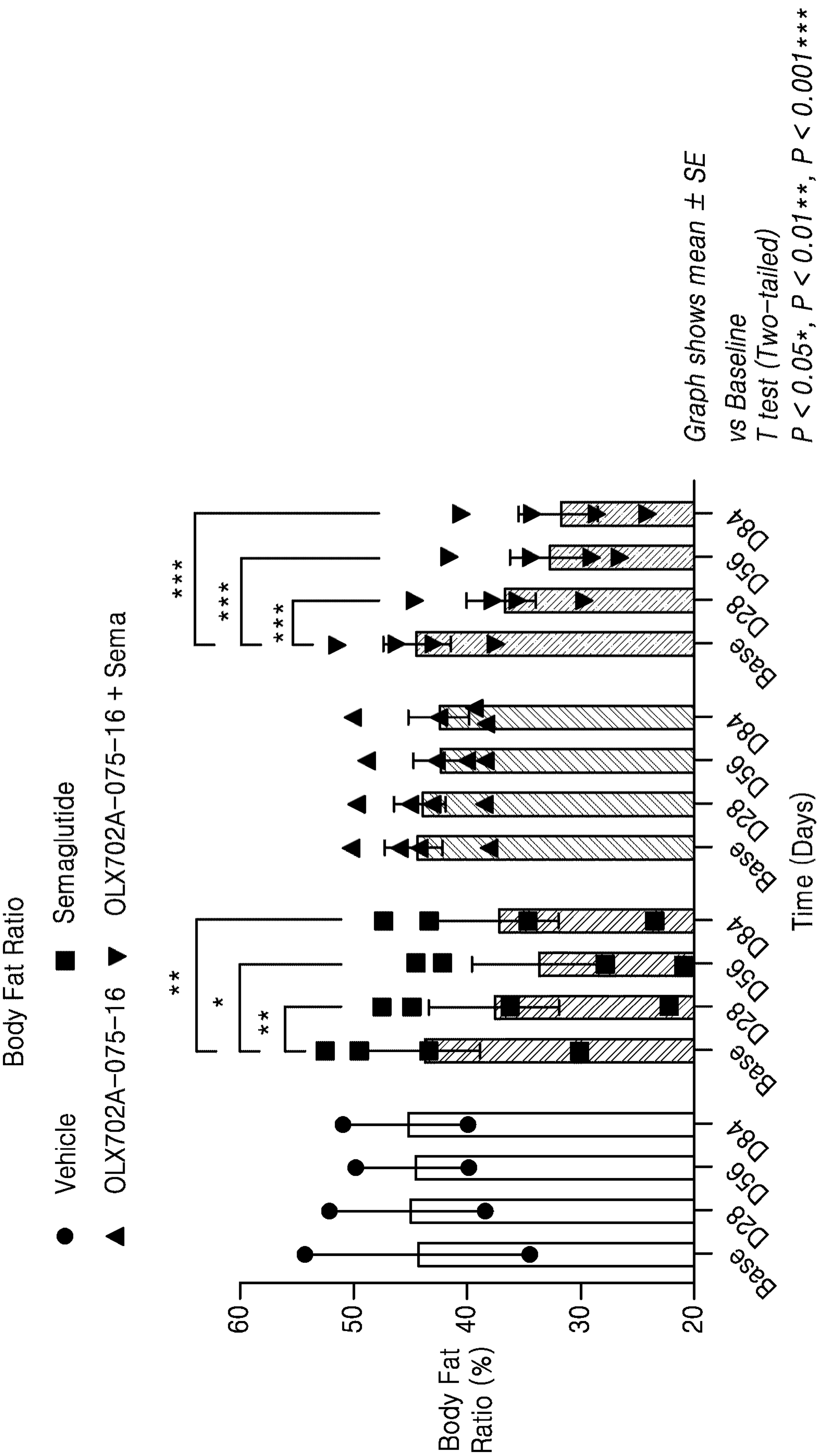
Body Fat Ratio
Vehicle
Semaglutide
OLX702A-075-16
OLX702A-075-16 + Sema
Body Fat Ratio (%)
60
50
40
30
20
Base
D28
D56
D84
**
*
**
***
***
***
Time (Days)
Graph shows mean ± SE
vs Baseline
T test (Two-tailed)
P < 0.05*, P < 0.01**, P < 0.001***

FIG. 22

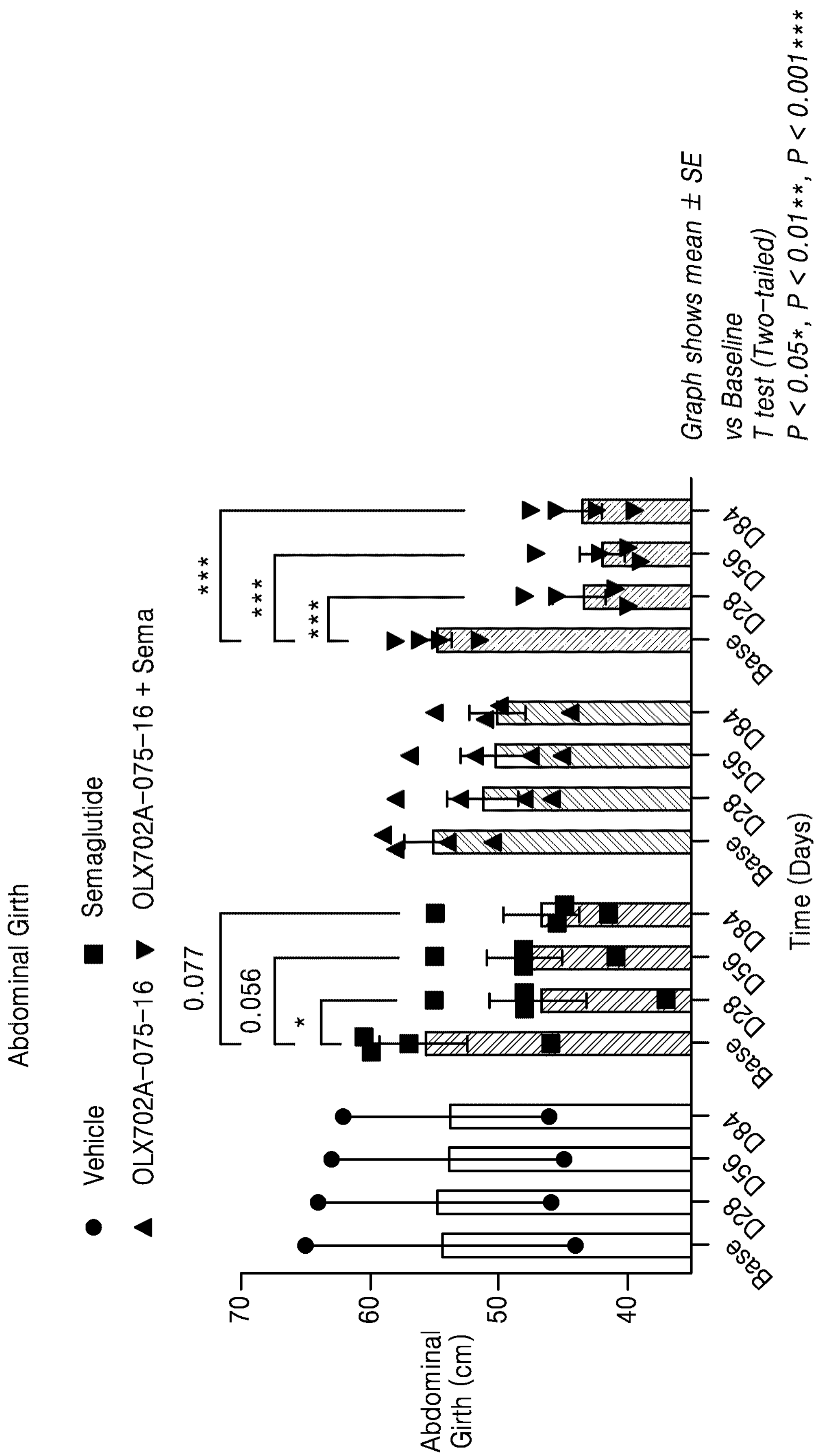
Abdominal Girth
Vehicle
Semaglutide
OLX702A-075-16
OLX702A-075-16 + Sema
Abdominal Girth (cm)
70
60
50
40
0.077
0.056
*
***
***
***
Base
D28
D56
D84
Time (Days)
Graph shows mean ± SE
vs Baseline
T test (Two-tailed)
P < 0.05*, P < 0.01**, P < 0.001***

**INTERNATIONAL SEARCH REPORT**

International application No.
**PCT/KR2024/003039**

**A. CLASSIFICATION OF SUBJECT MATTER**

**A61K 48/00**(2006.01)i; **A61K 31/713**(2006.01)i; **A61K 38/26**(2006.01)i; **A61P 3/04**(2006.01)i; **C12N 15/113**(2010.01)i; **A61K 31/7105**(2006.01)i

According to International Patent Classification (IPC) or to both national classification and IPC

**B. FIELDS SEARCHED**

Minimum documentation searched (classification system followed by classification symbols)

A61K 48/00(2006.01); A61K 31/00(2006.01); A61K 31/192(2006.01); A61K 38/28(2006.01); A61P 3/10(2006.01); C12N 15/113(2010.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 비만 (obesity), 센스 가닥 (sense strain), 안티센스 가닥 (antisense strain), RNAi

**C. DOCUMENTS CONSIDERED TO BE RELEVANT**

| Category* | Citation of document, with indication, where appropriate, of the relevant passages | Relevant to claim No. |
|---|---|---|
| X<br>Y | KR 10-2023-0010593 A (OLIX PHARMACEUTICALS, INC.) 19 January 2023 (2023-01-19)<br>See claims 1-21; and paragraphs [0004] and [0049]-[0061]. | 1-5<br>6-16 |
| Y | WO 2020-102351 A1 (CYMABAY THERAPEUTICS, INC.) 22 May 2020 (2020-05-22)<br>See claims 1 and 10-13; and paragraph [0039]. | 6-16 |
| A | US 2021-0038698 A1 (UNIVERSITY OF PITTSBURGH - OF THE COMMONWEALTH SYSTEM OF HIGHER EDUCATION) 11 February 2021 (2021-02-11)<br>See entire document. | 1-16 |
| A | WO 2022-036126 A2 (AMGEN INC.) 17 February 2022.<br>See entire document. | 1-16 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 June 2024** | **14 June 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2024/003039**

**C. DOCUMENTS CONSIDERED TO BE RELEVANT**

| Category* | Citation of document, with indication, where appropriate, of the relevant passages | Relevant to claim No. |
|---|---|---|
| A | RIVERA-PAREDEZ, B. et al. Association of MARC1, ADCY5, and BCO1 variants with the lipid profile, suggests an additive effect for hypertriglyceridemia in mexican adult men. International journal of molecular sciences. 2022, vol. 23, 11815, pp. 1-14. | 1-16 |

Form PCT/ISA/210 (second sheet) (July 2022)

**INTERNATIONAL SEARCH REPORT**

International application No.
**PCT/KR2024/003039**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/KR2024/003039**

| Patent document cited in search report | Publication date (day/month/year) | Patent family member(s) | Publication date (day/month/year) |
|---|---|---|---|
| KR 10-2023-0010593 A | 19 January 2023 | AU 2022-308807 A1 | 01 February 2024 |
| | | CA 3225054 A1 | 12 February 2023 |
| | | CN 117677697 A | 08 March 2024 |
| | | EP 4368717 A1 | 15 May 2024 |
| | | WO 2023-282704 A1 | 12 January 2023 |
| WO 2020-102351 A1 | 22 May 2020 | CA 3118965 A1 | 22 May 2020 |
| | | EP 3880187 A1 | 22 September 2021 |
| | | MX 2021005725 A | 21 July 2021 |
| | | US 2020-0155487 A1 | 21 May 2020 |
| US 2021-0038698 A1 | 11 February 2021 | DE 102021110865 A1 | 21 April 2022 |
| | | US 10835581 B2 | 17 November 2020 |
| | | US 2019-0160154 A1 | 30 May 2019 |
| | | US 2022-0062385 A1 | 03 March 2022 |
| WO 2022-036126 A2 | | AU 2023-326521 A1 | 09 March 2023 |
| | | CA 3190868 A1 | 17 February 2022 |
| | | CL 2023000407 A1 | 29 September 2023 |
| | | CN 116194119 A | 30 May 2023 |
| | | CO 2023002966 A2 | 17 March 2023 |
| | | CR 20230126 A | 03 May 2023 |
| | | EP 4196584 A2 | 21 June 2023 |
| | | IL 300360 A | 01 April 2023 |
| | | JP 2023-537943 A | 06 September 2023 |
| | | KR 10-2023-0046319 A | 05 April 2023 |
| | | MX 2023001786 A | 10 March 2023 |
| | | PE 20230993 A1 | 23 June 2023 |
| | | TW 202221124 A | 01 June 2022 |
| | | US 2022-0047621 A1 | 17 February 2022 |
| | | UY 39382 A | 25 February 2022 |
| | | WO 2022-036126 A3 | 21 April 2022 |

Form PCT/ISA/210 (patent family annex) (July 2022)